(19) Europäisches Patentamt
European Patent Office
Office européen des brevets

(11) EP 3 973 985 A1

(12) EUROPEAN PATENT APPLICATION

(43) Date of publication:
30.03.2022 Bulletin 2022/13

(21) Application number: 20306116.3

(22) Date of filing: 29.09.2020

(51) International Patent Classification (IPC):
*A61K 39/12* (2006.01) *C07K 14/005* (2006.01)

(52) Cooperative Patent Classification (CPC):
**C07K 14/005; A61K 39/12;** A61K 2039/53; A61K 2039/545; A61K 2039/55555; A61K 2039/575; C07K 2319/055; C12N 2770/20022; C12N 2770/20034

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ciloa**
**34080 Montpellier (FR)**

(72) Inventors:
- **TRENTIN, Bernadette Nadine**
**34725 Saint-André-de-Sangonis (FR)**
- **POLAK, Katarzyna**
**34080 Montpellier (FR)**
- **MAMOUN, Zaïne El Abiddine Robert**
**34725 Saint-André-de-Sangonis (FR)**

(74) Representative: **Icosa**
**83 avenue Denfert-Rochereau**
**75014 Paris (FR)**

# (54) EXTRACELLULAR VESICLES HARBORING A SPIKE PROTEIN, NUCLEIC ACIDS FOR PRODUCING THE SAME, AND METHOD OF IMMUNIZING A SUBJECT AGAINST SARS-COV-2 USING THE SAME

(57) The present invention relates to a nucleic acid comprising (i) a sequence of a *S* gene coding for a Spike protein and (ii) a sequence coding for a pilot peptide which interacts with ESCRT proteins. It further relates to extracellular vesicles, in particular exosomes, harboring at their external surface a Spike protein, obtainable by transfecting cells with the nucleic acid. It further relates to a method of immunizing a subject against a virus of the *Orthocoronavirinae* subfamily, in particular against SARS-CoV-2, by DNA prime-protein boost using the nucleic acid and the extracellular vesicles of the invention.

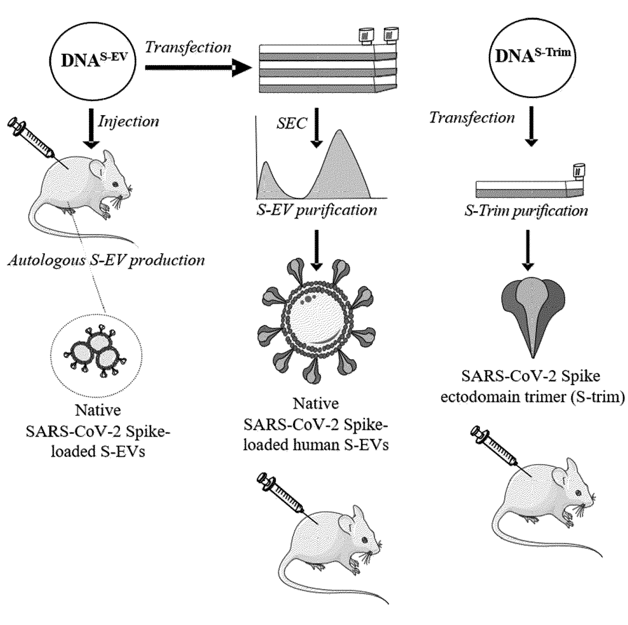


FIG. 2A

EP 3 973 985 A1

## Description

### FIELD OF INVENTION

The present invention relates to a nucleic acid comprising (i) a sequence of a S gene coding for a Spike protein and (ii) a sequence coding for a pilot peptide which interacts with ESCRT proteins. It further relates to extracellular vesicles, in particular exosomes, harboring at their external surface a Spike protein, obtainable by transfecting cells with the nucleic acid. It further relates to a method of immunizing a subject against a virus of the *Orthocoronavirinae* subfamily, in particular against SARS-CoV-2, by DNA prime-protein boost using the nucleic acid and the extracellular vesicles of the invention.

### BACKGROUND OF INVENTION

Unique efforts to develop a vaccine against the Severe Acute Respiratory Syndrome Coronavirus-2 (SARS-Cov-2) virus correspond to the level of the worldwide threat. Most of the vaccine candidates are based on the Spike (S) protein of the virus. In the absence of sufficient information on coronaviruses, it is important to learn on the lessons of the past on other enveloped viruses.

Coronaviruses, human immunodeficiency viruses (HIV), influenza viruses and others, possess type I transmembrane envelope proteins which enable their cellular entry. These proteins are synthesized as trimers that are then cleaved in two mature proteins S1 and S2 that form a "head" and a "stem" region, the later anchoring the mature trimer into the viral membrane and being responsible for the virus-cell membrane fusion. Proteins S1 and S2 are the equivalents of proteins SU and TM in HIV and of proteins HA1 and HA2 in influenza viruses.

The immunogenicity of viral envelope proteins is decreased by surface glycosylation and by the trimeric structure of the Spike, that occlude important epitopes. Decades on HIV vaccine development revealed the absolute necessity of using a fully native envelope protein embedded in a membrane in order to obtain a high-quality immune response. Surprisingly, protein SU-based vaccines provide no protection from HIV infection in human, although they could trigger neutralizing antibody production in small animals and primates (Mascola et al., 1996. J Infect Dis. 173(2):340-348). Additionally, influenza virus-neutralizing antibodies targeting protein HA1 epitopes are usually susceptible to strain variability (Hensley et al., 2009. Science. 326(5953):734-736).

The stem domain (protein TM in HIV, HA2 in influenza viruses or S2 in coronaviruses) is more conserved than the head domain (protein SU in HIV, HA1 in influenza viruses or S1 in coronaviruses). In HIV, the stem contains a highly conserved Membrane Proximal External Region (MPER) potent neutralizing epitope (Zwick, 2005. AIDS. 19(16):1725-1737). At the same time, influenza viruses stem is the target of broadly neutralizing antibodies (Wang et al., 2018. J Virol. 92(12):e00247-18). Thus, vaccines that mount broad protection against either HIV, influenza viruses, or other enveloped viruses, must elicit anti-stem domain antibodies (Khanna et al., 2014. Biomed Res Int. 2014:546274). Consequently, chimeric viruses or virus-like particles (VLPs) harboring native envelope proteins seem the best platforms as a result of taking the conformation of potential MPER peptides and the scaffold feature of lipids into consideration (Liu et al., 2018. Protein Cell. 9(7):596-615).

In addition to neutralizing antibodies, a consensus arises that triggering a strong T-cell immune response, even in the absence of cross-reactive neutralizing antibodies, correlates with a protection against several viral strains and for a longer time (Sridhar et al., 2013. Nat Med. 19(10):1305-1312; Wilkinson et al., 2012. Nat Med. 18(2):274-280). In addition, work on MERS-CoV and SARS-CoV revealed the importance of both humoral and cellular immune responses to induce a protection (Mubarak et al., 2019. J Immunol Res. 2019:6491738; Xu & Gao, 2004. CellMolImmunol. 1(2): 119-122). Finally, there is mounting evidence for a positive correlation between the resistance to the virus, the level of T cell response in SARS-Cov-2 infected people and T-cell cross reactivity against the S2 subunit (Braun et al., 2020. Nature. 2020; Sekine et al., 2020. Cell. 2020).

Among common immunization strategies is the so-called "DNA prime-protein boost" strategy, which comprises administering a DNA vaccine encoding the antigen *in vivo* during the priming step, then the antigen in proteinic form, produced *in vitro,* during the boosting step (Barnett et al., 1997. Vaccine. 15(8):869-873; Pal et al., 2006. Virology. 348(2):341-353; Wang et al., 2008. Vaccine. 26(31):3947-3957; Richmond et al., 1998. J Virol. 72(11):9092-9100; Liu et al., 2020. Sci Rep. 10(1):4144; Wang et al., 2004. Vaccine. 22(27-28):3622-3627; Xiao-wen et al., 2005. Vaccine. 23(14):1649-1656). In the field of coronaviruses, International patent application WO2016138160 has taught a method for inducing an immune response to the MERS-CoV Spike protein in the form of DNA prime-protein boost, with a priming step comprising administering a nucleic acid molecule encoding the MERS-CoV Spike protein and a boosting step comprising administering the MERS-CoV S1 subunit.

However, there remains a need for improved immunization strategies, eliciting both neutralizing antibodies and a strong T-cell immune response.

Here, the Inventors offer a novel immunization strategy, more closely reflecting the three-dimensional conformation of the antigen and its structural arrangement *vis-a-vis* the lipid membrane in which it is anchored.

Extracellular vesicles (EVs; of which, exosomes) are nano-sized particles secreted by all eukaryotic cells, capable of carrying antigenic information and triggering both humoral and cellular immune responses. To exploit EVs as natural vaccines, the Inventors have developed a technology that allows the natural loading of EVs with any fully native membrane protein (granted patents EP 2 268 816 and US 9,546,371; Desplantes et al., 2017. Sci Rep. 7(1):1032). Briefly, a protein of interest is fused to a proprietary Pilot Peptide (CilPP), which interacts with the Endosomal Sorting Complexes Required for Transport (ESCRT) cellular machinery to sort the resulting chimeric protein onto EVs.

By taking advantage of this technology, the Inventors present herein a SARS-CoV-2 vaccine, CoVEVax, which displays S1/S2 subunits naturally embedded in the membrane of EVs (hereafter, S-EVs), perfectly mimicking the original virus with its full transmembrane and membrane proximal external region (MPER) subdomains.

**SUMMARY**

The present invention relates to a nucleic acid comprising:

(i) a sequence of a S gene coding for a Spike protein from a virus of the *Orthocoronavirinae* subfamily, or a variant thereof; and
(ii) a sequence coding for a pilot peptide which interacts with ESCRT proteins.

In one embodiment, the virus of the *Orthocoronavirinae* subfamily is Severe Acute Respiratory Syndrome Coronavirus 2 (SARS-CoV-2).

In one embodiment, the S gene has a nucleic acid sequence coding for a Spike protein with SEQ ID NO: 2, or a variant thereof.

In one embodiment, the S gene has a nucleic acid sequence coding for a variant of the Spike protein with SEQ ID NO: 6.

In one embodiment, the pilot peptide comprises at least one YxxL motif with SEQ ID NO: 17 or DYxxL motif with SEQ ID NO: 20, and at least one PxxP motif with SEQ ID NO: 24, in which "x" represents any amino acid residue.

In one embodiment, the pilot peptide comprises an amino acid sequence with SEQ ID NO: 8 or a variant thereof, with the proviso that a variant of SEQ ID NO: 8 retains three YxxL motifs with SEQ ID NO: 17 and four PxxP motifs with SEQ ID NO: 24, in which "x" represents any amino acid residue.

In one embodiment, the nucleic acid is inserted into a nucleic acid expression vector, and is operably linked to regulatory elements.

The present invention also relates to an extracellular vesicle harboring at its external surface a Spike protein from a virus of the *Orthocoronavirinae* subfamily "S-EV", or a variant thereof.

In one embodiment, the virus of the *Orthocoronavirinae* subfamily is Severe Acute Respiratory Syndrome Coronavirus 2 (SARS-CoV-2)

In one embodiment, the extracellular vesicle is an exosome, preferably having a diameter ranging from about 30 nm to about 120 nm.

In one embodiment, the extracellular vesicle is obtainable by a method comprising steps of:

1) transfecting cells with the nucleic acid of the invention;
2) culturing the transfected cells for a time sufficient to allow extracellular vesicle production; and
3) purifying said extracellular vesicle.

The present invention also relates to a population of extracellular vesicles of the invention.

The present invention also relates to the nucleic acid of the invention, for use in a method of immunizing a subject against a virus of the *Orthocoronavirinae* subfamily, said method comprising:

1) at least one priming step, wherein the nucleic acid of the invention is to be administered to said subject; and
2) at least one boosting step, wherein:

   a. the extracellular vesicle of the invention or the population of extracellular vesicles of the invention is to be administered to said subject, or
   b. a trimeric Spike protein from a virus of the *Orthocoronavirinae* subfamily is to be administered to said subject,

thereby immunizing the subject against a virus of the *Orthocoronavirinae* subfamily.

In one embodiment, the virus of the *Orthocoronavirinae* subfamily is Severe Acute Respiratory Syndrome Coronavirus 2 (SARS-CoV-2).

In one embodiment, the method of immunizing comprises two iterations of the priming step and one iteration of the boosting step, preferably the period of time between each iteration ranges from about 2 weeks to about 1 month, more preferably the period of time between each iteration is about 3 weeks.

## DEFINITIONS

In the present invention, the following terms have the following meanings.

**"Adjuvant"** refers to a molecule or complex of molecules which allow(s) or otherwise facilitate(s) (1) the mobilization of antigen-presenting and/or polymorphonuclear cells; (2) the antigen uptake and presentation of the antigen(s) in a vaccine by antigen-presenting cells; (3) the secretion of proteins by antigen-presenting cells; (4) the recruitment, targeting and activation of antigen-specific cells; (5) the modulation of activities that regulate the ensuing immune responses; and/or (6) the protection of the antigen from degradation and elimination.

**"ESCRT"** or **"endosomal sorting complexes required for transport"** refers originally to a cellular machinery made up of five multi-subunit protein complexes, which act cooperatively at specialized endosomes to facilitate the movement of specific cargoes from the limiting membrane into vesicles that bud into the endosome lumen. This machinery is hijacked by several envelope viruses to bud from cellular membranes, including the plasma membrane.

**"Exosome"** refers to an extracellular vesicle that is produced in the endosomal compartment of eukaryotic cells (Théry et al., 2018. J Extracell Vesicles. 7(1):1535750; Yáñez-Mó et al., 2015. J Extracell Vesicles. 4:27066; van Niel et al., 2018. Nat Rev Mol Cell Biol. 19(4):213-228). Typically, exosomes harbor at their surface the CD81, CD63 and CD9 markers.

**"Expression vector"** refers to a vector capable of directing expression of a nucleic acid sequence of interest (such as, *e.g.,* a nucleic acid according to the present invention) in an appropriate host cell, comprising a promoter operatively linked to the nucleic acid sequence of interest, itself operatively linked to a termination sequence.

**"Extracellular vesicle"** refers to any vesicle composed of a lipid bilayer that are naturally released from a cell and comprise a cytosolic fraction of said cell. This expression in particular includes vesicles secreted into the extracellular space, *i.e.,* **"exosomes".**

**"Isolate"** refers to a genetic variant of a given viral strain. In brief, after rounds of replication, a virus-infected cell will contain a population of viral genomes, and virions derived from these viral genomes will vary slightly from each other. Likewise, a sample taken from a virus culture or an infected animal will contain numerous virions, many of which vary slightly. Consequently, an **"isolate"** refers to populations of slightly different viruses from a same strain, collected from a single source (a single subject, a single sample, etc.). By extension, the sequence of an **"isolate"** is thus a consensus sequence of the population of viral genomes collected from a single source. In the frame of this disclosure, SARS-CoV-2 isolate "Wuhan-Hu-1" with GenBank accession number MN908947.3, version 3 of March 18, 2020, is considered as the reference strain for all SARS-CoV-2 viruses. However, to date, over 100 000 viral genomic sequences of different SARS-CoV-2 isolates have been submitted on the GISAID EpiCoV™ database.

**"Isolated"** and any declensions thereof, as well as **"purified"** and any declensions thereof, are used interchangeably, and mean that a molecular entity to which it refers (*e.g*., a protein or peptide, a nucleic acid, an extracellular vesicle, etc.) is substantially free of other components (*i.e.*, of contaminants) found in the natural environment in which said molecular entity is normally found. Preferably, an isolated or purified molecular entity (*e.g.,* an isolated or purified protein or peptide, an isolated or purified nucleic acid, an isolated or purified extracellular vesicle, etc.) is substantially free of other molecular entities with which it is associated in a cell or a virus. By **"substantially free",** it is meant that said isolated or purified molecular entity represents more than 50% of a heterogeneous composition (*i.e.,* is at least 50% pure), preferably, more than 60%, more than 70%, more than 80%, more than 90%, more than 95%, and more preferably still more than 98% or 99%. Purity can be evaluated by various methods known by the one skilled in the art, including, but not limited to, chromatography, gel electrophoresis, immunoassay, composition analysis, biological assay, and the like.

**"Kit-of-parts"** refers to a kit comprising a plurality of (different) items that may be functionally used together, concomitantly or one after another.

**"Regulatory element"** refers to nucleic acid sequences which are necessary or desirable to affect the expression of coding and non-coding sequences of interest (such as, *e.g.,* a nucleic acid according to the present invention), to which they are operatively linked. Examples of regulatory elements include, but are not limited to, initiation signals, enhancers, regulators, promoters, and termination sequences. The nature and use of such regulatory sequences can differ, as is well known to the one skilled in the art, depending upon the host organism or cell.

**"*S* gene"** refers to a gene found in viruses of the *Orthocoronavirinae* subfamily, coding for the Spike protein.

**"SARS-CoV-2",** also known as **"severe acute respiratory syndrome coronavirus 2",** "2019 novel coronavirus", "2019-nCoV", "HCoV-19" or "hCoV-19", is a strain of *betacoronavirus* that causes coronavirus disease 2019 (also known as COVID-19). SARS-CoV-2 complete reference genome is available online, under GenBank accession number MN908947.3, version 3 of March 18, 2020. It comprises 10 genes: *orf1ab, S, orf3a*, *E, M, orf6, orf7a*, *orf8*, *N*, and *orf10.*

**"Spike"** or **"Spike protein",** also known as **"surface glycoprotein",** refers to a transmembrane protein, also termed peplomer, found in viruses of the *Orthocoronavirinae* subfamily, and protruding from the virus surface as spikes, hence its name. In SARS-CoV-1 and SARS-CoV-2, it allows the virus to bind to its host cell via the cell's receptor angiotensin-converting enzyme 2 (ACE2), and uses the serine protease TMPRSS2 to initiate entry and infection (Hoffmann et al., 2020. Cell. 181(2):271-280.e8). *In vivo,* the Spike protein is found on viruses as homotrimers, each consisting of the two subunits: a "head" called S1 and a "stem" called S2 (Wrapp et al., 2020. Science. 367(6483):1260-1263; Walls et al., 2020. Cell. 181(2):281-292.e6; Yuan et al., 2020. Science. 368(6491):630-633). The amino acid sequence of the Spike protein of the reference strain of SARS-CoV-2 (Wuhan-Hu-1 isolate, complete genome available under GenBank accession number MN908947.3, version 3 of March 18, 2020) is as set forth in **SEQ ID NO: 2.** It is composed of an extracellular domain (amino acid residues 13 to 1217 of **SEQ ID NO: 2),** a transmembrane domain (amino acid residues 1218 to 1234 of **SEQ ID NO: 2)** and a cytoplasmic domain (amino acid residues 1235 to 1273 of **SEQ ID NO: 2).** Subunit S1 corresponds to amino acid residues 13 to 685 of **SEQ ID NO: 2** and subunit S2 corresponds to amino acid residues 686 to 1273 of **SEQ ID NO: 2.** In the present disclosure, it is readily understood that the Spike protein with **SEQ ID NO: 2** is only a representative protein; hence, the expression **"Spike protein of SARS-CoV-2"** broadly encompasses Spike proteins of any isolate of the SARS-CoV-2 virus. One skilled in the art can easily determine amino acid residue correspondence between the representative protein sequence and any sequence of a Spike protein from another isolate of the SARS-CoV-2 virus.

**"Trimerization domain"** refers to an amino acid sequence within a polypeptide or protein that promotes self-assembly by associating with two other trimerization domains to form a trimer. Typically, trimerization domains comprise an amino acid sequence able to form an $\alpha$-helical coiled-coil domain or an isoleucine zipper domain.

**"Vector"** refers to a nucleic acid capable of transporting a nucleic acid of interest (such as, *e.g.,* a nucleic acid according to the present invention) to which it has been linked. Vectors capable of directing the expression of a nucleic acid of interest (such as, *e.g.,* a nucleic acid according to the present invention) are referred to as **"expression vectors".** In general, expression vectors are in the form of plasmids. Herein, the terms **"plasmid"** and **"vector"** are used interchangeably. However, other forms of expression vectors, which serve equivalent functions, are also encompassed under the term vector.

## DETAILED DESCRIPTION

An object of the present invention is a nucleic acid "*$DNA^{S\text{-}EV}$*" comprising:

(i) a sequence of a *S* gene coding for a Spike protein from a virus of the *Orthocoronavirinae* subfamily, or a variant thereof; and
(ii) a sequence coding for a pilot peptide which interacts with ESCRT proteins.

Orthocoronavirinae, more commonly known as coronaviruses, are enveloped, positive-sense single-stranded RNA viruses which infect birds and mammals. Coronaviruses are divided into four genera: *alphacoronavirus*, *betacoronavirus, gammacoronavirus* and *deltacoronavirus.* Alphacoronaviruses and betacoronaviruses infect mammals, while gammacoronaviruses and deltacoronaviruses primarily infect birds.

Exemplary species of alphacoronaviruses include, but are not limited to, alphacoronavirus 1 (also known as transmissible gastroenteritis virus, transmissible gastroenteritis coronavirus, or TGEV), human coronavirus 229E (also known as HCoV-229E), human coronavirus NL63 (also known as HCoV-NL63), miniopterus bat coronavirus 1 (also known as Bat-CoV MOP1), miniopterus bat coronavirus HKU8 (also known as Bat-CoV HKU8), porcine epidemic diarrhea virus (also known as PED virus or PEDV), rhinolophus bat coronavirus HKU2 (also known as Chinese horseshoe bat virus or Bat-CoV HKU2), and scotophilus bat coronavirus 512 (also known as Bat-CoV 512).

Exemplary species of betacoronaviruses include, but are not limited to, betacoronavirus 1 (including, without limitation, bovine coronavirus and human coronavirus OC43), hedgehog coronavirus 1, human coronavirus HKU1 (also known as HCoV-HKU1), Middle East respiratory syndrome-related coronavirus (also known as MERS-CoV, EMC/2012 or HCoV-EMC/2012), murine coronavirus (also known as M-CoV), pipistrellus bat coronavirus HKU5 (also known as Bat-CoV HKU5), rousettus bat coronavirus HKU9 (also known as HKU9-1), severe acute respiratory syndrome-related coronavirus (also known as SARSr-CoV or SARS-CoV; and including, without limitation, SARS-CoV and SARS-CoV-2), and tylonycteris bat coronavirus HKU4 (also known as Bat-CoV HKU4).

Exemplary species of gammacoronaviruses include, but are not limited to, avian coronavirus (also known as IBV) and beluga whale coronavirus SW1 (also known as Whale-CoV SW1).

Exemplary species of deltacoronaviruses include, but are not limited to, bulbul coronavirus HKU11 (also known as Bulbul-CoV HKU11) and porcine coronavirus HKU15 (also known as porcine coronavirus HKU15 or PorCoV HKU15).

In one embodiment, the virus of the *Orthocoronavirinae* subfamily is a virus of the *betacoronavirus* genus.

In one embodiment, the virus of the *Orthocoronavirinae* subfamily is a virus of the *betacoronavirus* genus, and severe acute respiratory syndrome-related coronavirus species.

In one embodiment, the virus of the *Orthocoronavirinae* subfamily is a virus of the *betacoronavirus* genus, severe acute respiratory syndrome-related coronavirus species and strain SARS-CoV-2.

In one embodiment, the virus of the *Orthocoronavirinae* subfamily is SARS-CoV-2.

In one embodiment, the S gene of SARS-CoV-2 has a nucleic acid sequence coding for a Spike protein with **SEQ ID NO: 2,** or a variant thereof. In one embodiment, the S gene coding for a Spike protein of SARS-CoV-2 with **SEQ ID NO: 2** or a variant thereof has a nucleic acid sequence with **SEQ ID NO: 1,** or a variant thereof.

**SEQ ID NO: 1** – *S* gene from SARS-CoV-2 Wuhan-Hu-1 isolate (MN908947.3)

ATGTTTGTTTTTCTTGTTTTATTGCCACTAGTCTCTAGTCAGTGTGTTAATCTT
ACAACCAGAACTCAATTACCCCCTGCATACACTAATTCTTTCACACGTGGTG
TTTATTACCCTGACAAAGTTTTCAGATCCTCAGTTTTACATTCAACTCAGGA
CTTGTTCTTACCTTTCTTTTCCAATGTTACTTGGTTCCATGCTATACATGTCTC
TGGGACCAATGGTACTAAGAGGTTTGATAACCCTGTCCTACCATTTAATGAT
GGTGTTTATTTTGCTTCCACTGAGAAGTCTAACATAATAAGAGGCTGGATTT
TTGGTACTACTTTAGATTCGAAGACCCAGTCCCTACTTATTGTTAATAACGC
TACTAATGTTGTTATTAAAGTCTGTGAATTTCAATTTTGTAATGATCCATTTT
TGGGTGTTTATTACCACAAAAACAACAAAAGTTGGATGGAAAGTGAGTTCA
GAGTTTATTCTAGTGCGAATAATTGCACTTTTGAATATGTCTCTCAGCCTTTT
CTTATGGACCTTGAAGGAAAACAGGGTAATTTCAAAAATCTTAGGGAATTT
GTGTTTAAGAATATTGATGGTTATTTTAAAATATATTCTAAGCACACGCCTA
TTAATTTAGTGCGTGATCTCCCTCAGGGTTTTTCGGCTTTAGAACCATTGGTA
GATTTGCCAATAGGTATTAACATCACTAGGTTTCAAACTTTACTTGCTTTAC
ATAGAAGTTATTTGACTCCTGGTGATTCTTCTTCAGGTTGGACAGCTGGTGC
TGCAGCTTATTATGTGGGTTATCTTCAACCTAGGACTTTTCTATTAAAATATA
ATGAAAATGGAACCATTACAGATGCTGTAGACTGTGCACTTGACCCTCTCTC
AGAAACAAAGTGTACGTTGAAATCCTTCACTGTAGAAAAAGGAATCTATCA
AACTTCTAACTTTAGAGTCCAACCAACAGAATCTATTGTTAGATTTCCTAAT
ATTACAAACTTGTGCCCTTTTGGTGAAGTTTTTAACGCCACCAGATTTGCAT
CTGTTTATGCTTGGAACAGGAAGAGAATCAGCAACTGTGTTGCTGATTATTC
TGTCCTATATAATTCCGCATCATTTTCCACTTTTAAGTGTTATGGAGTGTCTC
CTACTAAATTAAATGATCTCTGCTTTACTAATGTCTATGCAGATTCATTTGTA
ATTAGAGGTGATGAAGTCAGACAAATCGCTCCAGGGCAAACTGGAAAGATT

GCTGATTATAATTATAAATTACCAGATGATTTTACAGGCTGCGTTATAGCTT
GGAATTCTAACAATCTTGATTCTAAGGTTGGTGGTAATTATAATTACCTGTA
TAGATTGTTTAGGAAGTCTAATCTCAAACCTTTTGAGAGAGATATTTCAACT
GAAATCTATCAGGCCGGTAGCACACCTTGTAATGGTGTTGAAGGTTTTAATT
GTTACTTTCCTTTACAATCATATGGTTTCCAACCCACTAATGGTGTTGGTTAC
CAACCATACAGAGTAGTAGTACTTTCTTTTGAACTTCTACATGCACCAGCAA
CTGTTTGTGGACCTAAAAAGTCTACTAATTTGGTTAAAAACAAATGTGTCAA
TTTCAACTTCAATGGTTTAACAGGCACAGGTGTTCTTACTGAGTCTAACAAA
AAGTTTCTGCCTTTCCAACAATTTGGCAGAGACATTGCTGACACTACTGATG
CTGTCCGTGATCCACAGACACTTGAGATTCTTGACATTACACCATGTTCTTTT
GGTGGTGTCAGTGTTATAACACCAGGAACAAATACTTCTAACCAGGTTGCT
GTTCTTTATCAGGATGTTAACTGCACAGAAGTCCCTGTTGCTATTCATGCAG
ATCAACTTACTCCTACTTGGCGTGTTTATTCTACAGGTTCTAATGTTTTTCAA
ACACGTGCAGGCTGTTTAATAGGGGCTGAACATGTCAACAACTCATATGAG
TGTGACATACCCATTGGTGCAGGTATATGCGCTAGTTATCAGACTCAGACTA
ATTCTCCTCGGCGGGCACGTAGTGTAGCTAGTCAATCCATCATTGCCTACAC
TATGTCACTTGGTGCAGAAAATTCAGTTGCTTACTCTAATAACTCTATTGCC
ATACCCACAAATTTTACTATTAGTGTTACCACAGAAATTCTACCAGTGTCTA
TGACCAAGACATCAGTAGATTGTACAATGTACATTTGTGGTGATTCAACTGA
ATGCAGCAATCTTTTGTTGCAATATGGCAGTTTTTGTACACAATTAAACCGT
GCTTTAACTGGAATAGCTGTTGAACAAGACAAAAACACCCAAGAAGTTTTT
GCACAAGTCAAACAAATTTACAAAACACCACCAATTAAAGATTTTGGTGGT
TTTAATTTTTCACAAATATTACCAGATCCATCAAAACCAAGCAAGAGGTCAT
TTATTGAAGATCTACTTTTCAACAAAGTGACACTTGCAGATGCTGGCTTCAT
CAAACAATATGGTGATTGCCTTGGTGATATTGCTGCTAGAGACCTCATTTGT
GCACAAAAGTTTAACGGCCTTACTGTTTTGCCACCTTTGCTCACAGATGAAA
TGATTGCTCAATACACTTCTGCACTGTTAGCGGGTACAATCACTTCTGGTTG
GACCTTTGGTGCAGGTGCTGCATTACAAATACCATTTGCTATGCAAATGGCT
TATAGGTTTAATGGTATTGGAGTTACACAGAATGTTCTCTATGAGAACCAAA
AATTGATTGCCAACCAATTTAATAGTGCTATTGGCAAAATTCAAGACTCACT
TTCTTCCACAGCAAGTGCACTTGGAAAACTTCAAGATGTGGTCAACCAAAA
TGCACAAGCTTTAAACACGCTTGTTAAACAACTTAGCTCCAATTTTGGTGCA

ATTTCAAGTGTTTTAAATGATATCCTTTCACGTCTTGACAAAGTTGAGGCTG
AAGTGCAAATTGATAGGTTGATCACAGGCAGACTTCAAAGTTTGCAGACAT
ATGTGACTCAACAATTAATTAGAGCTGCAGAAATCAGAGCTTCTGCTAATCT
TGCTGCTACTAAAATGTCAGAGTGTGTACTTGGACAATCAAAAAGAGTTGA
TTTTTGTGGAAAGGGCTATCATCTTATGTCCTTCCCTCAGTCAGCACCTCATG
GTGTAGTCTTCTTGCATGTGACTTATGTCCCTGCACAAGAAAAGAACTTCAC
AACTGCTCCTGCCATTTGTCATGATGGAAAAGCACACTTTCCTCGTGAAGGT
GTCTTTGTTTCAAATGGCACACACTGGTTTGTAACACAAAGGAATTTTTATG
AACCACAAATCATTACTACAGACAACACATTTGTGTCTGGTAACTGTGATGT
TGTAATAGGAATTGTCAACAACACAGTTTATGATCCTTTGCAACCTGAATTA
GACTCATTCAAGGAGGAGTTAGATAAATATTTTAAGAATCATACATCACCA
GATGTTGATTTAGGTGACATCTCTGGCATTAATGCTTCAGTTGTAAACATTC
AAAAAGAAATTGACCGCCTCAATGAGGTTGCCAAGAATTTAAATGAATCTC
TCATCGATCTCCAAGAACTTGGAAAGTATGAGCAGTATATAAAATGGCCAT
GGTACATTTGGCTAGGTTTTATAGCTGGCTTGATTGCCATAGTAATGGTGAC
AATTATGCTTTGCTGTATGACCAGTTGCTGTAGTTGTCTCAAGGGCTGTTGTT
CTTGTGGATCCTGCTGCAAATTTGATGAAGACGACTCTGAGCCAGTGCTCAA
AGGAGTCAAATTACATTACACATAA

---

**SEQ ID NO: 2** – Spike protein encoded by SEQ ID NO: 1

MFVFLVLLPLVSSQCVNLTTRTQLPPAYTNSFTRGVYYPDKVFRSSVLHSTQDL
FLPFFSNVTWFHAIHVSGTNGTKRFDNPVLPFNDGVYFASTEKSNIIRGWIFGTT
LDSKTQSLLIVNNATNVVIKVCEFQFCNDPFLGVYYHKNNKSWMESEFRVYSS
ANNCTFEYVSQPFLMDLEGKQGNFKNLREFVFKNIDGYFKIYSKHTPINLVRDL
PQGFSALEPLVDLPIGINITRFQTLLALHRSYLTPGDSSSGWTAGAAAYYVGYLQ
PRTFLLKYNENGTITDAVDCALDPLSETKCTLKSFTVEKGIYQTSNFRVQPTESI
VRFPNITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFN
CYFPLQSYGFQPTNGVGYQPYRVVVLSFELLHAPATVCGPKKSTNLVKNKCVN
FNFNGLTGTGVLTESNKKFLPFQQFGRDIADTTDAVRDPQTLEILDITPCSFGGV

SVITPGTNTSNQVAVLYQDVNCTEVPVAIHADQLTPTWRVYSTGSNVFQTRAG
CLIGAEHVNNSYECDIPIGAGICASYQTQTNSPRRARSVASQSIIAYTMSLGAENS
VAYSNNSIAIPTNFTISVTTEILPVSMTKTSVDCTMYICGDSTECSNLLLQYGSFC
TQLNRALTGIAVEQDKNTQEVFAQVKQIYKTPPIKDFGGFNFSQILPDPSKPSKR
SFIEDLLFNKVTLADAGFIKQYGDCLGDIAARDLICAQKFNGLTVLPPLLTDEMI
AQYTSALLAGTITSGWTFGAGAALQIPFAMQMAYRFNGIGVTQNVLYENQKLI
ANQFNSAIGKIQDSLSSTASALGKLQDVVNQNAQALNTLVKQLSSNFGAISSVL
NDILSRLDKVEAEVQIDRLITGRLQSLQTYVTQQLIRAAEIRASANLAATKMSEC
VLGQSKRVDFCGKGYHLMSFPQSAPHGVVFLHVTYVPAQEKNFTTAPAICHDG
KAHFPREGVFVSNGTHWFVTQRNFYEPQIITTDNTFVSGNCDVVIGIVNNTVYD
PLQPELDSFKEELDKYFKNHTSPDVDLGDISGINASVVNIQKEIDRLNEVAKNLN
ESLIDLQELGKYEQYIKWPWYIWLGFIAGLIAIVMVTIMLCCMTSCCSCLKGCC
SCGSCCKFDEDDSEPVLKGVKLHYT

By **"variant",** it is meant (1) a S gene from an isolate of SARS-CoV-2 other than the reference isolate; (2) a fragment of the S gene of SARS-CoV-2, or (3) a mutant of the *S* gene of SARS-CoV-2; including any combinations of the above. Additionally, **"variant"** also encompasses (1) a Spike protein from an isolate of SARS-CoV-2 other than the reference isolate; (2) a fragment of the Spike protein of SARS-CoV-2, or (3) a mutant of the Spike protein of SARS-CoV-2; including any combinations of the above.

In one embodiment, the S gene of SARS-CoV-2 coding for the Spike protein is from a SARS-CoV-2 isolate other than "Wuhan-Hu-1".

By **"fragment",** it is meant a nucleic acid sequence or an amino acid sequence comprising at least 70%, preferably at least 75%, 80%, 85%, 90%, 95%, 96%, 97%, 98%, 99% or more contiguous nucleotides or amino acid residues of the reference nucleic acid sequence or amino acid sequence.

In one embodiment, a fragment of the S gene of SARS-CoV-2 has a nucleic acid sequence coding for a Spike protein comprising at least 70%, preferably at least 75%, 80%, 85%, 90%, 95%, 96%, 97%, 98%, 99% or more contiguous amino acid residues of the amino acid sequence with **SEQ ID NO: 2.** In one embodiment, a fragment of the S gene of SARS-CoV-2 coding for the Spike protein comprises at least 70%, preferably at least 75%, 80%, 85%, 90%, 95%, 96%, 97%, 98%, 99% or more contiguous nucleotides of the nucleic acid sequence with **SEQ ID NO: 1.**

In one embodiment, a fragment of the S gene of SARS-CoV-2 has a nucleic acid sequence coding for a Spike protein which retains at least part or the entirety of its cytoplasmic domain, and the entirety of its transmembrane domain. In one embodiment, a fragment of the S gene of SARS-CoV-2 coding for the Spike protein retains at least part or the entirety of the cytoplasmic domain, and the entirety of the transmembrane domain of said Spike protein.

By **"mutant",** it is meant a nucleic acid or amino acid sequence comprising at least 70%, preferably at least 75%, 80%, 85%, 90%, 95%, 96%, 97%, 98%, 99% or more sequence identity, preferably local sequence identity with the reference nucleic acid or amino acid sequence.

In one embodiment, a mutant of the S gene of SARS-CoV-2 has a nucleic acid sequence coding for a Spike protein comprising at least 70%, preferably at least 75%, 80%, 85%, 90%, 95%, 96%, 97%, 98%, 99% or more sequence identity, preferably local sequence identity with the amino acid sequence with **SEQ ID NO: 2.** In one embodiment, a mutant of the S gene of SARS-CoV-2 comprises at least 70%, preferably at least 75%, 80%, 85%, 90%, 95%, 96%, 97%, 98%, 99% or more sequence identity, preferably local sequence identity with the nucleic acid sequence with **SEQ ID NO: 1.**

Sequence identity refers to the number of identical or similar nucleotides or amino acid residues in a comparison between a test and a reference sequence. Sequence identity can be determined by sequence alignment of nucleic acid or amino acid sequences to identify regions of similarity or identity. For purposes herein, sequence identity is generally determined by alignment to identify identical nucleotides or amino acid residues. The alignment can be local or global. Matches, mismatches and gaps can be identified between compared sequences. Gaps are null nucleotides or amino acid residues inserted between the residues of aligned sequences so that identical or similar characters are aligned.

Generally, there can be internal and terminal gaps. When using gap penalties, sequence identity can be determined with no penalty for end gaps (*e.g.*, terminal gaps are not penalized). Alternatively, sequence identity can be determined without taking into account gaps as $\frac{\text{number of identical positions}}{\text{length of the total aligned sequence}} \times 100.$

A global alignment is an alignment that aligns two sequences from beginning to end, aligning each letter in each sequence only once. An alignment is produced, regardless of whether or not there is similarity or identity between the sequences. For example, 50% sequence identity based on global alignment means that in an alignment of the full sequence of two compared sequences, each of 100 nucleotides or amino acid residues in length, 50% of the residues are the same. It is understood that global alignment can also be used in determining sequence identity even when the length of the aligned sequences is not the same. The differences in the terminal ends of the sequences will be taken into account in determining sequence identity, unless the "no penalty for end gaps" is selected. Generally, a global alignment is used on sequences that share significant similarity over most of their length. Exemplary algorithms for performing global alignment include the Needleman-Wunsch algorithm (Needleman & Wunsch, 1970. J Mol Biol. 48(3):443-53). Exemplary programs and software for performing global alignment are publicly available and include the Global Sequence Alignment Tool available at the National Center for Biotechnology Information (NCBI) website (http://ncbi.nlm.nih.gov), and the program available at deepc2.psi.iastate.edu/aat/align/align.html.

A local alignment is an alignment that aligns two sequence, but only aligns those portions of the sequences that share similarity or identity. Hence, a local alignment determines if sub-segments of one sequence are present in another sequence. If there is no similarity, no alignment will be returned. Local alignment algorithms include BLAST or Smith-Waterman algorithm (Smith & Waterman, 1981. Adv Appl Math. 2(4):482-9). For example, 50% sequence identity based on local alignment means that in an alignment of the full sequence of two compared sequences of any length, a region of similarity or identity of 100 nucleotides or amino acid residues in length has 50% of the residues that are the same in the region of similarity or identity.

For purposes herein, sequence identity can be determined by standard alignment algorithm programs used with default gap penalties established by each supplier. Default parameters for the GAP program can include:

(1) a unary comparison matrix (containing a value of 1 for identities and 0 for non-identities) and the weighted comparison matrix of Gribskov & Burgess (1986. Nucleic Acids Res. 14(16):6745-63), as described by Schwartz & Dayhoff (1979. Matrices for detecting distant relationships. In Dayhoff (Ed.), Atlas of protein sequences. 5:353-358. Washington, DC: National Biomedical Research Foundation);
(2) a penalty of 3.0 for each gap and an additional 0.10 penalty for each symbol in each gap; and
(3) no penalty for end gaps.

Whether any mutant of the *S* gene of SARS-CoV-2 has a nucleic acid sequences that is at least 70%, preferably at least 75%, 80%, 85%, 90%, 95%, 96%, 97%, 98%, 99% or more "identical", or other similar variations reciting a percent identity, can be determined using known computer algorithms based on local or global alignment (see, *e.g.,* https://en.wikipedia.org/wiki/List_of_sequence_alignment_software [last accessed on September 15, 2020], providing links to dozens of known and publicly available alignment databases and programs).

Generally, for purposes herein, sequence identity is determined using computer algorithms based on global alignment, such as the Needleman-Wunsch Global Sequence Alignment tool available from NCBI/BLAST (http://blast.ncbi.nlm.nih.gov/Blast.cgi); or LAlign (William Pearson implementing the Huang and Miller algorithm [Huang & Miller, 1991. Adv Appl Math. 12(3):337-57).

Typically, the full-length sequence of each of the compared nucleic acid or amino acid sequence is aligned across the full-length of each sequence in a global alignment. Local alignment also can be used when the sequences being compared are substantially the same length.

Therefore, the term identity represents a comparison or alignment between a test and a reference nucleic acid or amino acid sequence. In one exemplary embodiment, "at least 70% of sequence identity" refers to percent identities from 70 to 100% relative to the reference nucleic acid or amino acid sequence. Identity at a level of 70% or more is indicative of the fact that, assuming for exemplification purposes a test and reference nucleic acid or amino acid sequence length of 100 nucleotides or amino acid residues are compared, no more than 30 out of 100 nucleotides or amino acid residues in the test nucleic acid or amino acid sequence differ from those of the reference nucleic acid or amino acid sequence. Such differences can be represented as point mutations randomly distributed over the entire length of a nucleic acid or amino acid sequence or they can be clustered in one or more locations of varying length up to the maximum allowable, *e.g.,* 30/100 nucleotide or amino acid residue difference (approximately 70% identity). Differences can also be due to deletions or truncations of nucleotides or amino acid residues. Differences are defined as nucleotide or amino acid residue substitutions, insertions or deletions. Depending on the length of the compared sequences, at the level of homologies or identities above about 85-90%, the result can be independent of the program and gap parameters set; such high levels of identity can be assessed readily, often without relying on software.

In one embodiment, a variant of the S gene of SARS-CoV-2 is a nucleic acid sequence with **SEQ ID NO: 5.** In one embodiment, a variant of the S gene of SARS-CoV-2 has a nucleic acid sequence coding for a Spike protein with **SEQ ID NO: 6.**

**SEQ ID NO: 5** – Variant of the *S* gene

ATGGTGCTGCTGCTGATCCTGTCTGTGCTGCTCCTGAAAGAAGATGTGCGGG
GCTCTGCCCAGAGCACCGGTCAATGTGTGAACCTGACCACCAGAACACAGC
TGCCTCCAGCCTACACCAACAGCTTCACCAGAGGCGTGTACTACCCCGACA
AGGTGTTCAGATCCAGCGTGCTGCACTCTACCCAGGACCTGTTCCTGCCTTT
CTTCAGCAACGTGACCTGGTTCCACGCCATCCACGTGTCCGGCACCAATGGC
ACCAAGAGATTCGACAACCCCGTGCTGCCCTTCAACGACGGGGTGTACTTT
GCCAGCACCGAGAAGTCCAACATCATCAGAGGCTGGATCTTCGGCACCACA
CTGGACAGCAAGACCCAGAGCCTGCTGATCGTGAACAACGCCACCAACGTG

GTCATCAAAGTGTGCGAGTTCCAGTTCTGCAACGACCCCTTCCTGGGCGTCT
ACTACCACAAGAACAACAAGAGCTGGATGGAAAGCGAGTTCCGGGTGTAC
AGCAGCGCCAACAACTGCACCTTCGAGTACGTGTCCCAGCCTTTCCTGATGG
ACCTGGAAGGCAAGCAGGGCAACTTCAAGAACCTGCGCGAGTTCGTGTTCA
AGAACATCGACGGCTACTTCAAGATCTACAGCAAGCACACCCCTATCAACC
TCGTGCGGGATCTGCCTCAGGGCTTCTCTGCTCTGGAACCCCTGGTGGATCT
GCCCATCGGCATCAACATCACCCGGTTTCAGACACTGCTGGCCCTGCACAG
AAGCTACCTGACACCTGGCGATAGCAGCTCTGGATGGACAGCTGGCGCCGC
TGCCTACTATGTGGGATACCTGCAGCCTCGGACCTTCCTGCTGAAGTACAAC
GAGAACGGCACCATCACCGACGCCGTGGATTGTGCTCTGGATCCTCTGAGC
GAGACAAAGTGCACCCTGAAGTCCTTCACCGTGGAAAAGGGCATCTACCAG
ACCAGCAACTTCCGGGTGCAGCCCACCGAATCCATCGTGCGGTTCCCCAAT
ATCACCAATCTGTGCCCCTTCGGCGAGGTGTTCAATGCCACCAGATTCGCCT
CTGTGTACGCCTGGAACCGGAAGCGGATCAGCAATTGCGTGGCCGACTACT
CCGTGCTGTACAACTCCGCCAGCTTCAGCACCTTCAAGTGCTACGGCGTGTC
CCCTACCAAGCTGAACGACCTGTGCTTCACAAACGTGTACGCCGACAGCTTC
GTGATCCGGGGAGATGAAGTGCGGCAGATTGCCCCTGGACAGACAGGCAA
GATCGCCGACTACAACTACAAGCTGCCCGACGACTTCACCGGCTGTGTGATT
GCCTGGAACAGCAACAACCTGGACTCCAAAGTCGGCGGCAACTACAATTAC
CTGTACCGGCTGTTCCGGAAGTCCAATCTGAAGCCCTTCGAGCGGGACATCT
CCACCGAGATCTATCAGGCCGGCAGCACCCCTTGTAACGGCGTGGAAGGCT
TCAACTGCTACTTCCCACTGCAGTCCTACGGCTTTCAGCCCACAAATGGCGT
GGGCTACCAGCCTTACAGAGTGGTGGTGCTGAGCTTCGAGCTGCTGCATGCT
CCTGCCACAGTGTGCGGCCCTAAGAAAAGCACCAATCTCGTGAAGAACAAA
TGCGTGAACTTCAACTTCAACGGCCTGACCGGCACCGGCGTGCTGACAGAG
AGCAACAAGAAGTTCCTGCCATTCCAGCAGTTCGGCCGGGATATCGCCGAT
ACCACAGATGCCGTCAGAGATCCCCAGACACTGGAAATCCTGGACATCACC
CCTTGCAGCTTCGGCGGAGTGTCTGTGATCACCCCTGGCACCAACACCAGCA
ATCAGGTGGCAGTGCTGTACCAGGACGTGAACTGTACCGAAGTGCCCGTGG
CCATTCACGCCGATCAGCTGACACCTACATGGCGGGTGTACTCCACCGGCA
GCAATGTGTTTCAGACCAGAGCCGGCTGTCTGATCGGAGCCGAGCACGTGA
ACAATAGCTACGAGTGCGACATCCCCATCGGCGCTGGCATCTGCGCCTCTTA

CCAGACACAGACAAACAGCCCCAGACGGGCCAGATCTGTGGCCAGCCAGA
GCATCATTGCCTACACAATGTCTCTGGGCGCCGAGAACAGCGTGGCCTACTC
CAACAACTCTATCGCTATCCCCACCAACTTCACCATCAGCGTGACCACAGAG
ATCCTGCCTGTGTCCATGACCAAGACCAGCGTGGACTGCACCATGTACATCT
GCGGCGATTCCACCGAGTGCTCCAACCTGCTGCTGCAGTACGGCAGCTTCTG
CACCCAGCTGAATAGAGCCCTGACAGGGATCGCCGTGGAACAGGATGCCAA
TACCGGCGAAGTGTTCGCCCAAGTGAAGCAGATCTACAAGACCCCTCCTAT
CAAGGACTTCGGCGGCTTCAATTTCAGCCAGATTCTGCCCGATCCTAGCAAG
CCCAGCAAGCGGAGCTTCATCGAGGACCTGCTGTTCAACAAAGTGACACTG
GCCGACGCCGGCTTCATCAAGCAGTATGGCGATTGTCTGGGCGACATTGCC
GCCAGGGATCTGATTTGCGCCCAGAAGTTTAACGGACTGACAGTGCTGCCT
CCTCTGCTGACCGATGAGATGATCGCCCAGTACACATCTGCCCTGCTGGCCG
GCACAATCACAAGCGGCTGGACATTTGGAGCTGGCGCTGCCCTGCAGATCC
CCTTTGCTATGCAGATGGCCTACAGATTCAACGGCATCGGAGTGACCCAGA
ATGTGCTGTACGAGAACCAGAAGCTGATCGCCAACCAGTTCAACAGCGCCA
TCGGCAAGATCCAGGACAGCCTGTCTAGCACAGCCAGCGCTCTGGGAAAGC
TGCAGGACGTGGTCAACCAGAATGCCCAGGCACTGAACACCCTGGTCAAGC
AGCTGAGCAGCAATTTCGGCGCCATCAGCTCTGTGCTGAACGATATCCTGA
GCAGACTGGACCCTCCTGAGGCCGAGGTGCAGATCGACAGACTGATCACCG
GAAGGCTGCAGTCCCTGCAGACCTACGTTACCCAGCAGCTGATCAGAGCCG
CCGAGATTAGAGCCTCTGCCAATCTGGCCGCCACCAAGATGTCTGAGTGTGT
GCTGGGCCAGAGCAAGAGAGTGGACTTTTGCGGCAAGGGCTACCACCTGAT
GAGCTTCCCTCAGTCTGCCCCTCACGGCGTGGTGTTTCTGCACGTGACATAC
GTGCCCGCTCAAGAGAAGAATTTCACCACCGCTCCAGCCATCTGCCACGAC
GGCAAAGCCCACTTTCCTAGAGAAGGCGTGTTCGTGTCCAACGGCACCCAT
TGGTTCGTGACCCAGCGGAACTTCTACGAGCCCCAGATCATCACCACCGAC
AACACCTTCGTGTCTGGCAACTGCGACGTCGTGATCGGCATTGTGAACAATA
CCGTGTACGACCCTCTGCAGCCCGAGCTGGACAGCTTCAAAGAGGAACTGG
ATAAGTACTTTAAGAACCACACAAGCCCCGACGTGGACCTGGGCGATATCA
GCGGAATCAATGCCAGCGTCGTGAACATCCAGAAAGAGATCGACCGGCTGA
ACGAGGTGGCCAAGAATCTGAACGAGAGCCTGATCGACCTGCAGGAACTGG

GGAAGTACGAGCAGTACATCAAGTGGCCCTGGTACATCTGGCTGGGCTTA
TCGCCGGACTGATTGCCATCGTGATGGTCACAATCATGCTG

**SEQ ID NO: 6** – Variant Spike protein encoded by SEQ ID NO: 5
MVLLLILSVLLLKEDVRGSAQSTGQCVNLTTRTQLPPAYTNSFTRGVYYPDKVF
RSSVLHSTQDLFLPFFSNVTWFHAIHVSGTNGTKRFDNPVLPFNDGVYFASTEK
SNIIRGWIFGTTLDSKTQSLLIVNNATNVVIKVCEFQFCNDPFLGVYYHKNNKS
WMESEFRVYSSANNCTFEYVSQPFLMDLEGKQGNFKNLREFVFKNIDGYFKIY
SKHTPINLVRDLPQGFSALEPLVDLPIGINITRFQTLLALHRSYLTPGDSSSGWTA
GAAAYYVGYLQPRTFLLKYNENGTITDAVDCALDPLSETKCTLKSFTVEKGIYQ
TSNFRVQPTESIVRFPNITNLCPFGEVFNATRFASVYAWNRKRISNCVADYSVLY
NSASFSTFKCYGVSPTKLNDLCFTNVYADSFVIRGDEVRQIAPGQTGKIADYNY
KLPDDFTGCVIAWNSNNLDSKVGGNYNYLYRLFRKSNLKPFERDISTEIYQAGS
TPCNGVEGFNCYFPLQSYGFQPTNGVGYQPYRVVVLSFELLHAPATVCGPKKS
TNLVKNKCVNFNFNGLTGTGVLTESNKKFLPFQQFGRDIADTTDAVRDPQTLEI
LDITPCSFGGVSVITPGTNTSNQVAVLYQDVNCTEVPVAIHADQLTPTWRVYST
GSNVFQTRAGCLIGAEHVNNSYECDIPIGAGICASYQTQTNSPRRARSVASQSIIA
YTMSLGAENSVAYSNNSIAIPTNFTISVTTEILPVSMTKTSVDCTMYICGDSTECS
NLLLQYGSFCTQLNRALTGIAVEQDANTGEVFAQVKQIYKTPPIKDFGGFNFSQI
LPDPSKPSKRSFIEDLLFNKVTLADAGFIKQYGDCLGDIAARDLICAQKFNGLTV
LPPLLTDEMIAQYTSALLAGTITSGWTFGAGAALQIPFAMQMAYRFNGIGVTQN
VLYENQKLIANQFNSAIGKIQDSLSSTASALGKLQDVVNQNAQALNTLVKQLSS
NFGAISSVLNDILSRLDPPEAEVQIDRLITGRLQSLQTYVTQQLIRAAEIRASANL
AATKMSECVLGQSKRVDFCGKGYHLMSFPQSAPHGVVFLHVTYVPAQEKNFT
TAPAICHDGKAHFPREGVFVSNGTHWFVTQRNFYEPQIITTDNTFVSGNCDVVI
GIVNNTVYDPLQPELDSFKEELDKYFKNHTSPDVDLGDISGINASVVNIQKEIDR
LNEVAKNLNESLIDLQELGKYEQYIKWPWYIWLGFIAGLIAIVMVTIML

According to the present invention, the nucleic acid comprises a sequence coding for a pilot peptide which interacts with ESCRT proteins.

Pilot peptide which interacts with ESCRT proteins have been described in granted patents EP 2 268 816, and US 9,546,371, which are incorporated by reference.

In one embodiment, the pilot peptide is capable of being addressed to the membrane vesicles, in particular to the exosome-forming vesicles, or to the cell compartment(s) involved in the formation of the membrane vesicles, and in particular the exosome-forming vesicles in eukaryotic cells. When integrated into a chimeric protein, such as a chimeric protein comprising an amino acid sequence of the Spike protein or any of the variants thereof defined hereinabove, fused or linked with said pilot peptide, the pilot peptide enables addressing said chimeric protein to the membrane vesicles and/or to their location(s) of formation and in particular to address said chimeric protein to the membrane of membrane vesicles, such that said protein can be secreted by a cell in association with the membrane vesicles (in particular exosomes), in particular an appropriate eukaryotic cell.

In one embodiment, the pilot peptide comprises at least one YxxL motif **(SEQ ID NO: 17),** in which "x" represents any amino acid residue. In particular, it may comprise one, two or three YxxL motifs with **SEQ ID NO: 17.**

In one embodiment, said YxxL motif or one of the YxxL motifs of the pilot peptide may, for example, be YINL **(SEQ ID NO: 18)** or YSHL **(SEQ ID NO: 19).**

In one embodiment, the pilot peptide comprises a DYxxL motif **(SEQ ID NO: 20),** in which "x" represents any residue.

In one embodiment, said DYxxL motif may, for example, be DYINL **(SEQ ID NO: 21).**

Alternatively or additionally, the pilot peptide comprises at least one motif equivalent to a YxxL motif **(SEQ ID NO: 17),** for example, a YxxF motif **(SEQ ID NO: 22),** in which "x" represents any residue.

Alternatively or additionally, the pilot peptide comprises at least one motif equivalent to a DYxxL motif **(SEQ ID NO: 20),** for example, a DYxxF motif **(SEQ ID NO: 23),** in which "x" represents any residue.

In one embodiment, the pilot peptide further comprises at least one PxxP motif **(SEQ ID NO: 24),** in which "x" represents any residue. In particular, it may comprise one, two, three or four PxxP motifs with **SEQ ID NO: 24.**

In one embodiment, said PxxP motif or one of the PxxP motifs of the pilot peptide is PSAP **(SEQ ID NO: 25)** or PTAP **(SEQ ID NO: 26**)

In one embodiment, the pilot peptide comprises at least one YxxL motif with **SEQ ID NO: 17** or DYxxL motif with **SEQ ID NO: 20,** and at least one PxxP motif with **SEQ ID NO: 24**.

In one embodiment, the pilot peptide consists of an amino acid sequence having one, two or three YxxL motif(s) with **SEQ ID NO: 17;** and one, two, three or four PxxP motif(s) with **SEQ ID NO: 24.** In one embodiment, the pilot peptide consists of an amino acid sequence having three YxxL motifs with **SEQ ID NO: 17;** and four PxxP motifs with **SEQ ID NO: 24.**

In one embodiment, the YxxL motif with **SEQ ID NO: 17** or one of the YxxL motifs with **SEQ ID NO: 17** is located downstream, *i.e.,* in a C-terminal position, with respect to the one or more PxxP motif(s) with **SEQ ID NO: 24.**

The proteins having a pilot peptide comprising at least one YxxL motif with **SEQ ID NO: 17** include cellular proteins and viral proteins. In particular, these viral proteins are proteins of enveloped viruses, such as transmembrane glycoproteins of enveloped viruses, or herpesvirus proteins, *e.g.*, the LMP2-A protein of the Epstein-Barr virus which comprises at least two YxxL motifs with **SEQ ID NO: 17.**

In one embodiment, the pilot peptide is that of a transmembrane glycoprotein of a retrovirus. In one embodiment, the pilot peptide may be that of a transmembrane glycoprotein of a retrovirus selected from the group comprising or consisting of bovine leukemia virus (BLV), human immunodeficiency virus (HIV) (such as, without limitation, HIV-1 or HIV-2), human T-cell leukemia virus (HTLV) (such as, without limitation, HTLV-1 or HTLV-2), and Mason-Pfizer monkey virus (MPMV).

In one embodiment, the pilot peptide comprises one of the following amino acid sequences:

**SEQ ID NO: 27:** PxxPxxxxPxxPxSxYxxLxPxxPExYxxLxPxxPDYxxL;
**SEQ ID NO: 28:** PxxP$x_n$PxxP$x_n$SxYxxL$x_n$PxxPE$x_n$YxxL$x_n$PxxPDYxxL;
**SEQ ID NO: 29:** PxxPxxxxPxxPxSxYxxLxPxxPExYxxLxPxxPDYxxLxxxx; and
**SEQ ID NO: 30:** PxxP$x_n$PxxP$x_n$SxYxxL$x_n$PxxPE$x_n$YxxL$x_n$PxxPDYxxLxxxx;

in which "x" and "$x_n$", respectively, represent any residue and any one or several amino acid residue(s).

In one embodiment, the pilot peptide comprises one of the following amino acid sequences:

**SEQ ID NO: 31:** PxxPxxxxxxxxxxxxYxxL;
**SEQ ID NO: 32:** PxxPxxxxxxxxxxxDYxxL;
**SEQ ID NO: 33:** PxxPxxYxxxxxxxxxYxxL;
**SEQ ID NO: 34:** PxxPxxYxxxxxxxxDYxxL;
**SEQ ID NO: 35:** PxxPExYxxLxPxxPDYxxL;
**SEQ ID NO: 36:** PxxP$x_n$YxxL;
**SEQ ID NO: 37:** PxxPxnDYxxL;
**SEQ ID NO: 38:** PxxP$x_n$Y$x_n$YxxL;
**SEQ ID NO: 39:** PxxP$x_n$Y$x_n$DYxxL;
**SEQ ID NO: 40:** PxxPE$x_n$YxxL$x_n$PxxPDYxxL;
**SEQ ID NO: 41:** PxxPxxxxPxxPxxxYxxLxPxxPExYxxLxPxxPDYxxL;

**SEQ ID NO: 42:** $PxxPx_nPxxPx_nYxxLx_nPxxPEx_nYxxLx_nPxxPDYxxL$;
**SEQ ID NO: 43:** PxxPxxxxPxxPxxxYxxLxPxxPExYxxLxPxxPDYxxLxxxx; and
**SEQ ID NO: 44:** $PxxPx_nPxxPx_nYxxLx_nPxxPEx_nYxxLx_nPxxPDYxxLxxxx$,

in which "x" and "$x_n$", respectively, represent any residue and any one or several amino acid residue(s).

In particular, "n" may be greater than or equal to 1 and less than 50. "n" may, in particular, have any value between 1 and 20.

In one embodiment, the pilot peptide comprises from 6 to 100 amino acid residues, in particular from 20 to 80, from 30 to 70, or from 40 to 60 amino acid residues, for example 40, 41, 42, 43, 44, 45, 46, 47, 48, 49 or 50 amino acid residues.

In one embodiment, the pilot peptide comprises or consists of an amino acid sequence with **SEQ ID NO: 8** or a variant thereof. In one embodiment, the nucleic acid sequence coding for the pilot peptide with **SEQ ID NO: 8** comprises or consists of **SEQ ID NO: 7** or a variant thereof.

---

**SEQ ID NO:** 7 – CilPP

GCGCCCCACTTCCCTGAAATCTCCTTCCCCCCTAAACCCGATTCTGATTATC
AGGCCTTGCTACCATCCGCGCCAGAGATCTACTCTCACCTCTCCCCCACCAA
ACCCGATTACATCAACCTTCGACCGGCGCCCTAA

**SEQ ID NO: 8** - CilPP encoded by SEQ ID NO: 7 APHFPEISFPPKPDSDYQALLPSAPEIYSHLSPTKPDYINLRPAP

In one embodiment, a variant of the amino acid sequence with **SEQ ID NO: 8** comprises an amino acid sequence comprising at least 70%, preferably at least 75%, 80%, 85%, 90%, 95%, 96%, 97%, 98%, 99% or more contiguous amino acid residues of the amino acid sequence with **SEQ ID NO: 8.**

Additionally or alternatively, a variant of the amino acid sequence with **SEQ ID NO: 8** comprises an amino acid sequence comprising at least 70%, preferably at least 75%, 80%, 85%, 90%, 95%, 96%, 97%, 98%, 99% or more sequence identity, preferably local sequence identity with the amino acid sequence with **SEQ ID NO: 8.**

In one embodiment, a variant of the amino acid sequence with **SEQ ID NO: 8** retains at least one, two or three YxxL motif(s) with **SEQ ID NO: 17** and at least one, two, three or four PxxP motif(s) with **SEQ ID NO: 24.** In one embodiment, a variant of the amino acid sequence with **SEQ ID NO: 8** retains three YxxL motifs with **SEQ ID NO: 17** and four PxxP motifs with **SEQ ID NO: 24.**

In one embodiment, the nucleic acid according to the present invention is inserted into a nucleic acid vector, preferably a nucleic acid expression vector.

In one embodiment, the nucleic acid according to the present invention is inserted into a nucleic acid vector, preferably a nucleic acid expression vector, operably linked to regulatory elements.

In one embodiment, the nucleic acid vector, preferably the nucleic acid expression vector, comprises, in this order, a promoter, the nucleic acid according to the present invention, a polyadenylation signal, and a termination sequence.

In one embodiment, the promoter is a cytomegalovirus-human T-lymphotropic virus type I (CMV-HTLV-I) chimeric promoter (Barouch et al., 2005. J Virol. 79(14):8828-8834).

In one embodiment, the polyadenylation signal and termination sequence is a bovine growth hormone (bGH) poly(A) signal (Pfarr et al., 1986. DNA. 5(2): 115-122).

A further object of the present invention is an extracellular vesicle harboring at its external surface a Spike protein from a virus of the *Orthocoronavirinae* subfamily "*S-EV*", or a variant thereof, as defined hereinabove.

The expression **"harboring at its external surface"** means that a Spike protein is anchored, through its transmembrane domain, in the lipid bilayer of the extracellular vesicle, and exposed, partially or completely, outside said membrane vesicle, included (completely or partially) in the membrane of said extracellular vesicle.

In one embodiment, the virus of the *Orthocoronavirinae* subfamily is a virus of the *betacoronavirus* genus, as defined hereinabove. In one embodiment, the virus of the *Orthocoronavirinae* subfamily is a virus of the *betacoronavirus* genus, and severe acute respiratory syndrome-related coronavirus species, as defined hereinabove. In one embodiment, the virus of the *Orthocoronavirinae* subfamily is a virus of the *betacoronavirus* genus, severe acute respiratory syndrome-related coronavirus species and strain SARS-CoV-2, as defined hereinabove. In one embodiment, the virus of the *Orthocoronavirinae* subfamily is SARS-CoV-2, as defined hereinabove.

In one embodiment, the Spike protein of SARS-CoV-2 has an amino acid sequence with **SEQ ID NO: 2,** or a variant thereof, as defined hereinabove.

Variants of the Spike protein of SARS-CoV-2 have been defined hereabove in the context of the nucleic acid "*DNA*$^{S-EV}$", which applies here *mutatis mutandis.*

In one embodiment, the Spike protein is from a SARS-CoV-2 isolate other than "Wuhan-Hu-1" .

In one embodiment, a fragment of the Spike protein of SARS-CoV-2 comprises at least 70%, preferably at least 75%, 80%, 85%, 90%, 95%, 96%, 97%, 98%, 99% or more contiguous amino acid residues of the amino acid sequence with **SEQ ID NO: 2.**

In one embodiment, a fragment of the Spike protein of SARS-CoV-2 retains at least part or the entirety of its cytoplasmic domain, and the entirety of its transmembrane domain.

In one embodiment, a mutant of the Spike protein of SARS-CoV-2 comprises at least 70%, preferably at least 75%, 80%, 85%, 90%, 95%, 96%, 97%, 98%, 99% or more sequence identity, preferably local sequence identity with the amino acid sequence with **SEQ ID NO: 2.**

In one embodiment, a variant the Spike protein of SARS-CoV-2 comprises or consists of the amino acid sequence with **SEQ ID NO: 6.**

In one embodiment, the extracellular vesicle is an exosome.

In one embodiment, exosomes have a diameter ranging from about 30 nm to about 150 nm, preferably from about 30 nm to about 120 nm, more preferably from about 40 nm to about 80 nm.

A further object of the present invention is a population of extracellular vesicles harboring at their external surface a Spike protein from a virus of the *Orthocoronavirinae* subfamily "*S-EV*", or a variant thereof, as defined hereinabove.

In one embodiment, the population of extracellular vesicles according to the present invention is monodisperse in aqueous solutions, preferably in water and/or in PBS.

By **"monodisperse",** it is meant that the extracellular vesicles in the population of extracellular vesicles are substantially uniform in size. By **"substantially uniform",** it is meant that the extracellular vesicles have a narrow distribution of sizes around an average size. In one embodiment, the extracellular vesicles in water and/or in PBS have sizes exhibiting a standard deviation of less than 100 % with respect to their average size, such as less than 75 %, 50 %, 40 %, 30 %, 20 %, 10 %, or less than 5 %.

A further object of the present invention is a method of obtaining an extracellular vesicle or a population of extracellular vesicles harboring at its/their external surface a Spike protein from a virus of the *Orthocoronavirinae* subfamily "*S-EV*", or a variant thereof, as defined hereinabove.

General means and methods for obtaining extracellular vesicles or a population of extracellular vesicles are well known in the art. See, *e.g.*, Whitford & Guterstam, 2019. Future Med Chem. 11(10): 1225-1236; Taylor & Shah, 2015. Methods. 87:3-10; Desplantes et al., 2017. Sci Rep. 7(1):1032.

In one embodiment, the method of obtaining an extracellular vesicle or a population of extracellular vesicles comprises a step of producing the extracellular vesicle or the population of extracellular vesicles, as defined hereinabove.

In one embodiment, this step of producing the extracellular vesicle or the population of extracellular vesicles comprises transfecting cells with the nucleic acid "*DNA*$^{S-EV}$" comprising (i) a sequence of a S gene coding for a Spike protein from a virus of the *Orthocoronavirinae* subfamily, or a variant thereof; and (ii) a sequence coding for a pilot peptide which interacts with ESCRT proteins.

In one embodiment, the cells are HEK293 cells or cells from a derivative cell line. In one embodiment, the cells are immune cells, including, but not limited to, mastocytes, lymphocytes (such as, *e.g.,* T-cells or B-cells), and dendritic cells.

In one embodiment, the method of obtaining an extracellular vesicle or a population of extracellular vesicles further comprises a step of culturing the transfected cells for a time sufficient to allow extracellular vesicle production, preferably in a medium devoid of EVs (*i.e.,* a serum-free medium or a medium supplemented with EVs-depleted serum).

In one embodiment, the method of obtaining an extracellular vesicle or a population of extracellular vesicles further comprises a step of purifying said extracellular vesicle or population of extracellular vesicles.

In one embodiment, the step of purifying said extracellular vesicle or population of extracellular vesicles comprises clarification (such as, *e.g.,* by centrifugation or by depth-filtration), filtration, ultra-filtration, diafiltration, size-exclusion purification and/or ion exchange chromatography of the transfected cell culture supernatant.

A further object of the present invention is a nucleic acid "*DNA*$^{S-Trim}$" comprising:

(i) a sequence of a *S* gene coding for a Spike protein from a virus of the *Orthocoronavirinae* subfamily, or a variant thereof; and
(ii) a sequence coding for a trimerization domain peptide.

In one embodiment, the virus of the *Orthocoronavirinae* subfamily is a virus of the *betacoronavirus* genus, as defined hereinabove. In one embodiment, the virus of the *Orthocoronavirinae* subfamily is a virus of the *betacoronavirus* genus, and severe acute respiratory syndrome-related coronavirus species, as defined hereinabove. In one embodiment, the virus of the *Orthocoronavirinae* subfamily is a virus of the *betacoronavirus* genus, severe acute respiratory syndrome-related coronavirus species and strain SARS-CoV-2, as defined hereinabove. In one embodiment, the virus of the *Orthocoronavirinae* subfamily is SARS-CoV-2, as defined hereinabove.

In one embodiment, the Spike protein of SARS-CoV-2 has an amino acid sequence with **SEQ ID NO: 2,** or a variant thereof, as defined hereinabove.

Variants of the Spike protein of SARS-CoV-2 have been defined hereabove in the context of the nucleic acid "*DNA*$^{S\text{-}EV}$", which applies here *mutatis mutandis.*

In one embodiment, the Spike protein is from a SARS-CoV-2 isolate other than "Wuhan-Hu-1".

In one embodiment, a fragment of the Spike protein of SARS-CoV-2 comprises at least 70%, preferably at least 75%, 80%, 85%, 90%, 95%, 96%, 97%, 98%, 99% or more contiguous amino acid residues of the amino acid sequence with **SEQ ID NO: 2.**

In one embodiment, a mutant of the Spike protein of SARS-CoV-2 comprises at least 70%, preferably at least 75%, 80%, 85%, 90%, 95%, 96%, 97%, 98%, 99% or more sequence identity, preferably local sequence identity with the amino acid sequence with **SEQ ID NO: 2.**

In one embodiment, a variant of the S gene of SARS-CoV-2 is a nucleic acid sequence with **SEQ ID NO: 11.** In one embodiment, a variant of the S gene of SARS-CoV-2 has a nucleic acid sequence coding for a Spike protein with **SEQ ID NO: 12.**

---

**SEQ ID NO: 11** – Modified *S* gene

ATGGTGCTGCTGCTGATCCTGTCTGTGCTGCTCCTGAAAGAAGATGTGCGGG
GCTCTGCCCAGAGCACCGGTCAATGTGTGAACCTGACCACCAGAACACAGC
TGCCTCCAGCCTACACCAACAGCTTCACCAGAGGCGTGTACTACCCCGACA
AGGTGTTCAGATCCAGCGTGCTGCACTCTACCCAGGACCTGTTCCTGCCTTT
CTTCAGCAACGTGACCTGGTTCCACGCCATCCACGTGTCCGGCACCAATGGC
ACCAAGAGATTCGACAACCCCGTGCTGCCCTTCAACGACGGGGTGTACTTT
GCCAGCACCGAGAAGTCCAACATCATCAGAGGCTGGATCTTCGGCACCACA
CTGGACAGCAAGACCCAGAGCCTGCTGATCGTGAACAACGCCACCAACGTG
GTCATCAAAGTGTGCGAGTTCCAGTTCTGCAACGACCCCTTCCTGGGCGTCT
ACTACCACAAGAACAACAAGAGCTGGATGGAAAGCGAGTTCCGGGTGTAC
AGCAGCGCCAACAACTGCACCTTCGAGTACGTGTCCCAGCCTTTCCTGATGG
ACCTGGAAGGCAAGCAGGGCAACTTCAAGAACCTGCGCGAGTTCGTGTTCA
AGAACATCGACGGCTACTTCAAGATCTACAGCAAGCACACCCCTATCAACC
TCGTGCGGGATCTGCCTCAGGGCTTCTCTGCTCTGGAACCCCTGGTGGATCT
GCCCATCGGCATCAACATCACCCGGTTTCAGACACTGCTGGCCCTGCACAG
AAGCTACCTGACACCTGGCGATAGCAGCTCTGGATGGACAGCTGGCGCCGC
TGCCTACTATGTGGGATACCTGCAGCCTCGGACCTTCCTGCTGAAGTACAAC
GAGAACGGCACCATCACCGACGCCGTGGATTGTGCTCTGGATCCTCTGAGC
GAGACAAAGTGCACCCTGAAGTCCTTCACCGTGGAAAAGGGCATCTACCAG

ACCAGCAACTTCCGGGTGCAGCCCACCGAATCCATCGTGCGGTTCCCCAAT
ATCACCAATCTGTGCCCCTTCGGCGAGGTGTTCAATGCCACCAGATTCGCCT
CTGTGTACGCCTGGAACCGGAAGCGGATCAGCAATTGCGTGGCCGACTACT
CCGTGCTGTACAACTCCGCCAGCTTCAGCACCTTCAAGTGCTACGGCGTGTC
CCCTACCAAGCTGAACGACCTGTGCTTCACAAACGTGTACGCCGACAGCTTC
GTGATCCGGGGAGATGAAGTGCGGCAGATTGCCCCTGGACAGACAGGCAA
GATCGCCGACTACAACTACAAGCTGCCCGACGACTTCACCGGCTGTGTGATT
GCCTGGAACAGCAACAACCTGGACTCCAAAGTCGGCGGCAACTACAATTAC
CTGTACCGGCTGTTCCGGAAGTCCAATCTGAAGCCCTTCGAGCGGGACATCT
CCACCGAGATCTATCAGGCCGGCAGCACCCCTTGTAACGGCGTGGAAGGCT
TCAACTGCTACTTCCCACTGCAGTCCTACGGCTTTCAGCCCACAAATGGCGT
GGGCTACCAGCCTTACAGAGTGGTGGTGCTGAGCTTCGAGCTGCTGCATGCT
CCTGCCACAGTGTGCGGCCCTAAGAAAAGCACCAATCTCGTGAAGAACAAA
TGCGTGAACTTCAACTTCAACGGCCTGACCGGCACCGGCGTGCTGACAGAG
AGCAACAAGAAGTTCCTGCCATTCCAGCAGTTCGGCCGGGATATCGCCGAT
ACCACAGATGCCGTCAGAGATCCCCAGACACTGGAAATCCTGGACATCACC
CCTTGCAGCTTCGGCGGAGTGTCTGTGATCACCCCTGGCACCAACACCAGCA
ATCAGGTGGCAGTGCTGTACCAGGACGTGAACTGTACCGAAGTGCCCGTGG
CCATTCACGCCGATCAGCTGACACCTACATGGCGGGTGTACTCCACCGGCA
GCAATGTGTTTCAGACCAGAGCCGGCTGTCTGATCGGAGCCGAGCACGTGA
ACAATAGCTACGAGTGCGACATCCCCATCGGCGCTGGCATCTGCGCCTCTTA
CCAGACACAGACAAACAGCCCCAGACGGGCCAGATCTGTGGCCAGCCAGA
GCATCATTGCCTACACAATGTCTCTGGGCGCCGAGAACAGCGTGGCCTACTC
CAACAACTCTATCGCTATCCCCACCAACTTCACCATCAGCGTGACCACAGAG
ATCCTGCCTGTGTCCATGACCAAGACCAGCGTGGACTGCACCATGTACATCT
GCGGCGATTCCACCGAGTGCTCCAACCTGCTGCTGCAGTACGGCAGCTTCTG
CACCCAGCTGAATAGAGCCCTGACAGGGATCGCCGTGGAACAGGATGCCAA
TACCGGCGAAGTGTTCGCCCAAGTGAAGCAGATCTACAAGACCCCTCCTAT
CAAGGACTTCGGCGGCTTCAATTTCAGCCAGATTCTGCCCGATCCTAGCAAG
CCCAGCAAGCGGAGCTTCATCGAGGACCTGCTGTTCAACAAAGTGACACTG
GCCGACGCCGGCTTCATCAAGCAGTATGGCGATTGTCTGGGCGACATTGCC
GCCAGGGATCTGATTTGCGCCCAGAAGTTTAACGGACTGACAGTGCTGCCT

CCTCTGCTGACCGATGAGATGATCGCCCAGTACACATCTGCCCTGCTGGCCG
GCACAATCACAAGCGGCTGGACATTTGGAGCTGGCGCTGCCCTGCAGATCC
CCTTTGCTATGCAGATGGCCTACAGATTCAACGGCATCGGAGTGACCCAGA
ATGTGCTGTACGAGAACCAGAAGCTGATCGCCAACCAGTTCAACAGCGCCA
TCGGCAAGATCCAGGACAGCCTGTCTAGCACAGCCAGCGCTCTGGGAAAGC
TGCAGGACGTGGTCAACCAGAATGCCCAGGCACTGAACACCCTGGTCAAGC
AGCTGAGCAGCAATTTCGGCGCCATCAGCTCTGTGCTGAACGATATCCTGA
GCAGACTGGACCCTCCTGAGGCCGAGGTGCAGATCGACAGACTGATCACCG
GAAGGCTGCAGTCCCTGCAGACCTACGTTACCCAGCAGCTGATCAGAGCCG
CCGAGATTAGAGCCTCTGCCAATCTGGCCGCCACCAAGATGTCTGAGTGTGT
GCTGGGCCAGAGCAAGAGAGTGGACTTTTGCGGCAAGGGCTACCACCTGAT
GAGCTTCCCTCAGTCTGCCCCTCACGGCGTGGTGTTTCTGCACGTGACATAC
GTGCCCGCTCAAGAGAAGAATTTCACCACCGCTCCAGCCATCTGCCACGAC
GGCAAAGCCCACTTTCCTAGAGAAGGCGTGTTCGTGTCCAACGGCACCCAT
TGGTTCGTGACCCAGCGGAACTTCTACGAGCCCCAGATCATCACCACCGAC
AACACCTTCGTGTCTGGCAACTGCGACGTCGTGATCGGCATTGTGAACAATA
CCGTGTACGACCCTCTGCAGCCCGAGCTGGACAGCTTCAAAGAGGAACTGG
ATAAGTACTTTAAGAACCACACAAGCCCCGACGTGGACCTGGGCGATATCA
GCGGAATCAATGCCAGCGTCGTGAACATCCAGAAAGAGATCGACCGGCTGA
ACGAGGTGGCCAAGAATCTGAACGAGAGCCTGATCGACCTGCAGGAACTGG
GGAAGTACGAGCAG

**SEQ ID NO: 12** – Modified Spike protein encoded by SEQ ID NO: 11
MVLLLILSVLLLKEDVRGSAQSTGQCVNLTTRTQLPPAYTNSFTRGVYYPDKVF
RSSVLHSTQDLFLPFFSNVTWFHAIHVSGTNGTKRFDNPVLPFNDGVYFASTEK
SNIIRGWIFGTTLDSKTQSLLIVNNATNVVIKVCEFQFCNDPFLGVYYHKNNKS
WMESEFRVYSSANNCTFEYVSQPFLMDLEGKQGNFKNLREFVFKNIDGYFKIY
SKHTPINLVRDLPQGFSALEPLVDLPIGINITRFQTLLALHRSYLTPGDSSSGWTA
GAAAYYVGYLQPRTFLLKYNENGTITDAVDCALDPLSETKCTLKSFTVEKGIYQ
TSNFRVQPTESIVRFPNITNLCPFGEVFNATRFASVYAWNRKRISNCVADYSVLY
NSASFSTFKCYGVSPTKLNDLCFTNVYADSFVIRGDEVRQIAPGQTGKIADYNY

KLPDDFTGCVIAWNSNNLDSKVGGNYNYLYRLFRKSNLKPFERDISTEIYQAGS
TPCNGVEGFNCYFPLQSYGFQPTNGVGYQPYRVVVLSFELLHAPATVCGPKKS
TNLVKNKCVNFNFNGLTGTGVLTESNKKFLPFQQFGRDIADTTDAVRDPQTLEI
LDITPCSFGGVSVITPGTNTSNQVAVLYQDVNCTEVPVAIHADQLTPTWRVYST
GSNVFQTRAGCLIGAEHVNNSYECDIPIGAGICASYQTQTNSPRRARSVASQSIIA
YTMSLGAENSVAYSNNSIAIPTNFTISVTTEILPVSMTKTSVDCTMYICGDSTECS
NLLLQYGSFCTQLNRALTGIAVEQDANTGEVFAQVKQIYKTPPIKDFGGFNFSQI
LPDPSKPSKRSFIEDLLFNKVTLADAGFIKQYGDCLGDIAARDLICAQKFNGLTV
LPPLLTDEMIAQYTSALLAGTITSGWTFGAGAALQIPFAMQMAYRFNGIGVTQN
VLYENQKLIANQFNSAIGKIQDSLSSTASALGKLQDVVNQNAQALNTLVKQLSS
NFGAISSVLNDILSRLDPPEAEVQIDRLITGRLQSLQTYVTQQLIRAAEIRASANL
AATKMSECVLGQSKRVDFCGKGYHLMSFPQSAPHGVVFLHVTYVPAQEKNFT
TAPAICHDGKAHFPREGVFVSNGTHWFVTQRNFYEPQIITTDNTFVSGNCDVVI
GIVNNTVYDPLQPELDSFKEELDKYFKNHTSPDVDLGDISGINASVVNIQKEIDR
LNEVAKNLNESLIDLQELGKYEQ

In one embodiment, the trimerization domain peptide is selected from the group comprising or consisting of the T4-fibritin "Foldon" trimerization domain (Meier et al., 2004. J Mol Biol. 344(4):1051-1069; Güthe et al., 2004. J Mol Biol. 337(4):905-915), the GCN4-based isoleucine zipper (Drees et al., 1997. J Mol Biol. 273(1):61-74; Yang et al., 2000. J Virol. 74(12):5716-5725; Yang et al., 2002. J Virol. 76(9):4634-4642), the catalytic subunit of *Escherichia coli* aspartate transcarbamoylase (Chen et al., 2004. J Virol. 78(9):4508-4516), the HIV-1 gp41 trimerization domain (Shu et al., 1999. Biochemistry. 38(17):5378-5385), the SIV gp41 trimerization domain (Blacklow et al., 1995. Biochemistry. 34(46): 14955-14962), the Ebola virus gp-2 trimerization domain, the HTLV-1 gp-21 trimerization domain, the yeast heat shock transcription factor trimerization domain, and the human collagen trimerization domain.

In one embodiment, the trimerization domain peptide is the T4-fibritin "Foldon" trimerization domain. In one embodiment, the sequence coding for a trimerization domain peptide is a sequence coding for the T4-fibritin "Foldon" trimerization domain.

In one embodiment, the T4-fibritin "Foldon" trimerization domain has an amino acid sequence with **SEQ ID NO: 45.**

**SEQ ID NO: 45:** GYIPEAPRDGQAYVRKDGEWVLLSTFL

In one embodiment, the sequence coding for a trimerization domain peptide further codes for a cleavage site upstream of the trimerization domain peptide (*i.e.,* in N-terminal position of the trimerization domain peptide).

In one embodiment, the protease is an endopeptidase. In one embodiment, the protease, preferably the endopeptidase, is a cysteine protease or a serine protease.

In one embodiment, the protease is selected from the group comprising or consisting of tobacco etch virus (TEV) protease, tobacco vein mottling virus (TVMV) protease, sugarcane mosaic virus (SMV) protease, hepatitis C virus (HCV) protease, pepsin, trypsin, chymotrypsin, thermolysin, subtilisin, papain, elastase, and plasminogen.

In one embodiment, the protease is TEV protease.

In one embodiment, the sequence coding for a trimerization domain peptide further codes for a purification tag downstream of the trimerization domain peptide *(i.e.,* in C-terminal position of the trimerization domain peptide).

Examples of purification tags include, but are not limited to, poly-histidine tags (such as, *e.g.,* 6×His or 10×His tags), avidin, FLAG-tag, glutathione S-transferase (GST) tag, maltose-binding protein (MBP) tag, chitin-binding protein (CBP) tag, V5-tag, Myc-tag, and HA-tags.

In one embodiment, the purification tag is a 6×His tag.

In one embodiment, the sequence coding for the trimerization domain peptide has a nucleic acid sequence coding for a cleavage site, a linker, a trimerization domain peptide and a purification tag, with an amino acid sequence with **SEQ ID NO: 14.** In one embodiment, the sequence coding for the trimerization domain peptide has a nucleic acid sequence with **SEQ ID NO: 13.**

**SEQ ID NO: 13** – Trimerization foldon

AGCGGCAGAGAGAACCTGTACTTCCAAGGCGGAGGCGGCGGAAGCGGCTA
TATTCCTGAAGCTCCTAGAGATGGCCAGGCCTACGTGCGGAAAGATGGCGA
ATGGGTCCTGCTGAGCACCTTTCTGGGACACCACCACCATCACCATTAGTAA

**SEQ ID NO: 14** - Trimerization foldon encoded by SEQ ID NO: 13 SGRENLYFQGGGGGSGYIPEAPRDGQAYVRKDGEWVLLSTFLGHHHHHH

A further object of the present invention is a trimeric Spike protein from a virus of the *Orthocoronavirinae* subfamily *"S-Trim",* or a variant thereof, as defined hereinabove.

In one embodiment, the virus of the *Orthocoronavirinae* subfamily is a virus of the *betacoronavirus* genus, as defined hereinabove. In one embodiment, the virus of the *Orthocoronavirinae* subfamily is a virus of the *betacoronavirus* genus, and severe acute respiratory syndrome-related coronavirus species, as defined hereinabove. In one embodiment, the virus of the *Orthocoronavirinae* subfamily is a virus of the *betacoronavirus* genus, severe acute respiratory syndrome-related coronavirus species and strain SARS-CoV-2, as defined hereinabove. In one embodiment, the virus of the *Orthocoronavirinae* subfamily is SARS-CoV-2, as defined hereinabove.

In one embodiment, each monomer of the trimeric Spike protein of SARS-CoV-2 has an amino acid sequence with **SEQ ID NO: 2,** or a variant thereof, as defined hereinabove.

Variants of the Spike protein of SARS-CoV-2 have been defined hereabove in the context of the nucleic acid "$DNA^{S\text{-}EV}$", which applies here *mutatis mutandis.*

In one embodiment, the Spike protein is from a SARS-CoV-2 isolate other than "Wuhan-Hu-1".

In one embodiment, a fragment of the Spike protein of SARS-CoV-2 comprises at least 70%, preferably at least 75%, 80%, 85%, 90%, 95%, 96%, 97%, 98%, 99% or more contiguous amino acid residues of the amino acid sequence with **SEQ ID NO: 2.**

In one embodiment, a fragment of the Spike protein of SARS-CoV-2 retains at least part or the entirety of its cytoplasmic domain, and the entirety of its transmembrane domain.

In one embodiment, a mutant of the Spike protein of SARS-CoV-2 comprises at least 70%, preferably at least 75%, 80%, 85%, 90%, 95%, 96%, 97%, 98%, 99% or more sequence identity, preferably local sequence identity with the amino acid sequence with **SEQ ID NO: 2.**

In one embodiment, a variant of a monomer of the trimeric Spike protein of SARS-CoV-2 comprises or consists of the amino acid sequence with **SEQ ID NO: 12.**

In one embodiment, a variant of a monomer of the trimeric Spike protein of SARS-CoV-2 comprises or consists of the amino acid sequences with **SEQ ID NO: 12** and **SEQ ID NO: 14.** In one embodiment, a variant of a monomer of the trimeric Spike protein of SARS-CoV-2 comprises or consists, for N- to C-terminal, of the amino acid sequence with **SEQ ID NO: 12** and the amino acid sequence with **SEQ ID NO: 14.**

In one embodiment, the trimeric Spike protein is isolated or otherwise purified.

A further object of the present invention is a method of obtaining a trimeric Spike protein from a virus of the *Orthocoronavirinae* subfamily *"S-Trim",* or a variant thereof, as defined hereinabove.

In one embodiment, the method of obtaining a trimeric Spike protein comprises a step of producing the trimeric Spike protein, as defined hereinabove.

In one embodiment, this step of producing the trimeric Spike protein comprises transfecting cells with the nucleic acid "$DNA^{S\text{-}Trim}$" comprising (i) a sequence of a S gene coding for a Spike protein from a virus of the Orthocoronavirinae subfamily, or a variant thereof, and (ii) a sequence coding for a trimerization domain peptide.

In one embodiment, the cells are HEK293 cells or cells from a derivative cell line.

In one embodiment, the method of obtaining a trimeric Spike protein further comprises a step of culturing the transfected cells for a time sufficient to allow Spike protein production.

In one embodiment, the method of obtaining a trimeric Spike protein further comprises a step of purifying said trimeric Spike protein.

In one embodiment, the step of purifying said trimeric Spike protein comprises lysing host cells, centrifuging and/or affinity purifying.

In particular, where the sequence coding for a trimerization domain peptide further codes for a purification tag in the nucleic acid "$DNA^{S\text{-}Trim}$", this purification tag can serve for affinity purifying.

A further object of the invention is a composition comprising a nucleic acid "$DNA^{S\text{-}EV}$" comprising:

(i) a sequence of a S gene coding for a Spike protein from a virus of the *Orthocoronavirinae* subfamily, or a variant thereof; and
(ii) a sequence coding for a pilot peptide which interacts with ESCRT proteins as defined hereinabove.

A further object of the invention is a composition comprising an extracellular vesicle harboring at its external surface a Spike protein from a virus of the *Orthocoronavirinae* subfamily "*S-EV*", or a variant thereof, as defined hereinabove.
A further object of the invention is a composition comprising a population of extracellular vesicles harboring at their external surface a Spike protein from a virus of the *Orthocoronavirinae* subfamily "*S-EV*", or a variant thereof, as defined hereinabove.
A further object of the invention is a composition comprising a nucleic acid "$DNA^{S\text{-}Trim}$" comprising:

(i) a sequence of a S gene coding for a Spike protein from a virus of the *Orthocoronavirinae* subfamily, or a variant thereof; and
(ii) a sequence coding for a trimerization domain peptide,

as defined hereinabove.
A further object of the invention is a composition comprising a trimeric Spike protein from a virus of the *Orthocoronavirinae* subfamily *"S-Trim",* or a variant thereof, as defined hereinabove.
In one embodiment, the compositions according to the present invention are pharmaceutical composition and further comprise at least one pharmaceutically acceptable excipient.
The term **"pharmaceutically acceptable excipient"** includes any and all solvents, diluents, dispersion media, coatings, antibacterial and antifungal agents, isotonic and absorption delaying agents and the like. Said excipient does not produce an adverse, allergic or other untoward reaction when administered to an animal, preferably a human. For human administration, preparations should meet sterility, pyrogenicity, and general safety and purity standards as required by regulatory offices, such as, for example, FDA Office or EMA.
Pharmaceutically acceptable excipients that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of vegetable oil saturated fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.
In one embodiment, the pharmaceutical compositions comprise vehicles which are pharmaceutically acceptable for a formulation capable of being injected to a subject. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.
In one embodiment, the compositions according to the present invention do not comprise any adjuvant.
Examples of adjuvants include, but are not limited to, cholera toxin, *Escherichia coli* heat-labile enterotoxin (LT), liposome, immune-stimulating complex (ISCOM), immunostimulatory sequences oligodeoxynucleotide (ISS-ODN), aluminum salts (such as, *e.g.*, aluminum hydroxide or aluminum phosphate), Freund's complete adjuvant, Freund's incomplete adjuvant, Ribi solution, *Corynebacterium parvum,* Bacillus Calmette-Guerin (BCG), glucan, dextran sulfate, iron oxide, sodium alginate, and muramyl peptides.
A further object is a kit-of-parts comprising:

(i) a nucleic acid "$DNA^{S\text{-}EV}$" comprising a sequence of a *S* gene coding for a Spike protein from a virus of the *Orthocoronavirinae* subfamily, or a variant thereof; and a sequence coding for a pilot peptide which interacts with ESCRT proteins, as defined hereinabove; or a composition or pharmaceutical composition comprising the same; and
(ii) either of:

a. an extracellular vesicle harboring at its external surface a Spike protein from a virus of the *Orthocoronavirinae* subfamily "*S-EV*", or a variant thereof, as defined hereinabove; or a composition or pharmaceutical composition comprising the same;
b. a population of extracellular vesicles harboring at their external surface a Spike protein from a virus of the *Orthocoronavirinae* subfamily "*S-EV*", or a variant thereof, as defined hereinabove; or a composition or pharmaceutical composition comprising the same; or
c. a trimeric Spike protein from a virus of the *Orthocoronavirinae* subfamily *"S-Trim",* or a variant thereof, as defined hereinabove; or a composition or pharmaceutical composition comprising the same.

A further object of the present invention is a method of immunizing a subject against a virus of the *Orthocoronavirinae* subfamily.

In one embodiment, the virus of the *Orthocoronavirinae* subfamily is a virus of the *betacoronavirus* genus, as defined hereinabove. In one embodiment, the virus of the *Orthocoronavirinae* subfamily is a virus of the *betacoronavirus* genus, and severe acute respiratory syndrome-related coronavirus species, as defined hereinabove. In one embodiment, the virus of the *Orthocoronavirinae* subfamily is a virus of the *betacoronavirus* genus, severe acute respiratory syndrome-related coronavirus species and strain SARS-CoV-2, as defined hereinabove. In one embodiment, the virus of the *Orthocoronavirinae* subfamily is SARS-CoV-2, as defined hereinabove.

In one embodiment, the method of immunizing a subject against a virus of the *Orthocoronavirinae* subfamily comprises a first step of administering to said subject a nucleic acid "$DNA^{S\text{-}EV}$" comprising a sequence of a *S* gene coding for a Spike protein from a virus of the *Orthocoronavirinae* subfamily, or a variant thereof; and a sequence coding for a pilot peptide which interacts with ESCRT proteins, as defined hereinabove; or a composition or pharmaceutical composition comprising the same.

Means and methods for administering a nucleic acid to a subject (*i.e.*, for DNA vaccine delivery) are well known in the art. Any convenient and appropriate method of delivery of the nucleic acid may be utilized. These include, *e.g.*, delivery with cationic lipids (Goddard et al., 1997. Gene Ther. 4(11): 1231-1236; Gorman et al., 1997. Gene Ther. 4(9):983-992; Chadwick et al., 1997. Gene Ther. 4(9):937-942; Gokhale et al., 1997. Gene Ther. 4(12):1289-1299; Gao & Huang, 1995. Gene Ther. 2(10):710-722), delivery by uptake of naked nucleic acid, and the like. Method of delivery of the nucleic acid can further include or be enhanced by electroporation, particle bombardment, sonoporation, magnetofection, hydrodynamic delivery and the like. Method of delivery of the nucleic acid can further include or be enhanced by the use of chemicals including, but not limited to, nucleic acid specifically modified to enhance delivery, lipoplexes, polymersomes, polyplexes, dendrimers, nanoparticles (*e.g.*, inorganic nanoparticles), cell-penetrating peptides, cell-penetrating proteins (*e.g.*, supercharged proteins), and the like.

In one embodiment, the method of immunizing a subject against a virus of the *Orthocoronavirinae* subfamily comprises a second step of administering to said subject either of:

a. an extracellular vesicle harboring at its external surface a Spike protein from a virus of the *Orthocoronavirinae* subfamily "*S-EV*", or a variant thereof, as defined hereinabove; or a composition or pharmaceutical composition comprising the same;
b. a population of extracellular vesicles harboring at their external surface a Spike protein from a virus of the *Orthocoronavirinae* subfamily "*S-EV*", or a variant thereof, as defined hereinabove; or a composition or pharmaceutical composition comprising the same; or
c. a trimeric Spike protein from a virus of the *Orthocoronavirinae* subfamily *"S-Trim",* or a variant thereof, as defined hereinabove; or a composition or pharmaceutical composition comprising the same.

In one embodiment, the method of immunizing a subject against a virus of the *Orthocoronavirinae* subfamily comprises:

1) a first step, termed *"priming step",* of administering to said subject a nucleic acid "$DNA^{S\text{-}EV}$" comprising a sequence of a *S* gene coding for a Spike protein from a virus of the *Orthocoronavirinae* subfamily, or a variant thereof; and a sequence coding for a pilot peptide which interacts with ESCRT proteins, as defined hereinabove; or a composition or pharmaceutical composition comprising the same; and
2) a second step, termed *"boosting step",* of administering to said subject either of:

   a. an extracellular vesicle harboring at its external surface a Spike protein from a virus of the *Orthocoronavirinae* subfamily "*S-EV*", or a variant thereof, as defined hereinabove; or a composition or pharmaceutical composition comprising the same;
   b. a population of extracellular vesicles harboring at their external surface a Spike protein from a virus of the *Orthocoronavirinae* subfamily "*S-EV*", or a variant thereof, as defined hereinabove; or a composition or pharmaceutical composition comprising the same; or
   c. a trimeric Spike protein from a virus of the *Orthocoronavirinae* subfamily *"S-Trim",* or a variant thereof, as defined hereinabove; or a composition or pharmaceutical composition comprising the same.

In one embodiment, the nucleic acid "$DNA^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same are formulated for administration to the subj ect.

In one embodiment, the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same is/are to be administered systemically or locally.

In one embodiment, the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same is/are to be administered by injection, orally, topically, nasally, buccally, rectally, vaginaly, intratracheally, by endoscopy, transmucosally, or by percutaneous administration.

In one embodiment, the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same is/are to be injected, preferably systemically injected.

Examples of formulations adapted for injection include, but are not limited to, solutions, such as, for example, sterile aqueous solutions, gels, dispersions, emulsions, suspensions, solid forms suitable for using to prepare solutions or suspensions upon the addition of a liquid prior to use, such as, for example, powder, liposomal forms and the like.

Examples of systemic injections include, but are not limited to, intravenous (iv) injection, subcutaneous injection, intramuscular (im) injection, intradermal (id) injection, intraperitoneal (ip), intranasal (in) injection and perfusion.

It will be understood that other suitable routes of administration are also contemplated in the present invention, and the administration mode will ultimately be decided by the attending physician within the scope of sound medical judgment.

In one embodiment, the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same is/are to be administered to the subject in need thereof in a therapeutically effective amount.

The term **"therapeutically effective amount"** or **"therapeutically effective dose",** as used herein, refers to an amount effective, at dosages and for periods of time necessary, to achieve a desired preventive result. In particular, a therapeutically effective amount refers to an amount effective, at dosages and for periods of time necessary, to achieve (1) a humoral immune response as can be determined, *e.g.*, by increased antibody titers against the "head" S1 and the "stem" S2 subunits of the Spike protein from the virus of the *Orthocoronavirinae* subfamily, or a variant thereof; and/or (2) a T-cell mediated immunity for the "head" S1 and the "stem" S2 subunits of the Spike protein from the virus of the *Orthocoronavirinae* subfamily, or a variant thereof; and/or (3) "head" S 1 and "stem" S2 subunits-specific interferon (IFN)-$\gamma$ production.

It will be however understood that the dosage of the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same will be decided by the attending physician within the scope of sound medical judgment. The specific therapeutically effective dose for any particular patient will depend upon a variety of factors including the disease being prevented and the severity of the disease; the activity of the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same employed; the age, body weight, general health, sex and diet of the subject; the time of administration, route of administration, and rate of excretion of the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same employed; the duration and regimen of the treatment; drugs used in combination or coincidental with the nucleic acid "*DNA*$^{S\text{-}EV}$" or the composition or pharmaceutical composition comprising the same, the extracellular vesicle "*S-EV*" or the population of extracellular vesicles "*S-EV*" or the composition or pharmaceutical composition comprising the same, and/or the trimeric Spike protein *"S-Trim"* or the composition or pharmaceutical composition comprising the same employed; and like factors well known in the medical arts.

In one embodiment, the *"priming step"* is to be carried out once, twice, three times, four times or more. In one embodiment, the *"boosting step"* is to be carried out once, twice, three times, four times or more.

In one embodiment, the *"priming step"* is to be carried out twice. In one embodiment, the *"boosting step"* is to be carried out once.

In one embodiment, the *"priming step"* is to be carried out twice and the *"boosting step"* is to be carried out once.

In one embodiment, the period of time between each iteration of the *"priming step"* and/or between each iteration of the *"boosting step"* and/or between the *"priming step"* and the *"boosting step"* range from about 1 day to about 6 months, preferably from about 1 week to about 3 months, more preferably from about 2 weeks to about 1 month.

In one embodiment, the period of time between each iteration of the *"priming step"* and/or between each iteration of the *"boosting step"* and/or between the *"priming step"* and the *"boosting step"* is about 1 day, 2 days, 3 days, 4 days, 5 days, 6 days, 1 week, 2 weeks, 3 weeks, 1 month, 2 months, 3 months, 4 months, 5 months, or 6 months.

In one embodiment, the period of time between each iteration of the *"priming step"* and/or between each iteration of the *"boosting step"* and/or between the *"priming step"* and the *"boosting step"* is about 3 weeks.

Therapeutically effective human doses can be calculated from animal doses, by converting these doses to Human Equivalent Doses (HED) based on body surface area (BSA). According to the FDA "Guidance for Industry" of July 2005, the BSA of a human with a body weight of 60 kg is about 1.62 $m^2$, while the BSA of a mouse (such as, ***e.g.,*** Balb/c mice used in the experimental design described below) with a body weight of 0.020 kg is about 0.007 $m^2$. To convert mouse dose in μg/kg to HED in μg/kg, the mouse dose can be divided by 12.3 (or alternatively multiplied by 0.081).

Additionally or alternatively, one skilled in the art is well aware that therapeutically effective human doses of nucleic acids for DNA vaccination typically range between about 0.1 mg and about 6 mg of nucleic acid per administration, with some studies describing doses up to about 10 mg of nucleic acid per administration.

In one embodiment, a therapeutically effective human dose of the nucleic acid "$DNA^{S-EV}$" or the composition or pharmaceutical composition comprising the same to be administered during the - or each one of the - *"priming step"* ranges from about 0.1 μg/kg to about 50 μg/kg of nucleic acid.

In one embodiment, a therapeutically effective human dose of the nucleic acid "$DNA^{S-EV}$" or the composition or pharmaceutical composition comprising the same to be administered during the - or each one of the - *"priming step"* is about 0.1 μg/kg ± 0.05 μg/kg, 0.2 μg/kg ± 0.05 μg/kg, 0.3 μg/kg ± 0.05 μg/kg, 0.4 μg/kg ± 0.05 μg/kg, 0.5 μg/kg ± 0.05 μg/kg, 0.6 μg/kg ± 0.05 μg/kg, 0.7 μg/kg ± 0.05 μg/kg, 0.8 μg/kg ± 0.05 μg/kg, 0.9 μg/kg ± 0.05 μg/kg, 1 μg/kg ± 0.5 μg/kg, 2 μg/kg ± 0.5 μg/kg, 3 μg/kg ± 0.5 μg/kg, 4 μg/kg ± 0.5 μg/kg, 5 μg/kg ± 0.5 μg/kg, 6 μg/kg ± 0.5 μg/kg, 7 μg/kg ± 0.5 μg/kg, 8 μg/kg ± 0.5 μg/kg, 9 μg/kg ± 0.5 μg/kg, 10 μg/kg ± 0.5 μg/kg, 11 μg/kg ± 0.5 μg/kg, 12 μg/kg ± 0.5 μg/kg, 13 μg/kg ± 0.5 μg/kg, 14 μg/kg ± 0.5 μg/kg, 15 μg/kg ± 0.5 μg/kg, 16 μg/kg ± 0.5 μg/kg, 17 μg/kg ± 0.5 μg/kg, 18 μg/kg ± 0.5 μg/kg, 19 μg/kg ± 0.5 μg/kg, 20 μg/kg ± 0.5 μg/kg, 21 μg/kg ± 0.5 μg/kg, 22 μg/kg ± 0.5 μg/kg, 23 μg/kg ± 0.5 μg/kg, 24 μg/kg ± 0.5 μg/kg, 25 μg/kg ± 0.5 μg/kg, 26 μg/kg ± 0.5 μg/kg, 27 μg/kg ± 0.5 μg/kg, 28 μg/kg ± 0.5 μg/kg, 29 μg/kg ± 0.5 μg/kg, 30 μg/kg ± 0.5 μg/kg, 31 μg/kg ± 0.5 μg/kg, 32 μg/kg ± 0.5 μg/kg, 33 μg/kg ± 0.5 μg/kg, 34 μg/kg ± 0.5 μg/kg, 35 μg/kg ± 0.5 μg/kg, 36 μg/kg ± 0.5 μg/kg, 37 μg/kg ± 0.5 μg/kg, 38 μg/kg ± 0.5 μg/kg, 39 μg/kg ± 0.5 μg/kg, 40 μg/kg ± 0.5 μg/kg, 41 μg/kg ± 0.5 μg/kg, 42 μg/kg ± 0.5 μg/kg, 43 μg/kg ± 0.5 μg/kg, 44 μg/kg ± 0.5 μg/kg, 45 μg/kg ± 0.5 μg/kg, 46 μg/kg ± 0.5 μg/kg, 47 μg/kg ± 0.5 μg/kg, 48 μg/kg ± 0.5 μg/kg, 49 μg/kg ± 0.5 μg/kg, or 50 μg/kg ± 0.5 μg/kg of nucleic acid.

In one embodiment, a therapeutically effective human dose of the extracellular vesicle "*S-EV*" or the population of extracellular vesicles "*S-EV*" or the composition or pharmaceutical composition comprising the same to be administered during the - or each one of the - *"boosting step"* ranges from about 0.1 μg/kg to about 50 μg/kg of extracellular vesicle.

In one embodiment, a therapeutically effective human doseof the extracellular vesicle "*S-EV*" or the population of extracellular vesicles "*S-EV*" or the composition or pharmaceutical composition comprising the same to be administered during the - or each one of the - *"boosting step"* is about 0.1 μg/kg ± 0.05 μg/kg, 0.2 μg/kg ± 0.05 μg/kg, 0.3 μg/kg ± 0.05 μg/kg, 0.4 μg/kg ± 0.05 μg/kg, 0.5 μg/kg ± 0.05 μg/kg, 0.6 μg/kg ± 0.05 μg/kg, 0.7 μg/kg ± 0.05 μg/kg, 0.8 μg/kg ± 0.05 μg/kg, 0.9 μg/kg ± 0.05 μg/kg, 1 μg/kg ± 0.5 μg/kg, 2 μg/kg ± 0.5 μg/kg, 3 μg/kg ± 0.5 μg/kg, 4 μg/kg ± 0.5 μg/kg, 5 μg/kg ± 0.5 μg/kg, 6 μg/kg ± 0.5 μg/kg, 7 μg/kg ± 0.5 μg/kg, 8 μg/kg ± 0.5 μg/kg, 9 μg/kg ± 0.5 μg/kg, 10 μg/kg ± 0.5 μg/kg, 11 μg/kg ± 0.5 μg/kg, 12 μg/kg ± 0.5 μg/kg, 13 μg/kg ± 0.5 μg/kg, 14 μg/kg ± 0.5 μg/kg, 15 μg/kg ± 0.5 μg/kg, 16 μg/kg ± 0.5 μg/kg, 17 μg/kg ± 0.5 μg/kg, 18 μg/kg ± 0.5 μg/kg, 19 μg/kg ± 0.5 μg/kg, 20 μg/kg ± 0.5 μg/kg, 21 μg/kg ± 0.5 μg/kg, 22 μg/kg ± 0.5 μg/kg, 23 μg/kg ± 0.5 μg/kg, 24 μg/kg ± 0.5 μg/kg, 25 μg/kg ± 0.5 μg/kg, 26 μg/kg ± 0.5 μg/kg, 27 μg/kg ± 0.5 μg/kg, 28 μg/kg ± 0.5 μg/kg, 29 μg/kg ± 0.5 μg/kg, 30 μg/kg ± 0.5 μg/kg, 31 μg/kg ± 0.5 μg/kg, 32 μg/kg ± 0.5 μg/kg, 33 μg/kg ± 0.5 μg/kg, 34 μg/kg ± 0.5 μg/kg, 35 μg/kg ± 0.5 μg/kg, 36 μg/kg ± 0.5 μg/kg, 37 μg/kg ± 0.5 μg/kg, 38 μg/kg ± 0.5 μg/kg, 39 μg/kg ± 0.5 μg/kg, 40 μg/kg ± 0.5 μg/kg, 41 μg/kg ± 0.5 μg/kg, 42 μg/kg ± 0.5 μg/kg, 43 μg/kg ± 0.5 μg/kg, 44 μg/kg ± 0.5 μg/kg, 45 μg/kg ± 0.5 μg/kg, 46 μg/kg ± 0.5 μg/kg, 47 μg/kg ± 0.5 μg/kg, 48 μg/kg ± 0.5 μg/kg, 49 μg/kg ± 0.5 μg/kg, 50 μg/kg ± 0.5 μg/kg, of extracellular vesicle.

A further object of the present invention is an isolated peptide targeting the membrane proximal external region (MPER) subdomain of the Spike protein from a virus of the *Orthocoronavirinae* subfamily, or a variant thereof, as defined hereinabove.

In one embodiment, the virus of the *Orthocoronavirinae* subfamily is a virus of the *betacoronavirus* genus, as defined hereinabove. In one embodiment, the virus of the *Orthocoronavirinae* subfamily is a virus of the *betacoronavirus* genus, and severe acute respiratory syndrome-related coronavirus species, as defined hereinabove. In one embodiment, the virus of the *Orthocoronavirinae* subfamily is a virus of the *betacoronavirus* genus, severe acute respiratory syndrome-related coronavirus species and strain SARS-CoV-2, as defined hereinabove. In one embodiment, the virus of the *Orthocoronavirinae* subfamily is SARS-CoV-2, as defined hereinabove.

In one embodiment, the Spike protein of SARS-CoV-2 has an amino acid sequence with **SEQ ID NO: 2,** or a variant thereof, as defined hereinabove.

Variants of the Spike protein of SARS-CoV-2 have been defined hereabove in the context of the nucleic acid "*DNA*$^{S\text{-}EV}$", which applies here *mutatis mutandis.*

In one embodiment, the Spike protein is from a SARS-CoV-2 isolate other than "Wuhan-Hu-1" .

In one embodiment, a fragment of the Spike protein of SARS-CoV-2 comprises at least 70%, preferably at least 75%, 80%, 85%, 90%, 95%, 96%, 97%, 98%, 99% or more contiguous amino acid residues of the amino acid sequence with SEQ **ID NO: 2.**

In one embodiment, a mutant of the Spike protein of SARS-CoV-2 comprises at least 70%, preferably at least 75%, 80%, 85%, 90%, 95%, 96%, 97%, 98%, 99% or more sequence identity, preferably local sequence identity with the amino acid sequence with **SEQ ID NO: 2.**

In one embodiment, the isolated peptide targeting the MPER subdomain of the Spike protein comprises or consists of an amino acid sequence comprising or consisting of the C-terminal part of the extracellular domain of said Spike protein. The extracellular domain of the Spike protein of SARS-CoV-2 with **SEQ ID NO: 2** comprises amino acid residues 13 to 1217 of **SEQ ID NO: 2.**

In one embodiment, the isolated peptide targeting the MPER subdomain of the Spike protein comprises or consists of an amino acid sequence comprising or consisting of 5 to 50 amino acid residues of the C-terminal part of the extracellular domain of said Spike protein, such as 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 amino acid residues.

In one embodiment, the isolated peptide targeting the MPER subdomain of the Spike protein comprises or consists of an amino acid sequence selected from **SEQ ID NO: 46** to **SEQ ID NO: 56.**

**SEQ ID NO: 46:** PWYIW
**SEQ ID NO: 47:** WPWYIW
**SEQ ID NO: 48:** KWPWYIW
**SEQ ID NO: 49:** IKWPWYIW
**SEQ ID NO: 50:** YIKWPWYIW
**SEQ ID NO: 51:** QYIKWPWYIW
**SEQ ID NO: 52:** EQYIKWPWYIW
**SEQ ID NO: 53:** YEQYIKWPWYIW
**SEQ ID NO: 54:** KYEQYIKWPWYIW
**SEQ ID NO: 55:** GKYEQYIKWPWYIW
**SEQ ID NO: 56:** LGKYEQYIKWPWYIW

In one embodiment, the isolated peptide targeting the MPER subdomain of the Spike protein does not comprises or consists of the amino acid sequence of the "HR2 region". By **"HR2 region",** it is meant the heptad repeat 2 region of the Spike protein. The HR2 region of the Spike protein of SARS-CoV-2 with **SEQ ID NO: 2** comprises amino acid residues 1163 to 1202 of **SEQ ID NO: 2.**

When reciting "the isolated peptide targeting the MPER subdomain of the Spike protein", also encompassed are isolated peptide variants, such as peptides of a variant Spike protein (as previously defined), peptidomimetics, conjugates, and the like, including any combinations thereof.

In one embodiment, a conjugate of the isolated peptide targeting the MPER subdomain of the Spike protein comprises said isolated peptide, fused or linked to a lipid moiety. In particular, such lipid moiety allows to associate the isolated peptide with the external surface of a cell or a virus, by, *e.g.*, anchoring said isolated peptide to the lipid bilayer. In this respect, the lipid moiety is termed "hydrophobic anchor".

Typical hydrophobic anchors include, but are not limited to, acyl (such as, *e.g*., myristoyl or palmitoyl) and prenyl (such as, *e.g.,* linear poly-isoprene) chains.

In one embodiment, peptidomimetics of the isolated peptide targeting the MPER subdomain of the Spike protein comprise, but are not limited to, retro analogues, inverso analogues and retroinverso analogues of said isolated peptide.

In one embodiment, a conjugate of the isolated peptide targeting the MPER subdomain of the Spike protein comprises or consists of said isolated peptide fused to a payload, such as, a therapeutic agent, a diagnostic agent, or a carrier. In particular, carriers can improve the pharmacokinetics and/or biodistribution of the isolated peptide. Such conjugates are sometimes referred to as "biobetters". For a review, see, *e.g.,* Strohl, 2015. BioDrugs. 29(4):215-239.

A further object of the present invention is the isolated peptide targeting the MPER subdomain of the Spike protein described above, for use in preventing or inhibiting an infection by a virus of the *Orthocoronavirinae* subfamily, as defined hereinabove.

A further object of the present invention is a method of preventing or inhibiting an infection by a virus of the *Orthocoronavirinae* subfamily, as defined hereinabove, in a subject in need thereof, comprising administering to said subject the isolated peptide targeting the MPER subdomain of the Spike protein described above.

In one embodiment, the virus of the *Orthocoronavirinae* subfamily is a virus of the *betacoronavirus* genus, as defined hereinabove. In one embodiment, the virus of the *Orthocoronavirinae* subfamily is a virus of the *betacoronavirus* genus, and severe acute respiratory syndrome-related coronavirus species, as defined hereinabove. In one embodiment, the virus of the *Orthocoronavirinae* subfamily is a virus of the *betacoronavirus* genus, severe acute respiratory syndrome-related coronavirus species and strain SARS-CoV-2, as defined hereinabove. In one embodiment, the virus of the *Orthocoronavirinae* subfamily is SARS-CoV-2, as defined hereinabove.

In one embodiment, preventing or inhibiting the infection comprises blocking or hindering the fusion of the virus membrane with the subject's cell membrane.

## BRIEF DESCRIPTION OF THE DRAWINGS

**Figure 1** shows the predicted B- and T-cell epitope homology within the Spike protein of SARS-CoV-2, SARS-CoV, MERS, common cold viruses, HIV gp41 and CoVEVax vaccine candidates.

**Figure 1A** is an alignment of HIV gp41 MPER-containing sequences. Alignment between HIV strains shows conservation of tryptophan rich region (in bold) and cholesterol interaction domain within predicted neutralizing epitopes.

Figure **1B** shows an alignment of Spike proteins between different coronaviruses [CoV-229E, CoV-NL63, CoV-HKU1, CoV-OC43, MERS, SARS-CoV and SARS-CoV-2], and the constructs used in this study [S-EV and S-Trim]. Tryptophan rich regions and T cell epitopes present within the corresponding Membrane Proximal External Region [MPER] and transmembrane domain [TM] are retained in the Spike protein presented by S-EVs but absent in the S-Trim truncated at residue 1219 (**SEQ ID NO:** 6 numbering; or residue 1208 with **SEQ ID NO: 2** numbering) and fused to a Foldon trimerization motif. S-EV and S-Trim comprise a modified signal peptide [SP]. S-EV comprises a proprietary pilot peptide (CilPP). Proline mutations were introduced in indicated locations (**SEQ ID NO: 2** numbering).

**Figure 2** shows the production and characterization of the CoVEVax vaccine candidates.

**Figure 2A** is a scheme of the production of EV-based vaccine candidates. Three different immunogens were generated to mount immune response against SARS-CoV-2 in mice. $DNA^{S\text{-}EV}$ vector allows *in situ* production of autologous S-EVs upon injection to mice. S-EVs and S-Trim are produced in mammalian cells (HEK293T) *in vitro.*

**Figure 2B** is a set of two gels showing Western blot analysis of cellular extracts from HEK293T cells transfected with $DNA^{S\text{-}Trim}$ and from purified S-Trim (left panel: revealed with anti-S1 antibodies; right panel: revealed with anti-S2 antibodies). The arrow labelled S indicate the size expected for a non-mature Spike protein.

**Figure 2C** is a gel showing Western blot analysis of cellular extracts from HEK293T cells transfected with $DNA^{S\text{-}EV}$ and revealed with anti-CilPP antibodies. The arrow labelled S indicate the size expected for a non-mature Spike protein; the arrow labelled S2 indicate the size expected for a mature S2 subunit.

**Figure 2D** is a set of three graphs showing the purified EVs characterization by ExoView platform. EVs expressing Spike protein are captured with either CD81, CD63 or CD9.

**Figure 2E** is a set of two graphs showing the ELISA analysis of proteins S1 and S2 displayed on S-EVs. Results are shown as $OD_{450\ nm}$ values.

**Figure 2F** is a set of two gels showing Western blot analysis of Spike protein expression on S-EVs. The arrow labelled S indicate the size expected for a non-mature Spike protein; the arrow labelled S1 indicate the size expected for a mature S1 subunit; the arrow labelled S2 indicate the size expected for a mature S2 subunit.

**Figure 2G** is a gel showing Western blot analysis of S-EVs purified from HEK293T cells transfected with $DNA^{S\text{-}EV}$ and revealed with anti-CilPP antibodies in reducing conditions. The arrow labelled S indicate the size expected for a non-mature Spike protein; the arrow labelled S2 indicate the size expected for a mature S2 subunit.

**Figure 2H** is a gel showing Western blot analysis of S-EVs purified from HEK293T cells transfected with $DNA^{S-EV}$ and revealed with anti-CilPP antibodies in non-reducing conditions. The arrow labelled S indicate the size expected for a non-mature and trimeric Spike protein.

**Figure 3** shows the humoral response in mice immunized with CoVEVax.

**Figure 3A** is a schema showing the CoVEVax immunization study design. Four groups of female BALB/cAnNCrl mice (n = 6) received two prime injections at day 0 and 21 and one boost injection at day 42. Mice were bled at day 0, 21, 42 and 63 and spleens were harvested at day 63 to analyze antigen specific cellular response.

**Figure 3B** is a set of two graphs showing the specific IgG response to S1 (left panel) or S2 (right panel) of 2 overlapping peptide pools (each representing respectively, whole S1 or whole S2) at day 63 measured by ELISA for each of the 4 groups. Results are presented as reciprocal endpoint dilution titers. Data points present mean $\pm$ SEM. Dashed line indicates mean titers. ns: non-significant; **: $p < 0.01$ calculated by Mann-Whitney test.

**Figure 3C** is a set of two graphs showing the specific IgG response to S1 (left panel) and S2 (right panel) peptide pools at day 42 measured by ELISA for each of the 4 groups. Results are presented as reciprocal endpoint dilution titers.

**Figure 3D** is a set of two graphs showing the pseudovirus neutralizing antibody titers with sera collected at day 63. Titers correspond to the sera dilutions neutralizing 50 % of the virus. Data points present mean $\pm$ SEM. Dashed line indicates mean titers. ns: non-significant; **: $p < 0.01$ calculated by Mann-Whitney test.

**Figure 4** shows the cellular immune response in mice immunized with CoVEVax. The set of two graphs shows the IFN$\gamma$ ELISpot analysis of antigen specific cellular response. Total T-cells were isolated from pooled splenocytes (from 3 mice) and stimulated with either S1 (left panel) or S2 (right panel) SARS-CoV-2 peptide pools. Results are presented as a number of spot forming cells (SPC) per $1 \times 10^6$ T-cells.

## EXAMPLES

The present invention is further illustrated by the following examples.

## Materials and Methods

### *Expression vectors and molecular cloning*

Expression system to sort membrane proteins on extracellular vesicles (EVs) was developed by CILOA SAS (granted patents EP 2 268 816 and US 9,546,371; De Gassart et al., 2009. Cell Biol Int. 33(1):36-48).

*$DNA^{S-EV}$*

Briefly, the S gene from SARS-CoV-2 Wuhan-Hu-1 isolate (Wu et al., 2020. Nature. 579(7798):265-269; GenBank accession number MN908947, version 3 of March 18, 2020) (**SEQ ID NO: 1**), encoding the Spike protein (**SEQ ID NO: 2**), was codon-optimized for human cells, and further modified as follows:

- substitution of the native Spike signal peptide (nucleotides 1 to 36 of **SEQ ID NO: 1,** corresponding to amino acids 1 to 12 of **SEQ ID NO: 2**) by the signal peptide of the metabotropic glutamate receptor 5 (mGluR5) (nucleic acid sequence with **SEQ ID NO: 3,** corresponding to the amino acid sequence with **SEQ ID NO: 4**);
- two consecutive proline substitutions at positions K986 and V987 (**SEQ ID NO: 2** numbering); and
- a K776A substitution and a Q779G substitution (**SEQ ID NO: 2** numbering).

**SEQ ID NO: 3** – mGluR5

ATGGTGCTGCTGCTGATCCTGTCTGTGCTGCTCCTGAAAGAAGATGTGCGGG
GCTCTGCCCAGAGCACC

**SEQ ID NO: 4** - mGluR5 encoded by SEQ ID NO: 3 MVLLLILSVLLLKEDVRGSAQST

This modified S gene (with **SEQ ID NO: 5,** encoding a modified Spike protein with **SEQ ID NO: 6**) was cloned in PacI-NotI in an in-house pCilA-DCTM eukaryotic expression vector, in order to generate a "DNA$^{S-EV}$" vector. pCilA-DCTM comprises a cytomegalovirus-human T-lymphotropic virus type I (CMV-HTLV-I) promoter upstream of the PacI-NotI expression cassette; and a bGH poly(A) and Ad2 VA1 sequence downstream of the PacI-NotI expression cassette.

This modified S gene was fused in C-terminus to a pilot peptide (herein named "CilPP") which sorts the Spike protein to the surface of EVs (nucleic acid sequence with **SEQ ID NO: 7,** encoding CilPP with amino acid sequence **SEQ ID NO: 8**), through a 3-amino acid linker (nucleic acid sequence with **SEQ ID NO: 9,** encoding the linker with amino acid sequence **SEQ ID NO: 10**).

**SEQ ID NO: 9 -** Linker TCTAGAGGC

**SEQ ID NO: 10** - Linker encoded by SEQ ID NO: 9 SRG

*DNA$^{S-Trim}$*

As was the case for the DNA$^{S-EV}$ vector, the S gene from SARS-CoV-2 Wuhan-Hu-1 isolate **(SEQ ID NO: 1)** was codon-optimized for human cells, and further modified as follows:

- substitution of the native Spike signal peptide (nucleotides 1 to 36 of **SEQ ID NO: 1,** corresponding to amino acid residues 1 to 12 of **SEQ ID NO: 2**) by the signal peptide of the metabotropic glutamate receptor 5 (mGluR5) (nucleic acid sequence with **SEQ ID NO: 3,** corresponding to the amino acid sequence with **SEQ ID NO: 4**);
- two consecutive proline substitutions at positions K986 and V987 (**SEQ ID NO: 2** numbering);
- a K776A substitution and a Q779G substitution (**SEQ ID NO: 2** numbering); and
- truncation at amino acid residue 1208 (**SEQ ID NO: 2** numbering).

This modified *S* gene (with **SEQ ID NO: 11,** encoding a modified Spike protein with **SEQ ID NO: 12**) was cloned in PacI-NotI in the in-house pCilA-DCTM eukaryotic expression vector, in order to generate a "DNA$^{S-Trim}$" vector.

This modified S gene was fused in C-terminus to a sequence comprising (i) a TEV cleavage site, (ii) a bacteriophage T4 foldon trimerization motif, and (iii) a 6×His tag (nucleic acid sequence with **SEQ ID NO: 13,** encoding the linker with amino acid sequence **SEQ ID NO: 14**).

*Pseudovirus neutralization assay*

To establish pseudovirus neutralization assay, the following constructs were obtained. The SARS-CoV-2 S gene from plasmid pUC57-2019-nCoV-S-Hu (GenScript, Piscataway, USA) was truncated in its cytoplasmic tail of 19 amino acids in order to enhance pseudotyping efficiency and then subcloned into in-house expression vector (Johnson et al., 2020. J Virol. JVI.01062-20).

In the pNL4-3.Luc.R-E-luciferase reporter vector (NIH-AIDS Reagent Program, catalog number 3418), luciferase was replaced by Nano Luciferase (named hereafter "pNL4-3.NanoLuc") (Connor et al., 1995. Virology. 206(2):935-944).

*Homo sapiens* angiotensin I converting enzyme 2 (*ACE2*) gene (NM_021804.2, with **SEQ ID NO: 15**) and *Homo sapiens* transmembrane serine protease 2 (*TMPRSS2*) gene (NM_005656.4, with **SEQ ID NO: 16**) were both cloned into pcDNA3.1(+) from GenScript (Piscataway, USA).

**SEQ ID NO: 15** – h*ACE2*

ATGTCAAGCTCTTCCTGGCTCCTTCTCAGCCTTGTTGCTGTAACTGCTGCTCA
GTCCACCATTGAGGAACAGGCCAAGACATTTTTGGACAAGTTTAACCACGA
AGCCGAAGACCTGTTCTATCAAAGTTCACTTGCTTCTTGGAATTATAACACC
AATATTACTGAAGAGAATGTCCAAAACATGAATAATGCTGGGGACAAATGG
TCTGCCTTTTTAAAGGAACAGTCCACACTTGCCCAAATGTATCCACTACAAG
AAATTCAGAATCTCACAGTCAAGCTTCAGCTGCAGGCTCTTCAGCAAAATG
GGTCTTCAGTGCTCTCAGAAGACAAGAGCAAACGGTTGAACACAATTCTAA
ATACAATGAGCACCATCTACAGTACTGGAAAAGTTTGTAACCCAGATAATC
CACAAGAATGCTTATTACTTGAACCAGGTTTGAATGAAATAATGGCAAACA
GTTTAGACTACAATGAGAGGCTCTGGGCTTGGGAAAGCTGGAGATCTGAGG
TCGGCAAGCAGCTGAGGCCATTATATGAAGAGTATGTGGTCTTGAAAAATG
AGATGGCAAGAGCAAATCATTATGAGGACTATGGGGATTATTGGAGAGGAG
ACTATGAAGTAAATGGGGTAGATGGCTATGACTACAGCCGCGGCCAGTTGA
TTGAAGATGTGGAACATACCTTTGAAGAGATTAAACCATTATATGAACATCT
TCATGCCTATGTGAGGGCAAAGTTGATGAATGCCTATCCTTCCTATATCAGT
CCAATTGGATGCCTCCCTGCTCATTTGCTTGGTGATATGTGGGGTAGATTTT
GGACAAATCTGTACTCTTTGACAGTTCCCTTTGGACAGAAACCAAACATAG
ATGTTACTGATGCAATGGTGGACCAGGCCTGGGATGCACAGAGAATATTCA
AGGAGGCCGAGAAGTTCTTTGTATCTGTTGGTCTTCCTAATATGACTCAAGG
ATTCTGGGAAAATTCCATGCTAACGGACCCAGGAAATGTTCAGAAAGCAGT

CTGCCATCCCACAGCTTGGGACCTGGGGAAGGGCGACTTCAGGATCCTTATGTGCACAAAGGTGACAATGGACGACTTCCTGACAGCTCATCATGAGATGGGGCATATCCAGTATGATATGGCATATGCTGCACAACCTTTTCTGCTAAGAAATGGAGCTAATGAAGGATTCCATGAAGCTGTTGGGGAAATCATGTCACTTTCTGCAGCCACACCTAAGCATTTAAAATCCATTGGTCTTCTGTCACCCGATTTTCAAGAAGACAATGAAACAGAAATAAACTTCCTGCTCAAACAAGCACTCACGATTGTTGGGACTCTGCCATTTACTTACATGTTAGAGAAGTGGAGGTGGATGGTCTTTAAAGGGGAAATTCCCAAAGACCAGTGGATGAAAAAGTGGTGGGAGATGAAGCGAGAGATAGTTGGGGTGGTGGAACCTGTGCCCCATGATGAAACATACTGTGACCCCGCATCTCTGTTCCATGTTTCTAATGATTACTCATTCATTCGATATTACACAAGGACCCTTTACCAATTCCAGTTTCAAGAAGCACTTTGTCAAGCAGCTAAACATGAAGGCCCTCTGCACAAATGTGACATCTCAAACTCTACAGAAGCTGGACAGAAACTGTTCAATATGCTGAGGCTTGGAAAATCAGAACCCTGGACCCTAGCATTGGAAAATGTTGTAGGAGCAAAGAACATGAATGTAAGGCCACTGCTCAACTACTTTGAGCCCTTATTTACCTGGCTGAAAGACCAGAACAAGAATTCTTTTGTGGGATGGAGTACCGACTGGAGTCCATATGCAGACCAAAGCATCAAAGTGAGGATAAGCCTAAAATCAGCTCTTGGAGATAAAGCATATGAATGGAACGACAATGAAATGTACCTGTTCCGATCATCTGTTGCATATGCTATGAGGCAGTACTTTTTAAAAGTAAAAAATCAGATGATTCTTTTTGGGGAGGAGGATGTGCGAGTGGCTAATTTGAAACCAAGAATCTCCTTTAATTTCTTTGTCACTGCACCTAAAAATGTGTCTGATATCATTCCTAGAACTGAAGTTGAAAAGGCCATCAGGATGTCCCGGAGCCGTATCAATGATGCTTTCCGTCTGAATGACAACAGCCTAGAGTTTCTGGGGATACAGCCAACACTTGGACCTCCTAACCAGCCCCCTGTTTCCATATGGCTGATTGTTTTTGGAGTTGTGATGGGAGTGATAGTGGTTGGCATTGTCATCCTGATCTTCACTGGGATCAGAGATCGGAAGAAGAAAAATAAAGCAAGAAGTGGAGAAAATCCTTATGCCTCCATCGATATTAGCAAAGGAGAAAATAATCCAGGATTCCAAAACACTGATGATGTTCAGACCTCCTTTAG

**SEQ ID NO: 16** – h*TMPRSS2*

ATGGCTTTGAACTCAGGGTCACCACCAGCTATTGGACCTTACTATGAAAACC
ATGGATACCAACCGGAAAACCCCTATCCCGCACAGCCCACTGTGGTCCCCA
CTGTCTACGAGGTGCATCCGGCTCAGTACTACCCGTCCCCCGTGCCCCAGTA
CGCCCCGAGGGTCCTGACGCAGGCTTCCAACCCCGTCGTCTGCACGCAGCC
CAAATCCCCATCCGGGACAGTGTGCACCTCAAAGACTAAGAAAGCACTGTG
CATCACCTTGACCCTGGGGACCTTCCTCGTGGGAGCTGCGCTGGCCGCTGGC
CTACTCTGGAAGTTCATGGGCAGCAAGTGCTCCAACTCTGGGATAGAGTGC
GACTCCTCAGGTACCTGCATCAACCCCTCTAACTGGTGTGATGGCGTGTCAC
ACTGCCCCGGCGGGGAGGACGAGAATCGGTGTGTTCGCCTCTACGGACCAA
ACTTCATCCTTCAGGTGTACTCATCTCAGAGGAAGTCCTGGCACCCTGTGTG
CCAAGACGACTGGAACGAGAACTACGGGCGGGCGGCCTGCAGGGACATGG
GCTATAAGAATAATTTTTACTCTAGCCAAGGAATAGTGGATGACAGCGGAT
CCACCAGCTTTATGAAACTGAACACAAGTGCCGGCAATGTCGATATCTATA
AAAAACTGTACCACAGTGATGCCTGTTCTTCAAAAGCAGTGGTTTCTTTACG
CTGTATAGCCTGCGGGGTCAACTTGAACTCAAGCCGCCAGAGCAGGATTGT
GGGCGGCGAGAGCGCGCTCCCGGGGGCCTGGCCCTGGCAGGTCAGCCTGCA
CGTCCAGAACGTCCACGTGTGCGGAGGCTCCATCATCACCCCCGAGTGGAT
CGTGACAGCCGCCCACTGCGTGGAAAAACCTCTTAACAATCCATGGCATTG
GACGGCATTTGCGGGGATTTTGAGACAATCTTTCATGTTCTATGGAGCCGGA
TACCAAGTAGAAAAAGTGATTTCTCATCCAAATTATGACTCCAAGACCAAG
AACAATGACATTGCGCTGATGAAGCTGCAGAAGCCTCTGACTTTCAACGAC
CTAGTGAAACCAGTGTGTCTGCCCAACCCAGGCATGATGCTGCAGCCAGAA
CAGCTCTGCTGGATTTCCGGGTGGGGGGCCACCGAGGAGAAAGGGAAGACC
TCAGAAGTGCTGAACGCTGCCAAGGTGCTTCTCATTGAGACACAGAGATGC
AACAGCAGATATGTCTATGACAACCTGATCACACCAGCCATGATCTGTGCC
GGCTTCCTGCAGGGGAACGTCGATTCTTGCCAGGGTGACAGTGGAGGGCCT
CTGGTCACTTCGAAGAACAATATCTGGTGGCTGATAGGGGATACAAGCTGG
GGTTCTGGCTGTGCCAAAGCTTACAGACCAGGAGTGTACGGGAATGTGATG
GTATTCACGGACTGGATTTATCGACAAATGAGGGCAGACGGCTAA

***Production and purification of S trimer (S-Trim)***

The production and purification of coronavirus S trimer was previously described by Pallesen et al. (2017. Proc Natl Acad Sci USA. 114(35):E7348-E7357).

In brief, HEK293T cells were transfected with DNA$^{S\text{-}Trim}$ plasmid using polyethylenimine (PEI) and harvested 48 h later. The cell pellet was then resuspended in 1X PBS supplemented with 0.5 mM PMSF and protease inhibitors (Protease Inhibitor Cocktail set III, EDTA-free, Calbiochem).

Lysis buffer (1X PBS, 2 % octyl β-*D*-1 thioglucopyranoside, 12 mM $MgCl_2$, 0.5 mM PMSF, protease inhibitors and DNase I) was then added and incubated for 30 minutes at 4°C.

The lysate was clarified by centrifugation at 800 rpm for 10 minutes at 4°C, then at 15 000 rpm for 15 minutes at 4°C. The supernatant was collected, equilibrated with 10 mM imidazole, and loaded onto IMAC resin (1 mL HisTALON™ Superflow Cartridge [Takara] coupled to a fast protein liquid chromatography system Äkta FPLC UPC-900/P-920 [GE Healthcare]).

S-Trim was eluted with 150 mM imidazole. Fractions containing pure S-Trim were identified by Western Blot and pooled. Protein concentration was estimated with bicinchoninic acid (BCA) assay (Pierce BCA Protein Assay Kit, ThermoFisher Scientific), by measuring absorbance at 562 nm.

***Production of EVs harboring Spike protein (S-EVs) in mammalian cells***

EVs were produced in HEK293T cells obtained from American Type Culture Collection (ATCC). Cells were cultured in DMEM supplemented with 5 % heat-inactivated fetal bovine serum (iFBS), 2 mM GlutaMAX and 5 μg/mL gentamicin at 37°C in a 5 % $CO_2$ humidified incubator. HEK293T cells were routinely tested and found negative by MycoAlert™ mycoplasma detection kit (Lonza Nottingham, Ltd.).

DNA$^{S\text{-}EV}$ were transfected into HEK293T cells using PEI. In order to generate large-scale exosome production, HEK293T cells were plated into cell chambers of 10 trays in 1 L of complete medium. Twenty-four hours post-transfection, cultures were fed with medium supplemented with EV-free iFBS and incubated for a further 48 hours.

***S-EVs purification***

Cell culture medium was harvested from transiently transfected HEK293T cells and S-EV isolation was performed as previously described (Taylor & Shah, 2015. Methods. 87:3-10; Desplantes et al., 2017. SciRep. 7(1):1032). Briefly, cell culture supernatant was clarified by two consecutive centrifugations: 10 minutes at 1300 rpm and 15 minutes at 4000 rpm, both at 4°C, followed by filtration through 0.22 μm membrane filters.

The supernatant was then concentrated by ultra-filtration and diafiltration and load onto size exclusion chromatography (SEC) columns (Sephacryl S1000, GE Healthcare). Fractions containing EVs biomarkers (CD81 and CD63) were identified by ELISA. EV fractions containing Spike protein identified by Western-blot were pooled, concentrated when necessary, and used for analysis and injections.

***ExoView EVs characterization***

Purified S-EVs were sized and characterized using the Single Particle Interferometric Reflectance Imaging Sensor (SP-IRIS) technology using the ExoView R100 platform (NanoView Biosciences, Boston, USA).

Tetraspanin kit chips (EV-TETRA-C), with capture antibodies against CD81, CD63, CD9 and IgG (MIgG), were used to detect S-EVs.

S-EVs at $10^{10}$ EVs/mL were coated on chips, then probed with detection antibodies provided with the kit (CD81/CF55, CD63/CF647, CD9/CF488A) according to the manufacturer's protocol. The chips were imaged with ExoView R100, label-free counts were used for S-EV size determination (with a size range set from 50 to 200 nm).

***SDS-PAGE, Western blotting and antibodies***

Protein concentration of S-EVs was measured using the BCA assay (Pierce BCA Protein Assay Kit, ThermoFisher Scientific).

S-EVs or S-Trim preparations were separated by SDS-PAGE on a 4-15 % acrylamide gel (4-15 % Mini-PROTEAN® TGX Stain-Free™ Gel, Bio-Rad) and subsequently transferred onto PVDF membrane. For Western blotting in non-reducing conditions, a loading buffer without DTT was used.

Immunodetection of Spike protein was carried out with primary antibodies against either the S1 head subunit (anti-SARS-CoV-2 Spike S1, rabbit monoclonal antibody [HL6], GTX635654, Genetex) or the S2 stem subunit (anti-SARS-CoV-2 Spike S2, mouse monoclonal antibody [1A9], GTX632604, Genetex) or anti-CilPP (in-house antibody raised in rabbit).

Membranes were then incubated with the corresponding secondary HRP-conjugated antibodies (donkey anti-mouse or anti-rabbit HRP, #715-035-150 or #711-035-152, Jackson ImmunoResearch). The signals were detected using an enhanced chemiluminescence detection kit (Super Signal West Pico Plus, 34580, ThermoFischer Scientific) and membranes imaged with ChemiDoc Imaging System (BioRad).

The same anti-S1 and anti-S2 antibodies, as well secondary antibodies, were also used to detect the Spike protein on S-EVs by ELISA (see below).

***Ethics statement***

All animal studies were performed in accordance with national regulations and approved prior to experimentation by the Ethics Committee for Animal Testing Languedoc-Roussillon (national agreement CEEA-036) and French Ministry of Research with the reference APAFIS #24869 (April 10, 2020). All the procedures and protocols were performed at DMEM unit of INRAE-UM in Montpellier, France (Veterinary Services National Agreement n° E34-172-10, March 04, 2019).

***Mice and study design***

Female BALB/cAnNCrl mice, 6 weeks old, were purchased from Charles River (Italy) and placed into four groups of n = 6. Mice were housed in individually ventilated cages with controlled environmental parameters: 24°C, 12 hours/12 hours light/dark cycle, nesting cotton squares for enrichment, free access to standard A04 irradiated food (SAFE, R04-25) and tap water. Behavior and health status were observed daily and weight checked weekly. One week after their arrival, animals received 2 prime immunizations at day 0 and 21 and a boost immunization at day 42.

- group **$DNA^{S\text{-}EV}$/S-EV:**

    ◦ prime immunization: $DNA^{S\text{-}EV}$ vectors were injected using a Gene Gun (BioRad, Helios) into the abdomen of mice previously shaved. Each mouse received 3 cartridges coated with gold beads containing $DNA^{S\text{-}EV}$ corresponding to a total of 3 μg of DNA, at day 0 and 21;
    ◦ boost immunization: 10 μg of S-EVs were injected subcutaneously in each mouse, in 100 μL volume of PBS, at day 42.

- group **$DNAS^{S\text{-}EV}$/S- Trim:**

    ◦ prime immunization: $DNA^{S\text{-}EV}$ vectors were injected using a Gene Gun (BioRad, Helios) into the abdomen of mice previously shaved. Each mouse received 3 cartridges coated with gold beads containing $DNA^{S\text{-}EV}$ corresponding to a total of 3 μg of DNA, at day 0 and 21;
    ◦ boost immunization: S-Trim was injected subcutaneously in each mouse, in 100 μL volume of PBS, at day 42.

- group **S-EV/S-EV:**

    ◦ prime immunization: 10 μg of S-EVs were injected subcutaneously in each mouse, in 100 μL volume of PBS, at day 0 and 21;
    ◦ boost immunization: 10 μg of S-EVs were injected subcutaneously in each mouse, in 100 μL volume of PBS, at day 42.

- group **PBS:** negative control, received PBS injections at days 0, 21 and 42.

Sera were collected via submandibular bleeding before each immunization: 70 to 100 mL of blood was collected with GOLDENROD animal Lancets (3 mm, Genobios). All animals were euthanized at day 63 and spleens were collected for cellular response analysis.

***S1/S2 and antigen specific IgG ELISA***

SARS-CoV-2 specific antibody titers of sera were determined by ELISA. Briefly, MaxiSorp ELISA plates (Nunc) were 2 μg/mL of SARS-CoV-2 Spike overlapping peptide pools corresponding to S1 or S2 subunits (PepMix™ SARS-CoV-2 Spike glycoprotein, JPT, #PM-WCPV-S) in 100 μL, 50 mM sodium carbonate/bicarbonate pH 9.6 buffer per well, overnight at 4°C.

Coated plates were washed 3 times with 200 μL of 1X PBS and saturated for 1 hour at 37°C with 200 μL 3 % BSA in 1X PBS per well.

Plates were washed three times with 1X PBS, then incubated in 3 % BSA and 5 % FBS with 3-fold mouse sera dilutions (starting from 1:100) for 2 hours at 37°C. This was followed by 3 washes with 200 μL of 1X PBS per well and

incubation with 100 μL per well of secondary donkey anti-mouse HRP conjugated antibody diluted 1:10000 in 3 % BSA in 1X PBS.

Following incubation with the secondary antibody, plates were washed 5 times with 200 μL of 1X PBS per well and developed with 100 μL of TMB per well (Bio-Rad #R8/R9) for 30 minutes. The reaction was stopped by adding 50 μL of stop solution (2 N sulfuric acid) per well.

The 450 nm-absorbance was read using ClarioStar Plus plate reader (BMG Labtech). The reciprocal endpoint titers were defined as the dilution with the 450 nm OD 3 times higher than the background. Undetectable antibody titers (negative values) were assigned values of 0.

ELISA analysis of Spike protein exposure on EVs was performed as above, with few modifications. MaxiSorp ELISA plates were coated with S-EVs at 10 μg/mL in 100 μL 50 mM sodium carbonate/bicarbonate pH 9.6 buffer per well, overnight at 4°C. Saturation was performed with 3 % BSA in 1X PBS and followed by incubation with dilutions of either anti-S1 or S2 antibodies. Secondary HRP-conjugated donkey anti-rabbit and anti-mouse antibodies were used, respectively.

***IFN-γ ELISpot assay***

Single cell suspensions were prepared from spleens of mice euthanized at day 63. Splenocytes from all immunized mice were analyzed in to pools of three animals. Total T-cells were isolated (EasySep™ Mouse T Cell Isolation Kit, StemCells #19851), and $2\times10^5$ T-cells were stimulated for 18 hours in cell culture medium (RPMI 1640 with L-glutamine, 25 mM Hepes, 10 % FBS and 5 μg/mL gentamycin) at 37°C and 5 % $CO_2$ with overlapping peptide pools representative of either S1 or S2 subunit of the Spike protein of SARS-CoV-2 (PepMix™ SARS-CoV-2 Spike glycoprotein, JPT, #PM-WCPV-S) at 1 μg/mL each, or DMSO in 96-well ELISpot IFN-γ plates (Mabtech #3321-4APW-2) in triplicates. As positive control stimuli, 0.5-1 $\times$ $10^5$ cells were stimulated with PHA-L (eBioscience, #15556286) at 1.25-5 μg/mL.

Following the 18-hour incubation, plates were treated according to the manufacturer's protocol. Acquisition and analysis were performed with a CTL Immunospot S6 analyzer.

Measurement values from DMSO-stimulated cells were subtracted from all the measurements. Negative values were corrected to 0. Results are represented as a number of spot forming cells (SPC) per 1 $\times$ $10^6$ T-cells.

***Pseudovirus neutralization assay***

To produce SARS-CoV-2 pseudoviruses, SARS-CoV-2 truncated Spike expression and lentiviral pNL4-3.NanoLuc vectors were co-transfected into HEK293T cells using PEI.

Pseudovirus-containing supernatant was collected 48 hours after, filtered through 0.45-μm filters, aliquoted and stored at -80°C.

To perform the neutralization assay, 2-fold mouse sera dilutions (starting from 1:10) collected at day 63 were mixed with equal volumes of pre-tittered (1000 times the background of fluorescence) Spike-pseudotyped HIV-NanoLuc viruses (1:2), incubated for 1 hour at 37°C, 5 % $CO_2$ and then added to HEK293T cells transiently expressing ACE2 receptor and TMPRSS2 protease in 96-well plates (in triplicates) for 1 hour at 37°C, 5 % $CO_2$. Cell culture medium was then changed. After a 48-hour incubation, cells were lysed and luciferase activity (RLU, relative light units) was measured using the Bright-Glo™ Luciferase Assay System (Promega). Background luminescence produced by cells-only controls (no pseudotyped virus) and positive luminescence produced by pseudotyped virus-infected cells were included and served to determine percentage of neutralization as 100 % and 0 %, respectively. Neutralization titers were defined as the sera dilutions that neutralize 50 % of the virus.

***Protein sequence alignment***

Multiple sequence alignment was performed with Clustal Omega tool (EMBL-EBI).

## Results

***Predicted B- and T-cell epitope homology within the Spike protein of SARS-CoV-2, SARS-CoV, common cold viruses, HIV gp41 and CoVEVax vaccine candidates***

For optimal vaccine design, several envelope protein sequences were analyzed, considering B and T epitopes not only from coronaviruses but also from HIV strains (**Fig. 1**).

The HIV's MPER sub-region required for the membrane fusion of HIV's protein TM contains a tryptophan-rich cluster, a cholesterol-binding domain and a neutralizing epitope conserved through all HIV clades (**Fig. 1A**) (Liu et al., 2018. Protein Cell. 9(7):596-615).

Interestingly, the S2 MPER sub-region of all coronaviruses (four human "common cold" coronaviruses, MERS, SARS-CoV and SARS-CoV-2) possesses an analogous highly-conserved tryptophan cluster and a putative cholesterol-binding region (**Fig. 1B**, top).

Similarly to HIV, this conserved MPER sub-region would contain a neutralizing B epitope and includes one of the T-cell epitopes cross-reactive in unexposed individuals (Mateus et al., 2020. Science. eabd3871). In addition, the most important reactive T-cell epitope of SARS-CoV, entirely conserved in SARS-CoV-2, is located in the transmembrane domain (**Fig. 1B**, top) (Wang et al., 2004. J Virol. 78(11):5612-5618). Altogether, these facts demonstrate the importance of including the MPER subregion and transmembrane domain in a vaccine.

The first molecular construct (S-Trim) was often used in other vaccines; it comprises an immunogen containing the full S1 subunit, the S2 subunit truncated at Q1219 (**SEQ ID NO: 6** numbering; or residue 1208 with **SEQ ID NO: 2** numbering) and an additional phage Foldon which allows artificial trimerization. This S1/S2 immunogen is soluble because it is devoid of the MPER subregion and transmembrane domains (**Fig. 1B**, bottom).

The second construct (S-EV) is a Spike protein, comprising subunits S1 and S2, anchored in a natural membrane. It contains the MPER subregion and transmembrane domain, identified as potentially important in triggering neutralizing humoral and cellular immune responses and a function in membrane anchoring (**Fig. 1B,** bottom).

***Production and characterization of the CoVEVax vaccine candidates***

It is well-known that T-cell immune responses can be elicited by DNA vaccines, but that a protein boost is required for high humoral responses.

Thus, we chose to develop a two-component vaccine, the first one being the $DNA^{S-EV}$ leading to *in situ* production and loading of Spike protein S1 and S2 subunits on autologous EVs (S-EVs), and the second one being exogenously produced and purified S-EVs (**Fig. 2A**).

As a control, we produced soluble S-Trim as a third immunogen (**Fig. 2A**). The expression of S-Trim was analyzed by Western blotting. A band of the size expected for a non-mature protein was revealed when probed with either anti-S1 or anti-S2 antibody (**Fig. 2B**), indicating that the artificial S-Trim was correctly expressed but remained immature and was not processed into S1 and S2.

$DNA^{S-EV}$, when transfected in HEK293T cells, allowed the expression of two main proteins detected by an anti-CilPP antibody, the first with a size expected for an immature S and the second with a size expected for a mature S2 subunit (**Fig. 2C**). This partial maturation of envelope proteins in cells was previously described for coronaviruses and HIV (Park et al., 2016. Proc Natl Acad Sci USA. 113(43):12262-12267; Moulard et al., 1999. Virus Res. 60(1):55-65).

Secreted EVs were purified and analyzed for their size and protein content. Using the ExoView platform, we observed that S-EVs are specifically captured by anti-CD81, anti-CD63 and anti-CD9 antibodies and exhibit a size of ~55 nm, all being signatures of EVs **(Fig. 2D).** ELISA analysis revealed that these purified S-EVs harbor both S1 and S2 subunits at their surface **(Fig. 2E).** Interestingly, when analyzed by Western blot, the secreted EVs contained S immature protein, but predominantly, mature S1 and S2 subunits **(Fig. 2F and 2G);** all being associated as a natural S1/S2 trimer, naturally anchored in the EV membrane **(Fig. 2H).**

***Humoral response in mice immunized with CoVEVax***

Mice were immunized at day 0, 21 and 42 with 3 different combinations of CoVEVax immunogens, or with PBS as a negative control **(Fig. 3A).** Notably, in this study, no adjuvants were administered ($DNA^{S-EV}$, purified S-EVs and soluble S-Trim were suspended in PBS). Sera were analyzed at day 42 (prime bleed) and day 63 (terminal bleed) together with spleen collection **(Fig. 3A).** The humoral immune response induced by CoVEVax was assessed by ELISA. To determine SARS-CoV-2 specific titers, we coated ELISA plates with peptide pools representative of either the S1 or S2 subunit.

Compared to the PBS group, all immunizations revealed evidence of antibodies directed against both S1 and S2 subunits after the protein boost. The mean titers against the S1 subunit obtained with sera of group 1 ($DNA^{S-EV}$/S-EV), group 2 ($DNA^{S-EV}$/S-Trim) and group 3 (S-EV/S-EV) were 1716, 663 and 4316, respectively. The mean titers against the S2 subunit obtained with sera of group 1, group 2 and group 3 were 1494, 591, 3116, respectively **(Fig. 3B).**

The lowest titers were obtained with S-Trim boost injection and no S-EV injection; average titers were obtained with one S-EV boost injection; the highest titers resulted from three S-EV injections. Hence, titers seem to correlate with the number of S-EV injections, which are important to mount an antibody response. Accordingly, sera from day 42 showed elevated ELISA titers within group 3 only **(Fig. 3C).**

Surprisingly, the humoral response against both S1 and S2 corresponding peptide pools was of a similar level, even though the S1 "head" subunit, with its receptor binding domain (RBD), is widely recognized as the main immunogen; here, when presented on EVs, the S2 "stem" subunit acts as a potent antigen as well.

In the depicted ELISA experiments, only the humoral response against linear epitopes was measured. We hypothesized that the Spike protein, when presented on EVs, would also trigger an immune response against conformational epitopes that are exhibited by the virus, and which are, generally, the true targets of neutralizing antibodies. To this end, the neutralizing activity of the sera was quantified with a commonly used pseudotyped lentivirus neutralization assay **(Fig. 3D).**

In brief, serial dilutions of sera were mixed with Spike-pseudotyped viruses and then used to infect HEK293T cells expressing human ACE2 receptor and TMPRSS2 protease. The results showed that, although group 3 displayed binding the highest ELISA titers, only one animal developed a low level (mean titer = 160) of neutralizing antibodies, while the rest of the group displayed none, similarly to the negative control group. In contrast, mice in group 2 have produced a good level of neutralizing antibodies, with a mean titer of 827 (range: 320-1600), *i.e.,* similar to the mean neutralizing titers reported for other vaccines (Folegatti et al., 2020. Lancet. 396(10249):467-478; Jackson et al., 2020. N Engl J Med. NEJMoa2022483; Yang et al., 2020. Nature. 10.1038/s41586-020-2599-8).

Most surprisingly, the sera of mice from group 1 exhibited very high level of neutralizing antibodies (mean titer = 4173; range: 900-15300) **(Fig. 3D).**

***Cellular immune response in mice immunized with CoVEVax***

Finally, we looked at the T-cell mediated immunity, a second type of protective immune response, using ELISpot assay measuring antigen specific IFN-$\gamma$ production.

As before, we aimed to identify and discriminate between the immune responses against S 1 and S2 subunits. Mice immunized with S-EVs only (group 3) did not develop cellular immune response as measured by IFN-$\gamma$ ELISpot.

By contrast, the two groups (1 and 2) immunized by two $DNA^{S-EV}$ primes and one protein boost (S-EV or S-Trim) developed strong cellular immune response, reaching ~350 SFC and ~150 SFC per $10^6$ T-cells for S 1 and S2 immunogens, respectively **(Fig. 4).**

## Discussion

In conclusion, we have developed a unique EV-based vaccine candidate harboring a fully native and mature Spike protein from SARS-CoV-2, that triggers both a strong neutralizing response and a strong cellular immune response.

These responses were obtained in the absence of any other viral component and without adjuvants. They are directed against both S1 "head" and S2 "stem" subunits of the Spike protein, which is important for a broad coverage of the vaccine in regard to the high conservation of the S2 subunit in all coronavirus clades.

The two components of the CoVEVax are crucial to obtain both types of protective immune responses, the prime $DNA^{S-EV}$-based immunization triggering T-cell response, and the boost protein-based immunization (either with S-EV or S-Trim) rising the humoral response.

The observed strong neutralizing activity prompt us to expect that CoVEVax is unlikely to promote the induction of antibodies which can trigger antibody dependent enhancement (ADE) adverse events.

Additionally, this EV-based vaccine, which contains highly conserved sequences, will in all likelihood exhibit protection against antigenic drift that began to appear for SARS-CoV-2 (Korber et al., 2020. Cell. 182(4):812-827; Becerra-Flores & Cardozo, 2020. Int J Clin Pract. e13525).

## Claims

**1.** A nucleic acid comprising:

(i) a sequence of a S gene coding for a Spike protein from a virus of the *Orthocoronavirinae* subfamily, or a variant thereof; and
(ii) a sequence coding for a pilot peptide which interacts with ESCRT proteins.

**2.** The nucleic acid according to claim **1**, wherein the virus of the *Orthocoronavirinae* subfamily is Severe Acute Respiratory Syndrome Coronavirus 2 (SARS-CoV-2).

**3.** The nucleic acid according to claim **1** or **2**, wherein the S gene has a nucleic acid sequence coding for a Spike protein with **SEQ ID NO: 2,** or a variant thereof.

**4.** The nucleic acid according to any one of claims **1** to **3,** wherein the S gene has a nucleic acid sequence coding for a variant of the Spike protein with **SEQ ID NO: 6.**

**5.** The nucleic acid according to any one of claims **1** to **4,** wherein the pilot peptide comprises at least one YxxL motif with **SEQ ID NO: 17** or DYxxL motif with **SEQ ID NO: 20,** and at least one PxxP motif with **SEQ ID NO: 24,** in which "x" represents any amino acid residue.

**6.** The nucleic acid according to any one of claims **1** to **5,** wherein the pilot peptide comprises an amino acid sequence with **SEQ ID NO: 8** or a variant thereof, with the proviso that a variant of **SEQ ID NO: 8** retains three YxxL motifs with **SEQ ID NO: 17** and four PxxP motifs with **SEQ ID NO: 24,** in which "x" represents any amino acid residue.

**7.** The nucleic acid according to any one of claims 1 to 6, being inserted into a nucleic acid expression vector, and being operably linked to regulatory elements.

**8.** An extracellular vesicle harboring at its external surface a Spike protein from a virus of the *Orthocoronavirinae* subfamily "*S-EV*", or a variant thereof.

**9.** The extracellular vesicle according to claim **8,** wherein the virus of the *Orthocoronavirinae* subfamily is Severe Acute Respiratory Syndrome Coronavirus 2 (SARS-CoV-2)

**10.** The extracellular vesicle according to claim **8** or **9,** wherein the extracellular vesicle is an exosome, preferably having a diameter ranging from about 30 nm to about 120 nm.

**11.** The extracellular vesicle according to any one of claims **8** to **10,** being obtainable by a method comprising steps of:

1) transfecting cells with the nucleic acid according to any one of claims **1** to 7;
2) culturing the transfected cells for a time sufficient to allow extracellular vesicle production; and
3) purifying said extracellular vesicle.

**12.** A population of extracellular vesicles according to any one of claims **8** to **11.**

**13.** The nucleic acid according to any one of claims **1** to **7,** for use in a method of immunizing a subject against a virus of the *Orthocoronavirinae* subfamily, said method comprising:

1) at least one priming step, wherein the nucleic acid according to any one of claims **1** to **7** is to be administered to said subject; and

2) at least one boosting step, wherein:

a. the extracellular vesicle according to any one of claims **8** to **11** or the population of extracellular vesicles according to according to claim **12** is to be administered to said subject, or
b. a trimeric Spike protein from a virus of the *Orthocoronavirinae* subfamily is to be administered to said subject,

thereby immunizing the subject against a virus of the *Orthocoronavirinae* subfamily.

**14.** The nucleic acid and the extracellular vesicle for use according to claim **13,** wherein the virus of the *Orthocoronavirinae* subfamily is Severe Acute Respiratory Syndrome Coronavirus 2 (SARS-CoV-2).

**15.** The nucleic acid and the extracellular vesicle for use according to claim **13** or **14,** wherein the method of immunizing comprises two iterations of the priming step and one iteration of the boosting step, preferably the period of time between each iteration ranges from about 2 weeks to about 1 month, more preferably the period of time between each iteration is about 3 weeks.

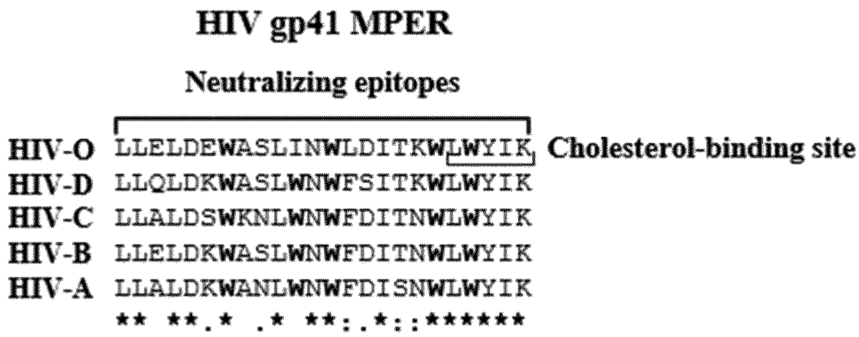
HIV gp41 MPER
Neutralizing epitopes
HIV-O LLELDEWASLINWLDITKWLWYIK Cholesterol-binding site
HIV-D LLQLDKWASLWNWFSITKWLWYIK
HIV-C LLALDSWKNLWNWFDITNWLWYIK
HIV-B LLELDKWASLWNWFDITNWLWYIK
HIV-A LLALDKWANLWNWFDISNWLWYIK
** **.* .* **:.*::******

FIG. 1A

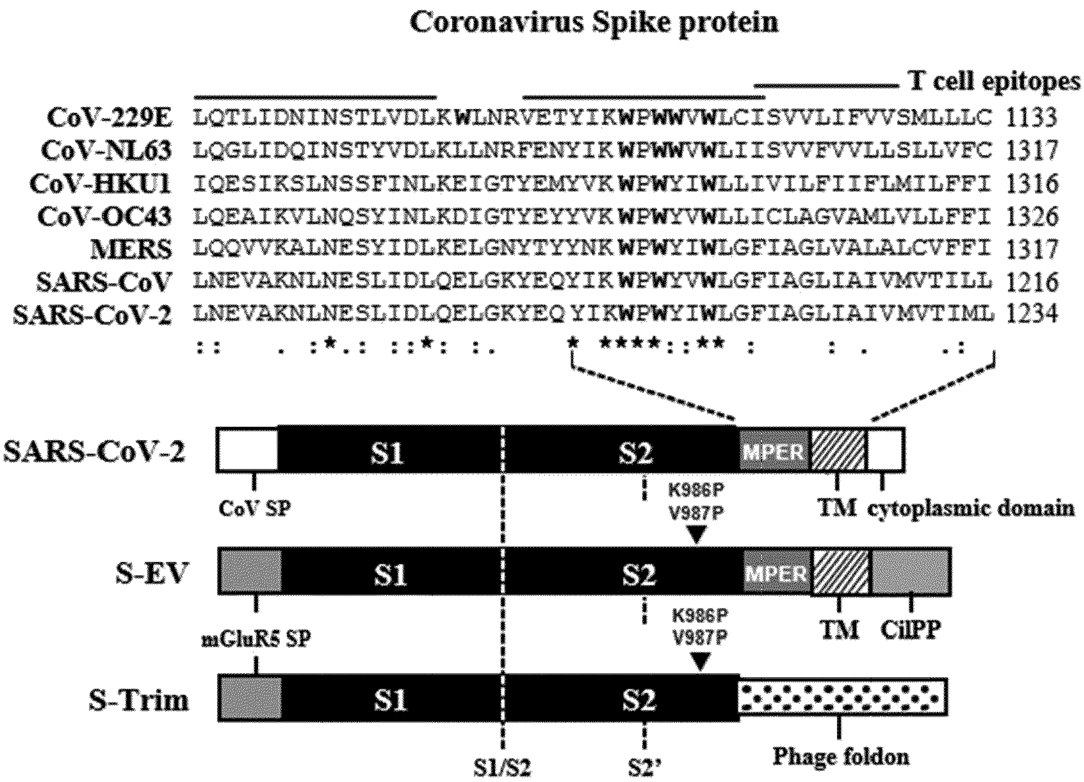
Coronavirus Spike protein
T cell epitopes
CoV-229E LQTLIDNINSTLVDLKWLNRVETYIKWPWWVWLCISVVLIFVVSMLLLC 1133
CoV-NL63 LQGLIDQINSTYVDLKLLNRFENYIKWPWWVWLIISVVFVVLLSLLVFC 1317
CoV-HKU1 IQESIKSLNSSFINLKEIGTYEMYVKWPWYIWLLIVILFIIFLMILFFI 1316
CoV-OC43 LQEAIKVLNQSYINLKDIGTYEYYVKWPWYVWLLICLAGVAMLVLLFFI 1326
MERS LQQVVKALNESYIDLKELGNYTYYNKWPWYIWLGFIAGLVALALCVFFI 1317
SARS-CoV LNEVAKNLNESLIDLQELGKYEQYIKWPWYVWLGFIAGLIAIVMVTILL 1216
SARS-CoV-2 LNEVAKNLNESLIDLQELGKYEQYIKWPWYIWLGFIAGLIAIVMVTIML 1234
SARS-CoV-2
S1
S2
MPER
CoV SP
K986P
V987P
TM cytoplasmic domain
S-EV
S1
S2
MPER
mGluR5 SP
K986P
V987P
TM CilPP
S-Trim
S1
S2
S1/S2
S2'
Phage foldon

FIG. 1B

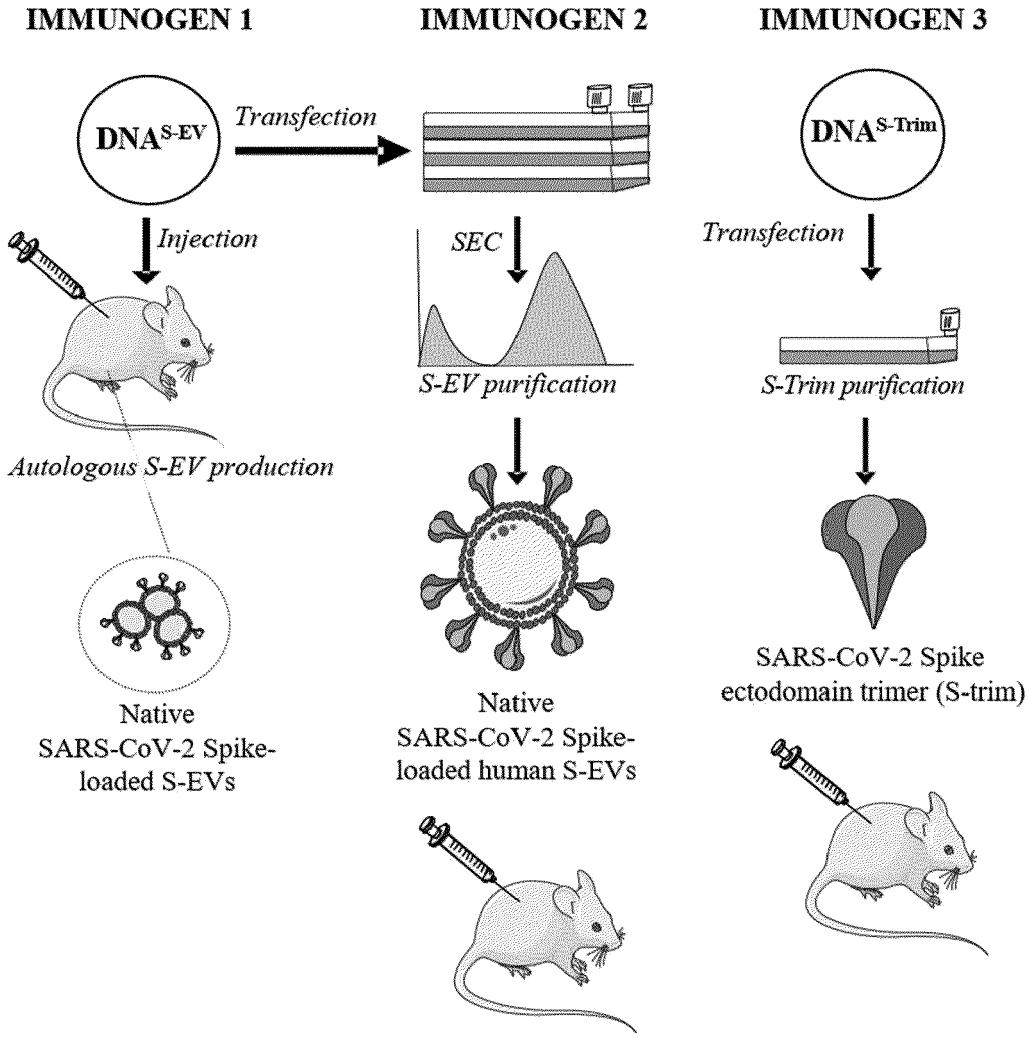
IMMUNOGEN 1
IMMUNOGEN 2
IMMUNOGEN 3
DNA S-EV
Transfection
DNA S-Trim
Injection
SEC
Transfection
S-EV purification
S-Trim purification
Autologous S-EV production
Native SARS-CoV-2 Spike-loaded S-EVs
Native SARS-CoV-2 Spike-loaded human S-EVs
SARS-CoV-2 Spike ectodomain trimer (S-trim)

FIG. 2A

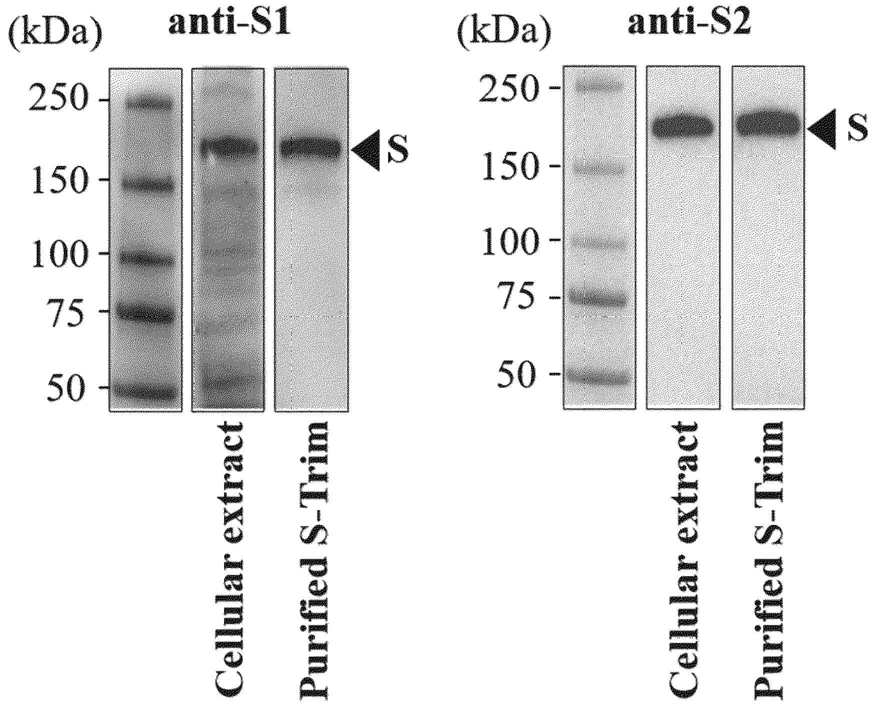
(kDa)
anti-S1
250
150
100
75
50
S
Cellular extract
Purified S-Trim
(kDa)
anti-S2
250
150
100
75
50
S
Cellular extract
Purified S-Trim

FIG. 2B

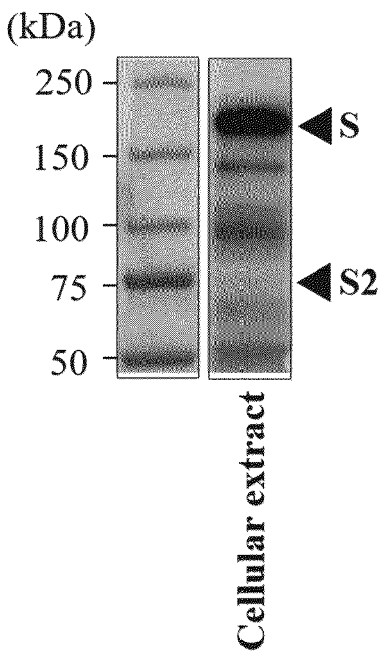
(kDa)
250
150
100
75
50
S
S2
Cellular extract

FIG. 2C

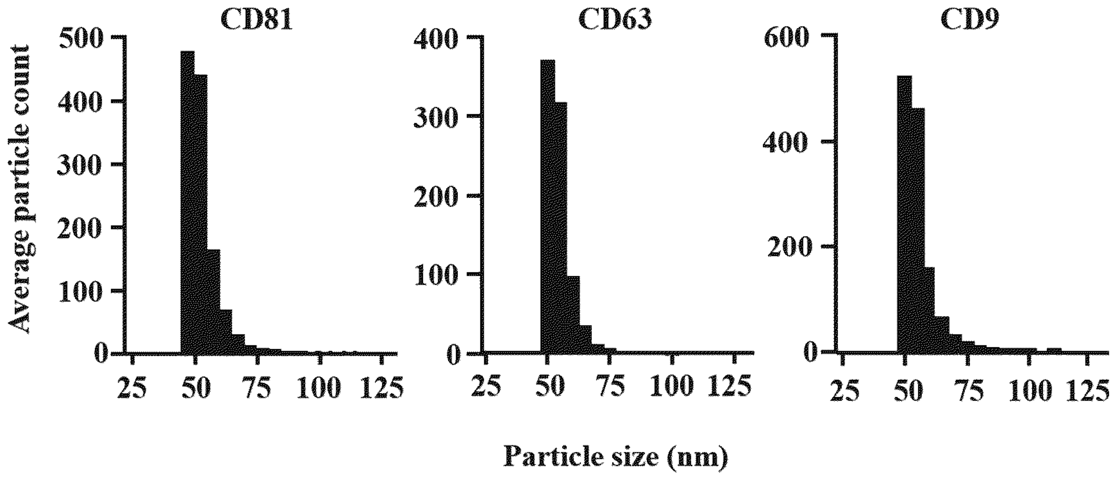
CD81
CD63
CD9
Average particle count
500
400
300
200
100
0
600
25
50
75
100
125
Particle size (nm)

FIG. 2D

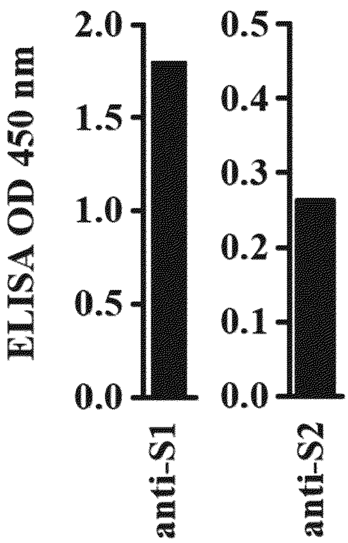
ELISA OD 450 nm
2.0
1.5
1.0
0.5
0.0
0.4
0.3
0.2
0.1
anti-S1
anti-S2

FIG. 2E

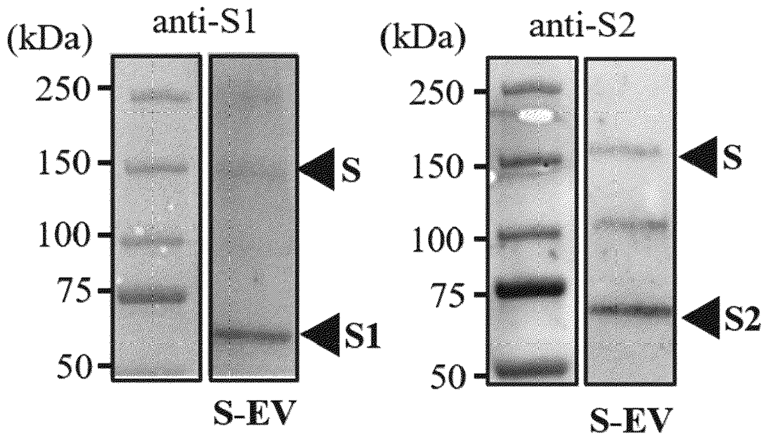
(kDa)
anti-S1
250
150
100
75
50
S
S1
S-EV
(kDa)
anti-S2
250
150
100
75
50
S
S2
S-EV

FIG. 2F

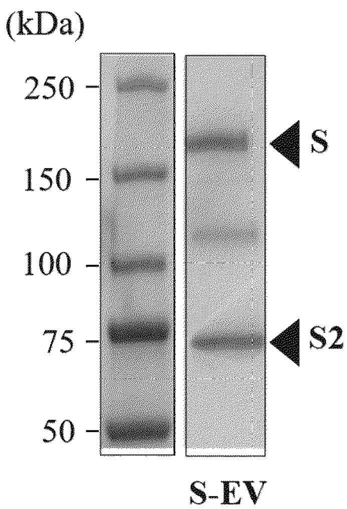
(kDa)
250
150
100
75
50
S
S2
S-EV

FIG. 2G

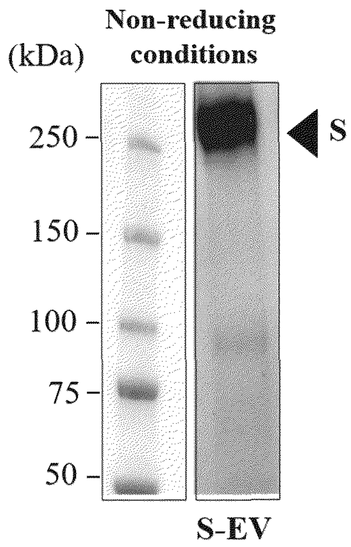
Non-reducing conditions
(kDa)
250
150
100
75
50
S
S-EV

FIG. 2H

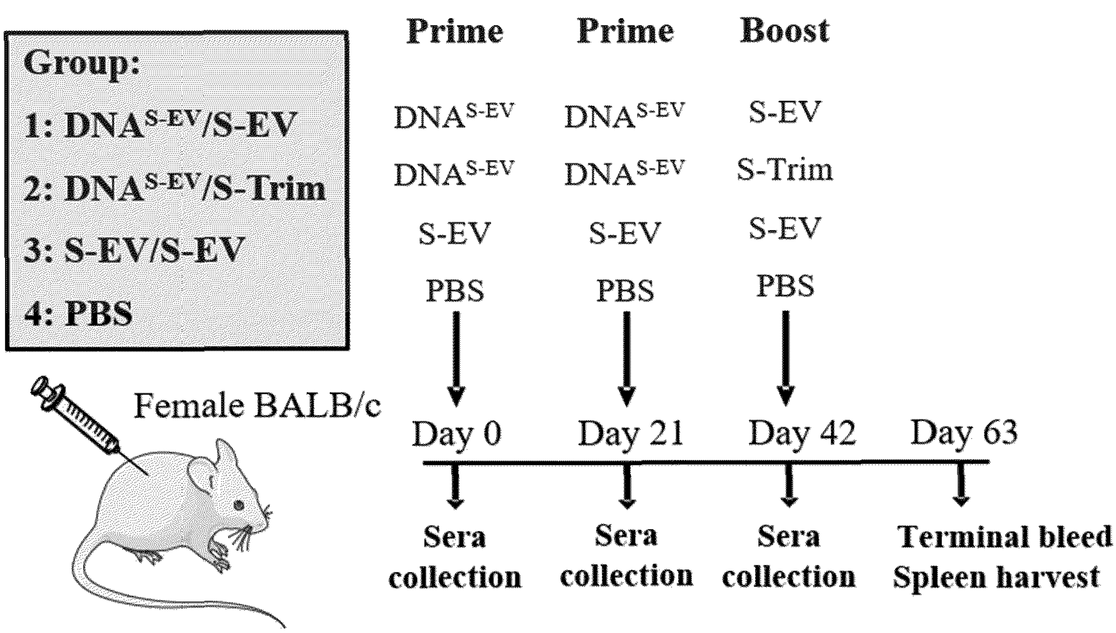
Group:
1: DNAS-EV/S-EV
2: DNAS-EV/S-Trim
3: S-EV/S-EV
4: PBS
Female BALB/c
Prime
Prime
Boost
DNAS-EV
DNAS-EV
S-EV
DNAS-EV
DNAS-EV
S-Trim
S-EV
S-EV
S-EV
PBS
PBS
PBS
Day 0
Day 21
Day 42
Day 63
Sera collection
Sera collection
Sera collection
Terminal bleed
Spleen harvest

FIG. 3A

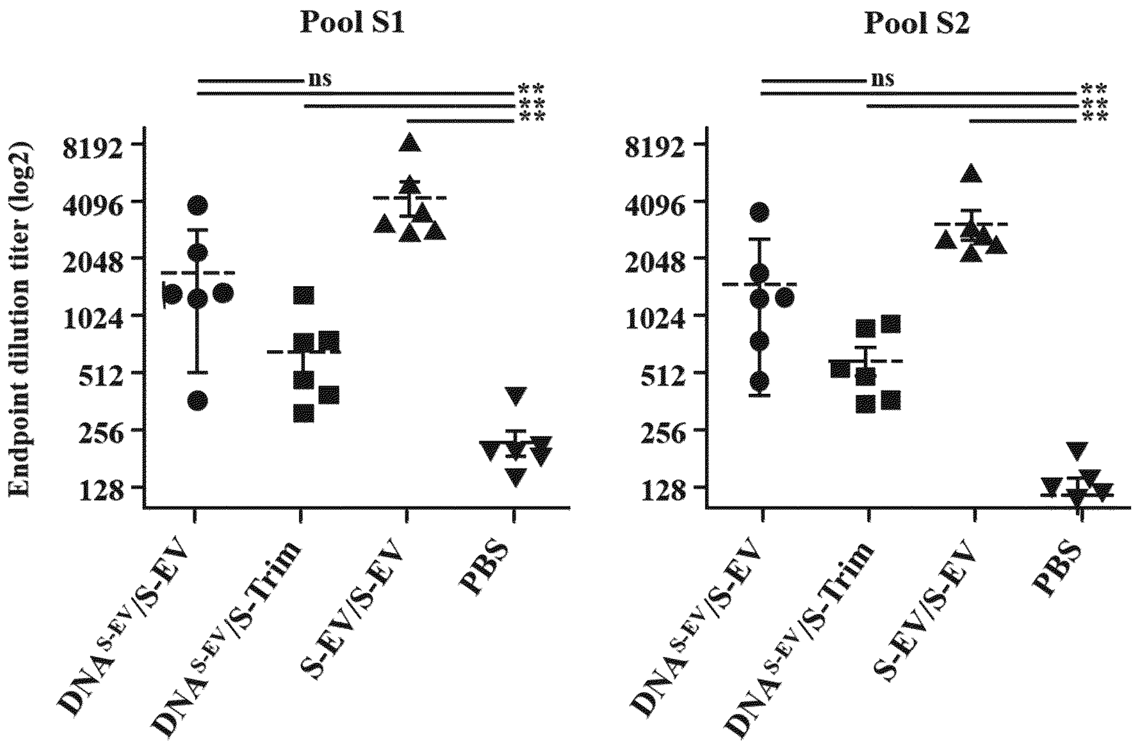
Pool S1
Pool S2
ns
**
**
**
Endpoint dilution titer (log2)
8192
4096
2048
1024
512
256
128
DNA$^{S-EV}$/S-EV
DNA$^{S-EV}$/S-Trim
S-EV/S-EV
PBS

FIG. 3B

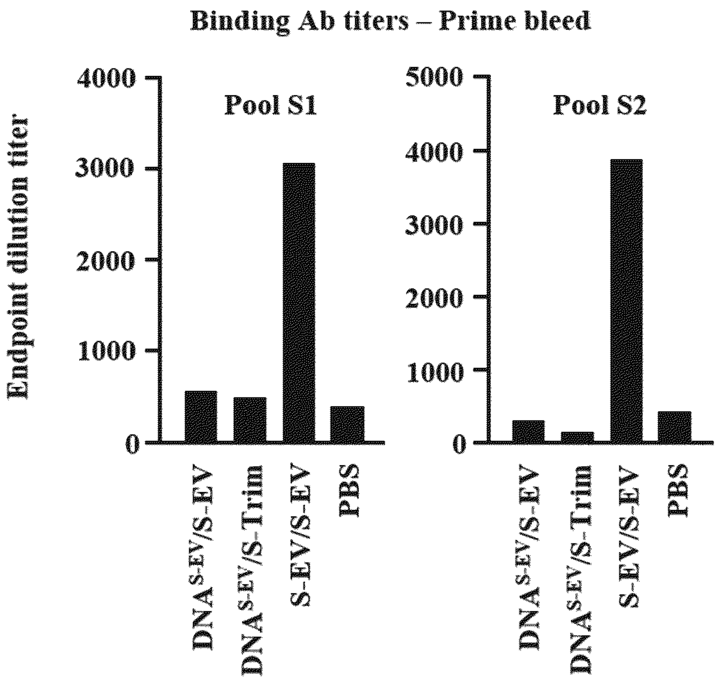
Binding Ab titers – Prime bleed
Pool S1
Pool S2
Endpoint dilution titer
4000
3000
2000
1000
0
5000
DNA$^{S-EV}$/S-EV
DNA$^{S-EV}$/S-Trim
S-EV/S-EV
PBS

FIG. 3C

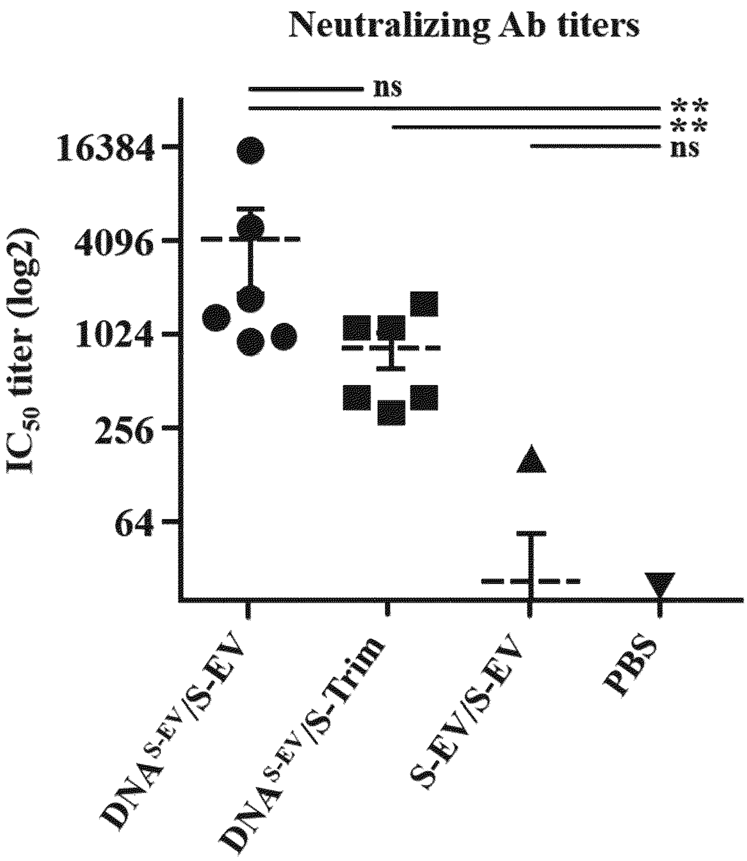
Neutralizing Ab titers
ns
**
**
ns
16384
4096
1024
256
64
IC50 titer (log2)
DNA S-EV/S-EV
DNA S-EV/S-Trim
S-EV/S-EV
PBS

FIG. 3D

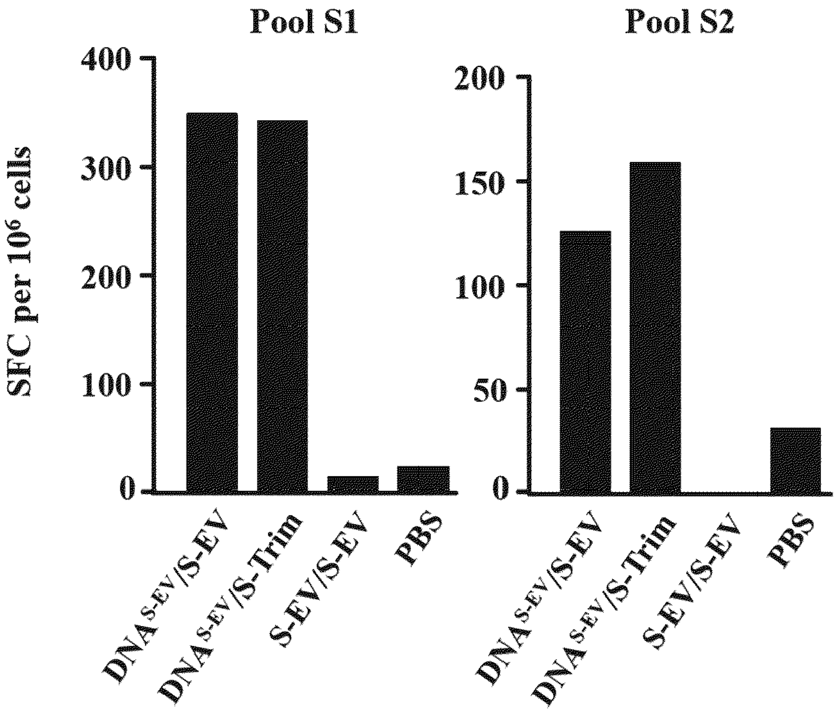
Pool S1
Pool S2
SFC per 10^6 cells
400
300
200
100
0
200
150
100
50
0
DNA S-EV/S-EV
DNA S-EV/S-Trim
S-EV/S-EV
PBS

FIG. 4

Europäisches Patentamt
European Patent Office
Office européen des brevets

# EUROPEAN SEARCH REPORT

Application Number
EP 20 30 6116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/321653 A1 (MAMOUN ROBERT ZAINE EL ABIDDINE [FR]) 20 December 2012 (2012-12-20) * paragraphs [0521], [0522] * * sequence 12 * * paragraphs [0002] - [0006], [0014], [0022], [0023] * * paragraphs [0156] - [0168], [0224], [0225], [0245] - [0247] * * figure 19; examples 1-4 * ----- | 1-3,5-15 | INV. A61K39/12 C07K14/005 |
| Y,D | WU FAN ET AL: "A new coronavirus associated with human respiratory disease in China", NATURE, vol. 579, no. 7798, 3 February 2020 (2020-02-03), pages 265-269, XP037099569, ISSN: 0028-0836, DOI: 10.1038/S41586-020-2008-3 [retrieved on 2020-02-03] * the whole document * ----- | 1-3,5-15 | |
| | | | TECHNICAL FIELDS SEARCHED (IPC) |
| X | KUATE ET AL: "Exosomal vaccines containing the S protein of the SARS coronavirus induce high levels of neutralizing antibodies", VIROLOGY, ELSEVIER, AMSTERDAM, NL, vol. 362, no. 1, 25 April 2007 (2007-04-25), pages 26-37, XP022046488, ISSN: 0042-6822, DOI: 10.1016/J.VIROL.2006.12.011 | 8,10,12 | A61K C07K C12N |
| Y | * abstract * * figure 1 * ----- -/-- | 9 | |

The present search report has been drawn up for all claims

1

| Place of search | Date of completion of the search | Examiner |
|---|---|---|
| Munich | 16 March 2021 | Lewis, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 30 6116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XIUYUAN OU ET AL: "Characterization of spike glycoprotein of SARS-CoV-2 on virus entry and its immune cross-reactivity with SARS-CoV", NATURE COMMUNICATIONS, vol. 11, no. 1, 27 March 2020 (2020-03-27), XP055724997, DOI: 10.1038/s41467-020-15562-9 * the whole document * ----- | 1-15 | |
| A,D | DE GASSART A ET AL: "Exosomal sorting of the cytoplasmic domain of bovine leukemia virus TM Env protein", CELL BIOLOGY INTERNATIONAL, vol. 33, no. 1, 1 January 2009 (2009-01-01), pages 36-48, XP025805208, ISSN: 1065-6995, DOI: 10.1016/J.CELLBI.2008.10.001 [retrieved on 2008-10-22] * the whole document * ----- | 1-15 | TECHNICAL FIELDS SEARCHED (IPC) |
| A,D | JESPER PALLESEN ET AL: "Immunogenicity and structures of a rationally designed prefusion MERS-CoV spike antigen", PROCEEDINGS OF THE NATIONAL ACADEMY OF SCIENCES, vol. 114, no. 35, 14 August 2017 (2017-08-14), pages E7348-E7357, XP055692891, US ISSN: 0027-8424, DOI: 10.1073/pnas.1707304114 * the whole document * ----- -/-- | 13 | |

The present search report has been drawn up for all claims

1

| Place of search | Date of completion of the search | Examiner |
|---|---|---|
| Munich | 16 March 2021 | Lewis, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 20 30 6116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MIR BILAL ET AL: "Extracellular Vesicles as Delivery Vehicles of Specific Cellular Cargo", CELLS, vol. 9, no. 7, 2 July 2020 (2020-07-02), page 1601, XP055785252, DOI: 10.3390/cells9071601 * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| Munich | 16 March 2021 | Lewis, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 20 30 6116

This annex lists the patent family members relating to the patent documents cited in the above-mentioned European search report. The members are as contained in the European Patent Office EDP file on
The European Patent Office is in no way liable for these particulars which are merely given for the purpose of information.

16-03-2021

| Patent document cited in search report | Publication date | Patent family member(s) | Publication date |
| --- | --- | --- | --- |
| US 2012321653 A1 | 20-12-2012 | CA 2775151 A1 | 31-03-2011 |
| | | EP 2480672 A1 | 01-08-2012 |
| | | FR 2950350 A1 | 25-03-2011 |
| | | JP 5932647 B2 | 08-06-2016 |
| | | JP 2013505713 A | 21-02-2013 |
| | | US 2012321653 A1 | 20-12-2012 |
| | | WO 2011036416 A1 | 31-03-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016138160 A
- EP 2268816 A
- US 9546371 B


### Non-patent literature cited in the description


- **MASCOLA et al.** *J Infect Dis.,* 1996, vol. 173 (2), 340-348
- **HENSLEY et al.** *Science,* 2009, vol. 326 (5953), 734-736
- **ZWICK.** *AIDS,* 2005, vol. 19 (16), 1725-1737
- **WANG et al.** *J Virol.,* 2018, vol. 92 (12), e00247-18
- **KHANNA et al.** *Biomed Res Int. 2014,* 2014, 546274
- **LIU et al.** *Protein Cell,* 2018, vol. 9 (7), 596-615
- **SRIDHAR et al.** *Nat Med.,* 2013, vol. 19 (10), 1305-1312
- **WILKINSON et al.** *Nat Med.,* 2012, vol. 18 (2), 274-280
- **MUBARAK et al.** *J Immunol Res.,* 2019, 6491738
- **XU ; GAO.** *CellMolImmunol.,* 2004, vol. 1 (2), 119-122
- **BRAUN et al.** *Nature,* 2020
- **SEKINE et al.** *Cell,* 2020
- **BARNETT et al.** *Vaccine,* 1997, vol. 15 (8), 869-873
- **PAL et al.** *Virology,* 2006, vol. 348 (2), 341-353
- **WANG et al.** *Vaccine,* 2008, vol. 26 (31), 3947-3957
- **RICHMOND et al.** *J Virol.,* 1998, vol. 72 (11), 9092-9100
- **LIU et al.** *Sci Rep.,* 2020, vol. 10 (1), 4144
- **WANG et al.** *Vaccine,* 2004, vol. 22 (27-28), 3622-3627
- **XIAO-WEN et al.** *Vaccine,* 2005, vol. 23 (14), 1649-1656
- **DESPLANTES et al.** *Sci Rep.,* 2017, vol. 7 (1), 1032
- **THÉRY et al.** *J Extracell Vesicles.,* 2018, vol. 7 (1), 1535750
- **YÁÑEZ-MÓ et al.** *J Extracell Vesicles.,* 2015, vol. 4, 27066
- **VAN NIEL et al.** *Nat Rev Mol Cell Biol.,* 2018, vol. 19 (4), 213-228
- **HOFFMANN et al.** *Cell,* 2020, vol. 181 (2), 271-280.e8
- **WRAPP et al.** *Science,* 2020, vol. 367 (6483), 1260-1263
- **WALLS et al.** *Cell,* 2020, vol. 181 (2), 281-292.e6
- **YUAN et al.** *Science,* 2020, vol. 368 (6491), 630-633
- **NEEDLEMAN ; WUNSCH.** *J Mol Biol.,* 1970, vol. 48 (3), 443-53
- **SMITH ; WATERMAN.** *Adv Appl Math.,* 1981, vol. 2 (4), 482-9
- **GRIBSKOV ; BURGESS.** *Nucleic Acids Res.,* 1986, vol. 14 (16), 6745-63
- Matrices for detecting distant relationships. **SCHWARTZ ; DAYHOFF.** Atlas of protein sequences. National Biomedical Research Foundation, 1979, vol. 5, 353-358
- **HUANG ; MILLER.** *Adv Appl Math.,* 1991, vol. 12 (3), 337-57
- **BAROUCH et al.** *J Virol.,* 2005, vol. 79 (14), 8828-8834
- **PFARR et al.** *DNA,* 1986, vol. 5 (2), 115-122
- **WHITFORD ; GUTERSTAM.** *Future Med Chem.,* 2019, vol. 11 (10), 1225-1236
- **TAYLOR ; SHAH.** *Methods,* 2015, vol. 87, 3-10
- **MEIER et al.** *J Mol Biol.,* 2004, vol. 344 (4), 1051-1069
- **GÜTHE et al.** *J Mol Biol.,* 2004, vol. 337 (4), 905-915
- **DREES et al.** *J Mol Biol.,* 1997, vol. 273 (1), 61-74
- **YANG et al.** *J Virol.,* 2000, vol. 74 (12), 5716-5725
- **YANG et al.** *J Virol.,* 2002, vol. 76 (9), 4634-4642
- **CHEN et al.** *J Virol.,* 2004, vol. 78 (9), 4508-4516
- **SHU et al.** *Biochemistry,* 1999, vol. 38 (17), 5378-5385
- **BLACKLOW et al.** *Biochemistry,* 1995, vol. 34 (46), 14955-14962

- **GODDARD et al.** *Gene Ther.,* 1997, vol. 4 (11), 1231-1236
- **GORMAN et al.** *Gene Ther.,* 1997, vol. 4 (9), 983-992
- **CHADWICK et al.** *Gene Ther.,* 1997, vol. 4 (9), 937-942
- **GOKHALE et al.** *Gene Ther.,* 1997, vol. 4 (12), 1289-1299
- **GAO ; HUANG.** *Gene Ther.,* 1995, vol. 2 (10), 710-722
- Guidance for Industry. July 2005
- **STROHL.** *BioDrugs,* 2015, vol. 29 (4), 215-239
- **DE GASSART et al.** *Cell Biol Int.,* 2009, vol. 33 (1), 36-48
- **WU et al.** *Nature,* 2020, vol. 579 (7798), 265-269
- **JOHNSON et al.** *J Virol.,* 2020
- **CONNOR et al.** *Virology,* 1995, vol. 206 (2), 935-944
- **PALLESEN et al.** *Proc Natl Acad Sci USA.,* 2017, vol. 114 (35), E7348-E7357
- **DESPLANTES et al.** *SciRep,* 2017, vol. 7 (1), 1032
- **MATEUS et al.** *Science,* 2020
- **WANG et al.** *J Virol.,* 2004, vol. 78 (11), 5612-5618
- **PARK et al.** *Proc Natl Acad Sci USA.,* 2016, vol. 113 (43), 12262-12267
- **MOULARD et al.** *Virus Res.,* 1999, vol. 60 (1), 55-65
- **FOLEGATTI et al.** *Lancet,* 2020, vol. 396 (10249), 467-478
- **JACKSON et al.** *N Engl J Med.,* 2020
- **YANG et al.** *Nature,* 2020
- **KORBER et al.** *Cell,* 2020, vol. 182 (4), 812-827
- **BECERRA-FLORES ; CARDOZO.** *Int J Clin Pract.,* 2020, e13525

SEQUENCE LISTING

<110> CILOA

<120> EXTRACELLULAR VESICLES HARBORING A SPIKE PROTEIN, NUCLEIC ACIDS FOR PRODUCING THE SAME, AND METHOD OF IMMUNIZING A SUBJECT AGAINST SARS-CoV-2 USING THE SAME

<130> CV - 1674/EP

<160> 56

<170> BiSSAP 1.3.6

<210> 1
<211> 3822
<212> DNA
<213> SARS-CoV-2 Wuhan-Hu-1 isolate

<220>
<223> S gene

<300>
<308> GenBank accession number MN908947.3
<309> Version 3 of March 18, 2020

<400> 1
atgtttgttt ttcttgtttt attgccacta gtctctagtc agtgtgttaa tcttacaacc 60

agaactcaat taccccctgc atacactaat tctttcacac gtggtgttta ttaccctgac 120

aaagttttca gatcctcagt tttacattca actcaggact tgttcttacc tttcttttcc 180

aatgttactt ggttccatgc tatacatgtc tctgggacca atggtactaa gaggtttgat 240

aaccctgtcc taccatttaa tgatggtgtt tattttgctt ccactgagaa gtctaacata 300

ataagaggct ggatttttgg tactacttta gattcgaaga cccagtccct acttattgtt 360

aataacgcta ctaatgttgt tattaaagtc tgtgaatttc aattttgtaa tgatccattt 420

ttgggtgttt attaccacaa aaacaacaaa agttggatgg aaagtgagtt cagagtttat 480

tctagtgcga ataattgcac ttttgaatat gtctctcagc cttttcttat ggaccttgaa 540

ggaaaacagg gtaatttcaa aaatcttagg gaatttgtgt ttaagaatat tgatggttat 600

tttaaaatat attctaagca cacgcctatt aatttagtgc gtgatctccc tcagggtttt 660

tcggctttag aaccattggt agatttgcca ataggtatta acatcactag gtttcaaact 720

ttacttgctt tacatagaag ttatttgact cctggtgatt cttcttcagg ttggacagct 780

ggtgctgcag cttattatgt gggttatctt caacctagga cttttctatt aaaatataat 840

gaaaatggaa ccattacaga tgctgtagac tgtgcacttg accctctctc agaaacaaag 900

tgtacgttga aatccttcac tgtagaaaaa ggaatctatc aaacttctaa ctttagagtc 960

caaccaacag aatctattgt tagatttcct aatattacaa acttgtgccc ttttggtgaa 1020

gtttttaacg ccaccagatt tgcatctgtt tatgcttgga acaggaagag aatcagcaac 1080

tgtgttgctg attattctgt cctatataat tccgcatcat tttccacttt taagtgttat 1140
ggagtgtctc ctactaaatt aaatgatctc tgctttacta atgtctatgc agattcattt 1200
gtaattagag gtgatgaagt cagacaaatc gctccagggc aaactggaaa gattgctgat 1260
tataattata aattaccaga tgattttaca ggctgcgtta tagcttggaa ttctaacaat 1320
cttgattcta aggttggtgg taattataat tacctgtata gattgtttag gaagtctaat 1380
ctcaaacctt ttgagagaga tatttcaact gaaatctatc aggccggtag cacaccttgt 1440
aatggtgttg aaggttttaa ttgttacttt cctttacaat catatggttt ccaacccact 1500
aatggtgttg gttaccaacc atacagagta gtagtacttt cttttgaact tctacatgca 1560
ccagcaactg tttgtggacc taaaaagtct actaatttgg ttaaaaacaa atgtgtcaat 1620
ttcaacttca atggtttaac aggcacaggt gttcttactg agtctaacaa aaagtttctg 1680
cctttccaac aatttggcag agacattgct gacactactg atgctgtccg tgatccacag 1740
acacttgaga ttcttgacat tacaccatgt tcttttggtg gtgtcagtgt tataacacca 1800
ggaacaaata cttctaacca ggttgctgtt ctttatcagg atgttaactg cacagaagtc 1860
cctgttgcta ttcatgcaga tcaacttact cctacttggc gtgtttattc tacaggttct 1920
aatgtttttc aaacacgtgc aggctgttta ataggggctg aacatgtcaa caactcatat 1980
gagtgtgaca tacccattgg tgcaggtata tgcgctagtt atcagactca gactaattct 2040
cctcggcggg cacgtagtgt agctagtcaa tccatcattg cctacactat gtcacttggt 2100
gcagaaaatt cagttgctta ctctaataac tctattgcca tacccacaaa ttttactatt 2160
agtgttacca cagaaattct accagtgtct atgaccaaga catcagtaga ttgtacaatg 2220
tacatttgtg gtgattcaac tgaatgcagc aatcttttgt tgcaatatgg cagtttttgt 2280
acacaattaa accgtgcttt aactggaata gctgttgaac aagacaaaaa cacccaagaa 2340
gtttttgcac aagtcaaaca aatttacaaa acaccaccaa ttaaagattt tggtggtttt 2400
aatttttcac aaatattacc agatccatca aaaccaagca agaggtcatt tattgaagat 2460
ctacttttca acaaagtgac acttgcagat gctggcttca tcaaacaata tggtgattgc 2520
cttggtgata ttgctgctag agacctcatt tgtgcacaaa agtttaacgg ccttactgtt 2580
ttgccacctt tgctcacaga tgaaatgatt gctcaataca cttctgcact gttagcgggt 2640
acaatcactt ctggttggac ctttggtgca ggtgctgcat tacaaatacc atttgctatg 2700
caaatggctt ataggtttaa tggtattgga gttacacaga atgttctcta tgagaaccaa 2760
aaattgattg ccaaccaatt taatagtgct attggcaaaa ttcaagactc actttcttcc 2820
acagcaagtg cacttggaaa acttcaagat gtggtcaacc aaaatgcaca agctttaaac 2880
acgcttgtta aacaacttag ctccaatttt ggtgcaattt caagtgtttt aaatgatatc 2940

ctttcacgtc ttgacaaagt tgaggctgaa gtgcaaattg ataggttgat cacaggcaga 3000

cttcaaagtt tgcagacata tgtgactcaa caattaatta gagctgcaga aatcagagct 3060

tctgctaatc ttgctgctac taaaatgtca gagtgtgtac ttggacaatc aaaaagagtt 3120

gatttttgtg gaaagggcta tcatcttatg tccttccctc agtcagcacc tcatggtgta 3180

gtcttcttgc atgtgactta tgtccctgca caagaaaaga acttcacaac tgctcctgcc 3240

atttgtcatg atggaaaagc acactttcct cgtgaaggtg tctttgtttc aaatggcaca 3300

cactggtttg taacacaaag gaatttttat gaaccacaaa tcattactac agacaacaca 3360

tttgtgtctg gtaactgtga tgttgtaata ggaattgtca acaacacagt ttatgatcct 3420

ttgcaacctg aattagactc attcaaggag gagttagata aatattttaa gaatcataca 3480

tcaccagatg ttgatttagg tgacatctct ggcattaatg cttcagttgt aaacattcaa 3540

aaagaaattg accgcctcaa tgaggttgcc aagaatttaa atgaatctct catcgatctc 3600

caagaacttg gaaagtatga gcagtatata aaatggccat ggtacatttg gctaggtttt 3660

atagctggct tgattgccat agtaatggtg acaattatgc tttgctgtat gaccagttgc 3720

tgtagttgtc tcaagggctg ttgttcttgt ggatcctgct gcaaatttga tgaagacgac 3780

tctgagccag tgctcaaagg agtcaaatta cattacacat aa 3822

<210> 2
<211> 1273
<212> PRT
<213> SARS-CoV-2 Wuhan-Hu-1 isolate

<220>
<223> Spike protein encoded by SEQ ID NO: 1

<400> 2
Met Phe Val Phe Leu Val Leu Leu Pro Leu Val Ser Ser Gln Cys Val
1 5 10 15
Asn Leu Thr Thr Arg Thr Gln Leu Pro Pro Ala Tyr Thr Asn Ser Phe
20 25 30
Thr Arg Gly Val Tyr Tyr Pro Asp Lys Val Phe Arg Ser Ser Val Leu
35 40 45
His Ser Thr Gln Asp Leu Phe Leu Pro Phe Phe Ser Asn Val Thr Trp
50 55 60
Phe His Ala Ile His Val Ser Gly Thr Asn Gly Thr Lys Arg Phe Asp
65 70 75 80
Asn Pro Val Leu Pro Phe Asn Asp Gly Val Tyr Phe Ala Ser Thr Glu
85 90 95
Lys Ser Asn Ile Ile Arg Gly Trp Ile Phe Gly Thr Thr Leu Asp Ser
100 105 110
Lys Thr Gln Ser Leu Leu Ile Val Asn Asn Ala Thr Asn Val Val Ile
115 120 125
Lys Val Cys Glu Phe Gln Phe Cys Asn Asp Pro Phe Leu Gly Val Tyr
130 135 140
Tyr His Lys Asn Asn Lys Ser Trp Met Glu Ser Glu Phe Arg Val Tyr
145 150 155 160
Ser Ser Ala Asn Asn Cys Thr Phe Glu Tyr Val Ser Gln Pro Phe Leu
165 170 175

Met Asp Leu Glu Gly Lys Gln Gly Asn Phe Lys Asn Leu Arg Glu Phe
180 185 190
Val Phe Lys Asn Ile Asp Gly Tyr Phe Lys Ile Tyr Ser Lys His Thr
195 200 205
Pro Ile Asn Leu Val Arg Asp Leu Pro Gln Gly Phe Ser Ala Leu Glu
210 215 220
Pro Leu Val Asp Leu Pro Ile Gly Ile Asn Ile Thr Arg Phe Gln Thr
225 230 235 240
Leu Leu Ala Leu His Arg Ser Tyr Leu Thr Pro Gly Asp Ser Ser Ser
245 250 255
Gly Trp Thr Ala Gly Ala Ala Ala Tyr Tyr Val Gly Tyr Leu Gln Pro
260 265 270
Arg Thr Phe Leu Leu Lys Tyr Asn Glu Asn Gly Thr Ile Thr Asp Ala
275 280 285
Val Asp Cys Ala Leu Asp Pro Leu Ser Glu Thr Lys Cys Thr Leu Lys
290 295 300
Ser Phe Thr Val Glu Lys Gly Ile Tyr Gln Thr Ser Asn Phe Arg Val
305 310 315 320
Gln Pro Thr Glu Ser Ile Val Arg Phe Pro Asn Ile Thr Asn Leu Cys
325 330 335
Pro Phe Gly Glu Val Phe Asn Ala Thr Arg Phe Ala Ser Val Tyr Ala
340 345 350
Trp Asn Arg Lys Arg Ile Ser Asn Cys Val Ala Asp Tyr Ser Val Leu
355 360 365
Tyr Asn Ser Ala Ser Phe Ser Thr Phe Lys Cys Tyr Gly Val Ser Pro
370 375 380
Thr Lys Leu Asn Asp Leu Cys Phe Thr Asn Val Tyr Ala Asp Ser Phe
385 390 395 400
Val Ile Arg Gly Asp Glu Val Arg Gln Ile Ala Pro Gly Gln Thr Gly
405 410 415
Lys Ile Ala Asp Tyr Asn Tyr Lys Leu Pro Asp Asp Phe Thr Gly Cys
420 425 430
Val Ile Ala Trp Asn Ser Asn Asn Leu Asp Ser Lys Val Gly Gly Asn
435 440 445
Tyr Asn Tyr Leu Tyr Arg Leu Phe Arg Lys Ser Asn Leu Lys Pro Phe
450 455 460
Glu Arg Asp Ile Ser Thr Glu Ile Tyr Gln Ala Gly Ser Thr Pro Cys
465 470 475 480
Asn Gly Val Glu Gly Phe Asn Cys Tyr Phe Pro Leu Gln Ser Tyr Gly
485 490 495
Phe Gln Pro Thr Asn Gly Val Gly Tyr Gln Pro Tyr Arg Val Val Val
500 505 510
Leu Ser Phe Glu Leu Leu His Ala Pro Ala Thr Val Cys Gly Pro Lys
515 520 525
Lys Ser Thr Asn Leu Val Lys Asn Lys Cys Val Asn Phe Asn Phe Asn
530 535 540
Gly Leu Thr Gly Thr Gly Val Leu Thr Glu Ser Asn Lys Lys Phe Leu
545 550 555 560
Pro Phe Gln Gln Phe Gly Arg Asp Ile Ala Asp Thr Thr Asp Ala Val
565 570 575
Arg Asp Pro Gln Thr Leu Glu Ile Leu Asp Ile Thr Pro Cys Ser Phe
580 585 590
Gly Gly Val Ser Val Ile Thr Pro Gly Thr Asn Thr Ser Asn Gln Val
595 600 605
Ala Val Leu Tyr Gln Asp Val Asn Cys Thr Glu Val Pro Val Ala Ile
610 615 620
His Ala Asp Gln Leu Thr Pro Thr Trp Arg Val Tyr Ser Thr Gly Ser
625 630 635 640
Asn Val Phe Gln Thr Arg Ala Gly Cys Leu Ile Gly Ala Glu His Val
645 650 655
Asn Asn Ser Tyr Glu Cys Asp Ile Pro Ile Gly Ala Gly Ile Cys Ala
660 665 670
Ser Tyr Gln Thr Gln Thr Asn Ser Pro Arg Arg Ala Arg Ser Val Ala

```
        675                 680                 685
Ser Gln Ser Ile Ile Ala Tyr Thr Met Ser Leu Gly Ala Glu Asn Ser
    690                 695                 700
Val Ala Tyr Ser Asn Asn Ser Ile Ala Ile Pro Thr Asn Phe Thr Ile
705                 710                 715                 720
Ser Val Thr Thr Glu Ile Leu Pro Val Ser Met Thr Lys Thr Ser Val
                725                 730                 735
Asp Cys Thr Met Tyr Ile Cys Gly Asp Ser Thr Glu Cys Ser Asn Leu
            740                 745                 750
Leu Leu Gln Tyr Gly Ser Phe Cys Thr Gln Leu Asn Arg Ala Leu Thr
        755                 760                 765
Gly Ile Ala Val Glu Gln Asp Lys Asn Thr Gln Glu Val Phe Ala Gln
    770                 775                 780
Val Lys Gln Ile Tyr Lys Thr Pro Pro Ile Lys Asp Phe Gly Gly Phe
785                 790                 795                 800
Asn Phe Ser Gln Ile Leu Pro Asp Pro Ser Lys Pro Ser Lys Arg Ser
                805                 810                 815
Phe Ile Glu Asp Leu Leu Phe Asn Lys Val Thr Leu Ala Asp Ala Gly
            820                 825                 830
Phe Ile Lys Gln Tyr Gly Asp Cys Leu Gly Asp Ile Ala Ala Arg Asp
        835                 840                 845
Leu Ile Cys Ala Gln Lys Phe Asn Gly Leu Thr Val Leu Pro Pro Leu
    850                 855                 860
Leu Thr Asp Glu Met Ile Ala Gln Tyr Thr Ser Ala Leu Leu Ala Gly
865                 870                 875                 880
Thr Ile Thr Ser Gly Trp Thr Phe Gly Ala Gly Ala Ala Leu Gln Ile
                885                 890                 895
Pro Phe Ala Met Gln Met Ala Tyr Arg Phe Asn Gly Ile Gly Val Thr
            900                 905                 910
Gln Asn Val Leu Tyr Glu Asn Gln Lys Leu Ile Ala Asn Gln Phe Asn
        915                 920                 925
Ser Ala Ile Gly Lys Ile Gln Asp Ser Leu Ser Ser Thr Ala Ser Ala
    930                 935                 940
Leu Gly Lys Leu Gln Asp Val Val Asn Gln Asn Ala Gln Ala Leu Asn
945                 950                 955                 960
Thr Leu Val Lys Gln Leu Ser Ser Asn Phe Gly Ala Ile Ser Ser Val
                965                 970                 975
Leu Asn Asp Ile Leu Ser Arg Leu Asp Lys Val Glu Ala Glu Val Gln
            980                 985                 990
Ile Asp Arg Leu Ile Thr Gly Arg Leu Gln Ser Leu Gln Thr Tyr Val
        995                 1000                1005
Thr Gln Gln Leu Ile Arg Ala Ala Glu Ile Arg Ala Ser Ala Asn Leu
    1010                1015                1020
Ala Ala Thr Lys Met Ser Glu Cys Val Leu Gly Gln Ser Lys Arg Val
1025                1030                1035                1040
Asp Phe Cys Gly Lys Gly Tyr His Leu Met Ser Phe Pro Gln Ser Ala
                1045                1050                1055
Pro His Gly Val Val Phe Leu His Val Thr Tyr Val Pro Ala Gln Glu
            1060                1065                1070
Lys Asn Phe Thr Thr Ala Pro Ala Ile Cys His Asp Gly Lys Ala His
        1075                1080                1085
Phe Pro Arg Glu Gly Val Phe Val Ser Asn Gly Thr His Trp Phe Val
    1090                1095                1100
Thr Gln Arg Asn Phe Tyr Glu Pro Gln Ile Ile Thr Thr Asp Asn Thr
1105                1110                1115                1120
Phe Val Ser Gly Asn Cys Asp Val Val Ile Gly Ile Val Asn Asn Thr
                1125                1130                1135
Val Tyr Asp Pro Leu Gln Pro Glu Leu Asp Ser Phe Lys Glu Glu Leu
            1140                1145                1150
Asp Lys Tyr Phe Lys Asn His Thr Ser Pro Asp Val Asp Leu Gly Asp
        1155                1160                1165
Ile Ser Gly Ile Asn Ala Ser Val Val Asn Ile Gln Lys Glu Ile Asp
    1170                1175                1180
```

```
Arg Leu Asn Glu Val Ala Lys Asn Leu Asn Glu Ser Leu Ile Asp Leu
1185                1190                1195                1200
Gln Glu Leu Gly Lys Tyr Glu Gln Tyr Ile Lys Trp Pro Trp Tyr Ile
                1205                1210                1215
Trp Leu Gly Phe Ile Ala Gly Leu Ile Ala Ile Val Met Val Thr Ile
            1220                1225                1230
Met Leu Cys Cys Met Thr Ser Cys Cys Ser Cys Leu Lys Gly Cys Cys
        1235                1240                1245
Ser Cys Gly Ser Cys Cys Lys Phe Asp Glu Asp Asp Ser Glu Pro Val
    1250                1255                1260
Leu Lys Gly Val Lys Leu His Tyr Thr
1265                1270

<210> 3
<211> 69
<212> DNA
<213> Artificial Sequence


<220>
<223> mGluR5

<400> 3
atggtgctgc tgctgatcct gtctgtgctg ctcctgaaag aagatgtgcg gggctctgcc     60

cagagcacc                                                             69


<210> 4
<211> 23
<212> PRT
<213> Artificial Sequence


<220>
<223> mGluR5

<400> 4
Met Val Leu Leu Leu Ile Leu Ser Val Leu Leu Leu Lys Glu Asp Val
1               5                   10                  15
Arg Gly Ser Ala Gln Ser Thr
            20

<210> 5
<211> 3735
<212> DNA
<213> Artificial Sequence


<220>
<223> Variant of the S gene

<400> 5
atggtgctgc tgctgatcct gtctgtgctg ctcctgaaag aagatgtgcg gggctctgcc      60

cagagcaccg gtcaatgtgt gaacctgacc accagaacac agctgcctcc agcctacacc     120

aacagcttca ccagaggcgt gtactacccc gacaaggtgt tcagatccag cgtgctgcac     180

tctacccagg acctgttcct gcctttcttc agcaacgtga cctggttcca cgccatccac     240

gtgtccggca ccaatggcac caagagattc gacaaccccg tgctgccctt caacgacggg     300
```

gtgtactttg ccagcaccga gaagtccaac atcatcagag gctggatctt cggcaccaca 360
ctggacagca agacccagag cctgctgatc gtgaacaacg ccaccaacgt ggtcatcaaa 420
gtgtgcgagt tccagttctg caacgacccc ttcctgggcg tctactacca caagaacaac 480
aagagctgga tggaaagcga gttccgggtg tacagcagcg ccaacaactg caccttcgag 540
tacgtgtccc agcctttcct gatggacctg gaaggcaagc agggcaactt caagaacctg 600
cgcgagttcg tgttcaagaa catcgacggc tacttcaaga tctacagcaa gcacacccct 660
atcaacctcg tgcgggatct gcctcagggc ttctctgctc tggaacccct ggtggatctg 720
cccatcggca tcaacatcac ccggtttcag acactgctgg ccctgcacag aagctacctg 780
acacctggcg atagcagctc tggatggaca gctggcgccg ctgcctacta tgtgggatac 840
ctgcagcctc ggaccttcct gctgaagtac aacgagaacg gcaccatcac cgacgccgtg 900
gattgtgctc tggatcctct gagcgagaca aagtgcaccc tgaagtcctt caccgtggaa 960
aagggcatct accagaccag caacttccgg gtgcagccca ccgaatccat cgtgcggttc 1020
cccaatatca ccaatctgtg ccccttcggc gaggtgttca atgccaccag attcgcctct 1080
gtgtacgcct ggaaccggaa gcggatcagc aattgcgtgg ccgactactc cgtgctgtac 1140
aactccgcca gcttcagcac cttcaagtgc tacggcgtgt cccctaccaa gctgaacgac 1200
ctgtgcttca caaacgtgta cgccgacagc ttcgtgatcc ggggagatga agtgcggcag 1260
attgcccctg gacagacagg caagatcgcc gactacaact acaagctgcc cgacgacttc 1320
accggctgtg tgattgcctg gaacagcaac aacctggact ccaaagtcgg cggcaactac 1380
aattacctgt accggctgtt ccggaagtcc aatctgaagc ccttcgagcg ggacatctcc 1440
accgagatct atcaggccgg cagcacccct tgtaacggcg tggaaggctt caactgctac 1500
ttcccactgc agtcctacgg ctttcagccc acaaatggcg tgggctacca gccttacaga 1560
gtggtggtgc tgagcttcga gctgctgcat gctcctgcca cagtgtgcgg ccctaagaaa 1620
agcaccaatc tcgtgaagaa caaatgcgtg aacttcaact tcaacggcct gaccggcacc 1680
ggcgtgctga cagagagcaa caagaagttc ctgccattcc agcagttcgg ccgggatatc 1740
gccgatacca cagatgccgt cagagatccc cagacactgg aaatcctgga catcacccct 1800
tgcagcttcg gcggagtgtc tgtgatcacc cctggcacca acaccagcaa tcaggtggca 1860
gtgctgtacc aggacgtgaa ctgtaccgaa gtgcccgtgg ccattcacgc cgatcagctg 1920
acacctacat ggcgggtgta ctccaccggc agcaatgtgt ttcagaccag agccggctgt 1980
ctgatcggag ccgagcacgt gaacaatagc tacgagtgcg acatccccat cggcgctggc 2040
atctgcgcct cttaccagac acagacaaac agccccagac gggccagatc tgtggccagc 2100
cagagcatca ttgcctacac aatgtctctg ggcgccgaga acagcgtggc ctactccaac 2160
aactctatcg ctatccccac caacttcacc atcagcgtga ccacagagat cctgcctgtg 2220

tccatgacca agaccagcgt ggactgcacc atgtacatct gcggcgattc caccgagtgc 2280
tccaacctgc tgctgcagta cggcagcttc tgcacccagc tgaatagagc cctgacaggg 2340
atcgccgtgg aacaggatgc caataccggc gaagtgttcg cccaagtgaa gcagatctac 2400
aagacccctc ctatcaagga cttcggcggc ttcaatttca gccagattct gcccgatcct 2460
agcaagccca gcaagcggag cttcatcgag gacctgctgt tcaacaaagt gacactggcc 2520
gacgccggct tcatcaagca gtatggcgat tgtctgggcg acattgccgc cagggatctg 2580
atttgcgccc agaagtttaa cggactgaca gtgctgcctc ctctgctgac cgatgagatg 2640
atcgcccagt acacatctgc cctgctggcc ggcacaatca caagcggctg gacatttgga 2700
gctggcgctg ccctgcagat ccccttttgct atgcagatgg cctacagatt caacggcatc 2760
ggagtgaccc agaatgtgct gtacgagaac cagaagctga tcgccaacca gttcaacagc 2820
gccatcggca agatccagga cagcctgtct agcacagcca gcgctctggg aaagctgcag 2880
gacgtggtca accagaatgc ccaggcactg aacaccctgg tcaagcagct gagcagcaat 2940
ttcggcgcca tcagctctgt gctgaacgat atcctgagca gactggaccc tcctgaggcc 3000
gaggtgcaga tcgacagact gatcaccgga aggctgcagt ccctgcagac ctacgttacc 3060
cagcagctga tcagagccgc cgagattaga gcctctgcca atctggccgc caccaagatg 3120
tctgagtgtg tgctgggcca gagcaagaga gtggactttt gcggcaaggg ctaccacctg 3180
atgagcttcc ctcagtctgc ccctcacggc gtggtgtttc tgcacgtgac atacgtgccc 3240
gctcaagaga agaatttcac caccgctcca gccatctgcc acgacggcaa agcccacttt 3300
cctagagaag gcgtgttcgt gtccaacggc acccattggt tcgtgaccca gcggaacttc 3360
tacgagcccc agatcatcac caccgacaac accttcgtgt ctggcaactg cgacgtcgtg 3420
atcggcattg tgaacaatac cgtgtacgac cctctgcagc ccgagctgga cagcttcaaa 3480
gaggaactgg ataagtactt taagaaccac acaagccccg acgtggacct gggcgatatc 3540
agcggaatca atgccagcgt cgtgaacatc cagaaagaga tcgaccggct gaacgaggtg 3600
gccaagaatc tgaacgagag cctgatcgac ctgcaggaac tggggaagta cgagcagtac 3660
atcaagtggc cctggtacat ctggctgggc tttatcgccg gactgattgc catcgtgatg 3720
gtcacaatca tgctg 3735

<210> 6
<211> 1245
<212> PRT
<213> Artificial Sequence

<220>
<223> Variant Spike protein encoded by SEQ ID NO: 5

<400> 6

```
Met Val Leu Leu Leu Ile Leu Ser Val Leu Leu Leu Lys Glu Asp Val
1               5                   10                  15
Arg Gly Ser Ala Gln Ser Thr Gly Gln Cys Val Asn Leu Thr Thr Arg
            20                  25                  30
Thr Gln Leu Pro Pro Ala Tyr Thr Asn Ser Phe Thr Arg Gly Val Tyr
        35                  40                  45
Tyr Pro Asp Lys Val Phe Arg Ser Ser Val Leu His Ser Thr Gln Asp
    50                  55                  60
Leu Phe Leu Pro Phe Phe Ser Asn Val Thr Trp Phe His Ala Ile His
65                  70                  75                  80
Val Ser Gly Thr Asn Gly Thr Lys Arg Phe Asp Asn Pro Val Leu Pro
                85                  90                  95
Phe Asn Asp Gly Val Tyr Phe Ala Ser Thr Glu Lys Ser Asn Ile Ile
            100                 105                 110
Arg Gly Trp Ile Phe Gly Thr Thr Leu Asp Ser Lys Thr Gln Ser Leu
        115                 120                 125
Leu Ile Val Asn Asn Ala Thr Asn Val Val Ile Lys Val Cys Glu Phe
    130                 135                 140
Gln Phe Cys Asn Asp Pro Phe Leu Gly Val Tyr Tyr His Lys Asn Asn
145                 150                 155                 160
Lys Ser Trp Met Glu Ser Glu Phe Arg Val Tyr Ser Ser Ala Asn Asn
                165                 170                 175
Cys Thr Phe Glu Tyr Val Ser Gln Pro Phe Leu Met Asp Leu Glu Gly
            180                 185                 190
Lys Gln Gly Asn Phe Lys Asn Leu Arg Glu Phe Val Phe Lys Asn Ile
        195                 200                 205
Asp Gly Tyr Phe Lys Ile Tyr Ser Lys His Thr Pro Ile Asn Leu Val
    210                 215                 220
Arg Asp Leu Pro Gln Gly Phe Ser Ala Leu Glu Pro Leu Val Asp Leu
225                 230                 235                 240
Pro Ile Gly Ile Asn Ile Thr Arg Phe Gln Thr Leu Leu Ala Leu His
                245                 250                 255
Arg Ser Tyr Leu Thr Pro Gly Asp Ser Ser Ser Gly Trp Thr Ala Gly
            260                 265                 270
Ala Ala Ala Tyr Tyr Val Gly Tyr Leu Gln Pro Arg Thr Phe Leu Leu
        275                 280                 285
Lys Tyr Asn Glu Asn Gly Thr Ile Thr Asp Ala Val Asp Cys Ala Leu
    290                 295                 300
Asp Pro Leu Ser Glu Thr Lys Cys Thr Leu Lys Ser Phe Thr Val Glu
305                 310                 315                 320
Lys Gly Ile Tyr Gln Thr Ser Asn Phe Arg Val Gln Pro Thr Glu Ser
                325                 330                 335
Ile Val Arg Phe Pro Asn Ile Thr Asn Leu Cys Pro Phe Gly Glu Val
            340                 345                 350
Phe Asn Ala Thr Arg Phe Ala Ser Val Tyr Ala Trp Asn Arg Lys Arg
        355                 360                 365
Ile Ser Asn Cys Val Ala Asp Tyr Ser Val Leu Tyr Asn Ser Ala Ser
    370                 375                 380
Phe Ser Thr Phe Lys Cys Tyr Gly Val Ser Pro Thr Lys Leu Asn Asp
385                 390                 395                 400
Leu Cys Phe Thr Asn Val Tyr Ala Asp Ser Phe Val Ile Arg Gly Asp
                405                 410                 415
Glu Val Arg Gln Ile Ala Pro Gly Gln Thr Gly Lys Ile Ala Asp Tyr
            420                 425                 430
Asn Tyr Lys Leu Pro Asp Asp Phe Thr Gly Cys Val Ile Ala Trp Asn
        435                 440                 445
Ser Asn Asn Leu Asp Ser Lys Val Gly Gly Asn Tyr Asn Tyr Leu Tyr
    450                 455                 460
Arg Leu Phe Arg Lys Ser Asn Leu Lys Pro Phe Glu Arg Asp Ile Ser
465                 470                 475                 480
Thr Glu Ile Tyr Gln Ala Gly Ser Thr Pro Cys Asn Gly Val Glu Gly
                485                 490                 495
```

Phe Asn Cys Tyr Phe Pro Leu Gln Ser Tyr Gly Phe Gln Pro Thr Asn
500 505 510
Gly Val Gly Tyr Gln Pro Tyr Arg Val Val Val Leu Ser Phe Glu Leu
515 520 525
Leu His Ala Pro Ala Thr Val Cys Gly Pro Lys Lys Ser Thr Asn Leu
530 535 540
Val Lys Asn Lys Cys Val Asn Phe Asn Phe Asn Gly Leu Thr Gly Thr
545 550 555 560
Gly Val Leu Thr Glu Ser Asn Lys Lys Phe Leu Pro Phe Gln Gln Phe
565 570 575
Gly Arg Asp Ile Ala Asp Thr Thr Asp Ala Val Arg Asp Pro Gln Thr
580 585 590
Leu Glu Ile Leu Asp Ile Thr Pro Cys Ser Phe Gly Gly Val Ser Val
595 600 605
Ile Thr Pro Gly Thr Asn Thr Ser Asn Gln Val Ala Val Leu Tyr Gln
610 615 620
Asp Val Asn Cys Thr Glu Val Pro Val Ala Ile His Ala Asp Gln Leu
625 630 635 640
Thr Pro Thr Trp Arg Val Tyr Ser Thr Gly Ser Asn Val Phe Gln Thr
645 650 655
Arg Ala Gly Cys Leu Ile Gly Ala Glu His Val Asn Asn Ser Tyr Glu
660 665 670
Cys Asp Ile Pro Ile Gly Ala Gly Ile Cys Ala Ser Tyr Gln Thr Gln
675 680 685
Thr Asn Ser Pro Arg Arg Ala Arg Ser Val Ala Ser Gln Ser Ile Ile
690 695 700
Ala Tyr Thr Met Ser Leu Gly Ala Glu Asn Ser Val Ala Tyr Ser Asn
705 710 715 720
Asn Ser Ile Ala Ile Pro Thr Asn Phe Thr Ile Ser Val Thr Thr Glu
725 730 735
Ile Leu Pro Val Ser Met Thr Lys Thr Ser Val Asp Cys Thr Met Tyr
740 745 750
Ile Cys Gly Asp Ser Thr Glu Cys Ser Asn Leu Leu Leu Gln Tyr Gly
755 760 765
Ser Phe Cys Thr Gln Leu Asn Arg Ala Leu Thr Gly Ile Ala Val Glu
770 775 780
Gln Asp Ala Asn Thr Gly Glu Val Phe Ala Gln Val Lys Gln Ile Tyr
785 790 795 800
Lys Thr Pro Pro Ile Lys Asp Phe Gly Gly Phe Asn Phe Ser Gln Ile
805 810 815
Leu Pro Asp Pro Ser Lys Pro Ser Lys Arg Ser Phe Ile Glu Asp Leu
820 825 830
Leu Phe Asn Lys Val Thr Leu Ala Asp Ala Gly Phe Ile Lys Gln Tyr
835 840 845
Gly Asp Cys Leu Gly Asp Ile Ala Ala Arg Asp Leu Ile Cys Ala Gln
850 855 860
Lys Phe Asn Gly Leu Thr Val Leu Pro Pro Leu Leu Thr Asp Glu Met
865 870 875 880
Ile Ala Gln Tyr Thr Ser Ala Leu Leu Ala Gly Thr Ile Thr Ser Gly
885 890 895
Trp Thr Phe Gly Ala Gly Ala Ala Leu Gln Ile Pro Phe Ala Met Gln
900 905 910
Met Ala Tyr Arg Phe Asn Gly Ile Gly Val Thr Gln Asn Val Leu Tyr
915 920 925
Glu Asn Gln Lys Leu Ile Ala Asn Gln Phe Asn Ser Ala Ile Gly Lys
930 935 940
Ile Gln Asp Ser Leu Ser Ser Thr Ala Ser Ala Leu Gly Lys Leu Gln
945 950 955 960
Asp Val Val Asn Gln Asn Ala Gln Ala Leu Asn Thr Leu Val Lys Gln
965 970 975
Leu Ser Ser Asn Phe Gly Ala Ile Ser Ser Val Leu Asn Asp Ile Leu
980 985 990
Ser Arg Leu Asp Pro Pro Glu Ala Glu Val Gln Ile Asp Arg Leu Ile

```
        995                 1000                1005
Thr Gly Arg Leu Gln Ser Leu Gln Thr Tyr Val Thr Gln Gln Leu Ile
    1010                1015                1020
Arg Ala Ala Glu Ile Arg Ala Ser Ala Asn Leu Ala Ala Thr Lys Met
1025                1030                1035                1040
Ser Glu Cys Val Leu Gly Gln Ser Lys Arg Val Asp Phe Cys Gly Lys
                1045                1050                1055
Gly Tyr His Leu Met Ser Phe Pro Gln Ser Ala Pro His Gly Val Val
            1060                1065                1070
Phe Leu His Val Thr Tyr Val Pro Ala Gln Glu Lys Asn Phe Thr Thr
        1075                1080                1085
Ala Pro Ala Ile Cys His Asp Gly Lys Ala His Phe Pro Arg Glu Gly
    1090                1095                1100
Val Phe Val Ser Asn Gly Thr His Trp Phe Val Thr Gln Arg Asn Phe
1105                1110                1115                1120
Tyr Glu Pro Gln Ile Ile Thr Thr Asp Asn Thr Phe Val Ser Gly Asn
                1125                1130                1135
Cys Asp Val Val Ile Gly Ile Val Asn Asn Thr Val Tyr Asp Pro Leu
            1140                1145                1150
Gln Pro Glu Leu Asp Ser Phe Lys Glu Glu Leu Asp Lys Tyr Phe Lys
        1155                1160                1165
Asn His Thr Ser Pro Asp Val Asp Leu Gly Asp Ile Ser Gly Ile Asn
    1170                1175                1180
Ala Ser Val Val Asn Ile Gln Lys Glu Ile Asp Arg Leu Asn Glu Val
1185                1190                1195                1200
Ala Lys Asn Leu Asn Glu Ser Leu Ile Asp Leu Gln Glu Leu Gly Lys
                1205                1210                1215
Tyr Glu Gln Tyr Ile Lys Trp Pro Trp Tyr Ile Trp Leu Gly Phe Ile
            1220                1225                1230
Ala Gly Leu Ile Ala Ile Val Met Val Thr Ile Met Leu
        1235                1240                1245
```

<210> 7
<211> 138
<212> DNA
<213> Artificial Sequence

<220>
<223> CilPP

<400> 7

```
gcgccccact tccctgaaat ctccttcccc cctaaacccg attctgatta tcaggccttg      60

ctaccatccg cgccagagat ctactctcac ctctccccca ccaaacccga ttacatcaac     120

cttcgaccgg cgccctaa                                                   138
```

<210> 8
<211> 45
<212> PRT
<213> Artificial Sequence

<220>
<223> CilPP encoded by SEQ ID NO: 7

<400> 8

```
Ala Pro His Phe Pro Glu Ile Ser Phe Pro Pro Lys Pro Asp Ser Asp
1               5                   10                  15
Tyr Gln Ala Leu Leu Pro Ser Ala Pro Glu Ile Tyr Ser His Leu Ser
            20                  25                  30
```

```
Pro Thr Lys Pro Asp Tyr Ile Asn Leu Arg Pro Ala Pro
        35                  40                  45

<210> 9
<211> 9
<212> DNA
<213> Artificial Sequence


<220>
<223> Linker

<400> 9
tctagaggc                                                          9


<210> 10
<211> 3
<212> PRT
<213> Artificial Sequence


<220>
<223> Linker encoded by SEQ ID NO: 9

<400> 10
Ser Arg Gly
1

<210> 11
<211> 3657
<212> DNA
<213> Artificial Sequence


<220>
<223> Modified S gene

<400> 11
atggtgctgc tgctgatcct gtctgtgctg ctcctgaaag aagatgtgcg gggctctgcc        60

cagagcaccg gtcaatgtgt gaacctgacc accagaacac agctgcctcc agcctacacc       120

aacagcttca ccagaggcgt gtactacccc gacaaggtgt tcagatccag cgtgctgcac       180

tctacccagg acctgttcct gcctttcttc agcaacgtga cctggttcca cgccatccac       240

gtgtccggca ccaatggcac caagagattc gacaaccccg tgctgccctt caacgacggg       300

gtgtactttg ccagcaccga gaagtccaac atcatcagag gctggatctt cggcaccaca       360

ctggacagca agacccagag cctgctgatc gtgaacaacg ccaccaacgt ggtcatcaaa       420

gtgtgcgagt tccagttctg caacgacccc ttcctgggcg tctactacca caagaacaac       480

aagagctgga tggaaagcga gttccgggtg tacagcagcg ccaacaactg caccttcgag       540

tacgtgtccc agcctttcct gatggacctg gaaggcaagc agggcaactt caagaacctg       600

cgcgagttcg tgttcaagaa catcgacggc tacttcaaga tctacagcaa gcacacccct       660

atcaacctcg tgcgggatct gcctcagggc ttctctgctc tggaacccct ggtggatctg       720
```

cccatcggca tcaacatcac ccggtttcag acactgctgg ccctgcacag aagctacctg 780
acacctggcg atagcagctc tggatggaca gctggcgccg ctgcctacta tgtgggatac 840
ctgcagcctc ggaccttcct gctgaagtac aacgagaacg gcaccatcac cgacgccgtg 900
gattgtgctc tggatcctct gagcgagaca aagtgcaccc tgaagtcctt caccgtggaa 960
aagggcatct accagaccag caacttccgg gtgcagccca ccgaatccat cgtgcggttc 1020
cccaatatca ccaatctgtg ccccttcggc gaggtgttca atgccaccag attcgcctct 1080
gtgtacgcct ggaaccggaa gcggatcagc aattgcgtgg ccgactactc cgtgctgtac 1140
aactccgcca gcttcagcac cttcaagtgc tacggcgtgt cccctaccaa gctgaacgac 1200
ctgtgcttca caaacgtgta cgccgacagc ttcgtgatcc ggggagatga agtgcggcag 1260
attgcccctg gacagacagg caagatcgcc gactacaact acaagctgcc cgacgacttc 1320
accggctgtg tgattgcctg gaacagcaac aacctggact ccaaagtcgg cggcaactac 1380
aattacctgt accggctgtt ccggaagtcc aatctgaagc ccttcgagcg ggacatctcc 1440
accgagatct atcaggccgg cagcacccct tgtaacggcg tggaaggctt caactgctac 1500
ttcccactgc agtcctacgg ctttcagccc acaaatggcg tgggctacca gccttacaga 1560
gtggtggtgc tgagcttcga gctgctgcat gctcctgcca cagtgtgcgg ccctaagaaa 1620
agcaccaatc tcgtgaagaa caaatgcgtg aacttcaact tcaacggcct gaccggcacc 1680
ggcgtgctga cagagagcaa caagaagttc ctgccattcc agcagttcgg ccgggatatc 1740
gccgatacca cagatgccgt cagagatccc cagacactgg aaatcctgga catcacccct 1800
tgcagcttcg gcggagtgtc tgtgatcacc cctggcacca acaccagcaa tcaggtggca 1860
gtgctgtacc aggacgtgaa ctgtaccgaa gtgcccgtgg ccattcacgc cgatcagctg 1920
acacctacat ggcgggtgta ctccaccggc agcaatgtgt ttcagaccag agccggctgt 1980
ctgatcggag ccgagcacgt gaacaatagc tacgagtgcg acatccccat cggcgctggc 2040
atctgcgcct cttaccagac acagacaaac agccccagac gggccagatc tgtggccagc 2100
cagagcatca ttgcctacac aatgtctctg ggcgccgaga acagcgtggc ctactccaac 2160
aactctatcg ctatccccac caacttcacc atcagcgtga ccacagagat cctgcctgtg 2220
tccatgacca agaccagcgt ggactgcacc atgtacatct gcggcgattc caccgagtgc 2280
tccaacctgc tgctgcagta cggcagcttc tgcacccagc tgaatagagc cctgacaggg 2340
atcgccgtgg aacaggatgc caataccggc gaagtgttcg cccaagtgaa gcagatctac 2400
aagacccctc ctatcaagga cttcggcggc ttcaatttca gccagattct gcccgatcct 2460
agcaagccca gcaagcggag cttcatcgag gacctgctgt tcaacaaagt gacactggcc 2520
gacgccggct tcatcaagca gtatggcgat tgtctgggcg acattgccgc cagggatctg 2580
atttgcgccc agaagtttaa cggactgaca gtgctgcctc ctctgctgac cgatgagatg 2640

atcgcccagt acacatctgc cctgctggcc ggcacaatca caagcggctg gacatttgga 2700

gctggcgctg ccctgcagat cccctttgct atgcagatgg cctacagatt caacggcatc 2760

ggagtgaccc agaatgtgct gtacgagaac cagaagctga tcgccaacca gttcaacagc 2820

gccatcggca agatccagga cagcctgtct agcacagcca gcgctctggg aaagctgcag 2880

gacgtggtca accagaatgc ccaggcactg aacaccctgg tcaagcagct gagcagcaat 2940

ttcggcgcca tcagctctgt gctgaacgat atcctgagca gactggaccc tcctgaggcc 3000

gaggtgcaga tcgacagact gatcaccgga aggctgcagt ccctgcagac ctacgttacc 3060

cagcagctga tcagagccgc cgagattaga gcctctgcca atctggccgc caccaagatg 3120

tctgagtgtg tgctgggcca gagcaagaga gtggactttt gcggcaaggg ctaccacctg 3180

atgagcttcc ctcagtctgc ccctcacggc gtggtgtttc tgcacgtgac atacgtgccc 3240

gctcaagaga agaatttcac caccgctcca gccatctgcc acgacggcaa agcccacttt 3300

cctagagaag gcgtgttcgt gtccaacggc acccattggt tcgtgaccca gcggaacttc 3360

tacgagcccc agatcatcac caccgacaac accttcgtgt ctggcaactg cgacgtcgtg 3420

atcggcattg tgaacaatac cgtgtacgac cctctgcagc ccgagctgga cagcttcaaa 3480

gaggaactgg ataagtactt taagaaccac acaagccccg acgtggacct gggcgatatc 3540

agcggaatca atgccagcgt cgtgaacatc cagaaagaga tcgaccggct gaacgaggtg 3600

gccaagaatc tgaacgagag cctgatcgac ctgcaggaac tggggaagta cgagcag 3657

<210> 12
<211> 1219
<212> PRT
<213> Artificial Sequence

<220>
<223> Modified Spike protein encoded by SEQ ID NO: 11

<400> 12
Met Val Leu Leu Leu Ile Leu Ser Val Leu Leu Leu Lys Glu Asp Val
1 5 10 15
Arg Gly Ser Ala Gln Ser Thr Gly Gln Cys Val Asn Leu Thr Thr Arg
20 25 30
Thr Gln Leu Pro Pro Ala Tyr Thr Asn Ser Phe Thr Arg Gly Val Tyr
35 40 45
Tyr Pro Asp Lys Val Phe Arg Ser Ser Val Leu His Ser Thr Gln Asp
50 55 60
Leu Phe Leu Pro Phe Phe Ser Asn Val Thr Trp Phe His Ala Ile His
65 70 75 80
Val Ser Gly Thr Asn Gly Thr Lys Arg Phe Asp Asn Pro Val Leu Pro
85 90 95
Phe Asn Asp Gly Val Tyr Phe Ala Ser Thr Glu Lys Ser Asn Ile Ile
100 105 110
Arg Gly Trp Ile Phe Gly Thr Thr Leu Asp Ser Lys Thr Gln Ser Leu
115 120 125
Leu Ile Val Asn Asn Ala Thr Asn Val Val Ile Lys Val Cys Glu Phe

130 135 140
Gln Phe Cys Asn Asp Pro Phe Leu Gly Val Tyr Tyr His Lys Asn Asn
145 150 155 160
Lys Ser Trp Met Glu Ser Glu Phe Arg Val Tyr Ser Ser Ala Asn Asn
165 170 175
Cys Thr Phe Glu Tyr Val Ser Gln Pro Phe Leu Met Asp Leu Glu Gly
180 185 190
Lys Gln Gly Asn Phe Lys Asn Leu Arg Glu Phe Val Phe Lys Asn Ile
195 200 205
Asp Gly Tyr Phe Lys Ile Tyr Ser Lys His Thr Pro Ile Asn Leu Val
210 215 220
Arg Asp Leu Pro Gln Gly Phe Ser Ala Leu Glu Pro Leu Val Asp Leu
225 230 235 240
Pro Ile Gly Ile Asn Ile Thr Arg Phe Gln Thr Leu Leu Ala Leu His
245 250 255
Arg Ser Tyr Leu Thr Pro Gly Asp Ser Ser Ser Gly Trp Thr Ala Gly
260 265 270
Ala Ala Ala Tyr Tyr Val Gly Tyr Leu Gln Pro Arg Thr Phe Leu Leu
275 280 285
Lys Tyr Asn Glu Asn Gly Thr Ile Thr Asp Ala Val Asp Cys Ala Leu
290 295 300
Asp Pro Leu Ser Glu Thr Lys Cys Thr Leu Lys Ser Phe Thr Val Glu
305 310 315 320
Lys Gly Ile Tyr Gln Thr Ser Asn Phe Arg Val Gln Pro Thr Glu Ser
325 330 335
Ile Val Arg Phe Pro Asn Ile Thr Asn Leu Cys Pro Phe Gly Glu Val
340 345 350
Phe Asn Ala Thr Arg Phe Ala Ser Val Tyr Ala Trp Asn Arg Lys Arg
355 360 365
Ile Ser Asn Cys Val Ala Asp Tyr Ser Val Leu Tyr Asn Ser Ala Ser
370 375 380
Phe Ser Thr Phe Lys Cys Tyr Gly Val Ser Pro Thr Lys Leu Asn Asp
385 390 395 400
Leu Cys Phe Thr Asn Val Tyr Ala Asp Ser Phe Val Ile Arg Gly Asp
405 410 415
Glu Val Arg Gln Ile Ala Pro Gly Gln Thr Gly Lys Ile Ala Asp Tyr
420 425 430
Asn Tyr Lys Leu Pro Asp Asp Phe Thr Gly Cys Val Ile Ala Trp Asn
435 440 445
Ser Asn Asn Leu Asp Ser Lys Val Gly Gly Asn Tyr Asn Tyr Leu Tyr
450 455 460
Arg Leu Phe Arg Lys Ser Asn Leu Lys Pro Phe Glu Arg Asp Ile Ser
465 470 475 480
Thr Glu Ile Tyr Gln Ala Gly Ser Thr Pro Cys Asn Gly Val Glu Gly
485 490 495
Phe Asn Cys Tyr Phe Pro Leu Gln Ser Tyr Gly Phe Gln Pro Thr Asn
500 505 510
Gly Val Gly Tyr Gln Pro Tyr Arg Val Val Val Leu Ser Phe Glu Leu
515 520 525
Leu His Ala Pro Ala Thr Val Cys Gly Pro Lys Lys Ser Thr Asn Leu
530 535 540
Val Lys Asn Lys Cys Val Asn Phe Asn Phe Asn Gly Leu Thr Gly Thr
545 550 555 560
Gly Val Leu Thr Glu Ser Asn Lys Lys Phe Leu Pro Phe Gln Gln Phe
565 570 575
Gly Arg Asp Ile Ala Asp Thr Thr Asp Ala Val Arg Asp Pro Gln Thr
580 585 590
Leu Glu Ile Leu Asp Ile Thr Pro Cys Ser Phe Gly Gly Val Ser Val
595 600 605
Ile Thr Pro Gly Thr Asn Thr Ser Asn Gln Val Ala Val Leu Tyr Gln
610 615 620
Asp Val Asn Cys Thr Glu Val Pro Val Ala Ile His Ala Asp Gln Leu
625 630 635 640

```
Thr Pro Thr Trp Arg Val Tyr Ser Thr Gly Ser Asn Val Phe Gln Thr
                645                 650                 655
Arg Ala Gly Cys Leu Ile Gly Ala Glu His Val Asn Asn Ser Tyr Glu
            660                 665                 670
Cys Asp Ile Pro Ile Gly Ala Gly Ile Cys Ala Ser Tyr Gln Thr Gln
        675                 680                 685
Thr Asn Ser Pro Arg Arg Ala Arg Ser Val Ala Ser Gln Ser Ile Ile
    690                 695                 700
Ala Tyr Thr Met Ser Leu Gly Ala Glu Asn Ser Val Ala Tyr Ser Asn
705                 710                 715                 720
Asn Ser Ile Ala Ile Pro Thr Asn Phe Thr Ile Ser Val Thr Thr Glu
                725                 730                 735
Ile Leu Pro Val Ser Met Thr Lys Thr Ser Val Asp Cys Thr Met Tyr
            740                 745                 750
Ile Cys Gly Asp Ser Thr Glu Cys Ser Asn Leu Leu Leu Gln Tyr Gly
        755                 760                 765
Ser Phe Cys Thr Gln Leu Asn Arg Ala Leu Thr Gly Ile Ala Val Glu
    770                 775                 780
Gln Asp Ala Asn Thr Gly Glu Val Phe Ala Gln Val Lys Gln Ile Tyr
785                 790                 795                 800
Lys Thr Pro Pro Ile Lys Asp Phe Gly Gly Phe Asn Phe Ser Gln Ile
                805                 810                 815
Leu Pro Asp Pro Ser Lys Pro Ser Lys Arg Ser Phe Ile Glu Asp Leu
            820                 825                 830
Leu Phe Asn Lys Val Thr Leu Ala Asp Ala Gly Phe Ile Lys Gln Tyr
        835                 840                 845
Gly Asp Cys Leu Gly Asp Ile Ala Ala Arg Asp Leu Ile Cys Ala Gln
    850                 855                 860
Lys Phe Asn Gly Leu Thr Val Leu Pro Pro Leu Leu Thr Asp Glu Met
865                 870                 875                 880
Ile Ala Gln Tyr Thr Ser Ala Leu Leu Ala Gly Thr Ile Thr Ser Gly
                885                 890                 895
Trp Thr Phe Gly Ala Gly Ala Ala Leu Gln Ile Pro Phe Ala Met Gln
            900                 905                 910
Met Ala Tyr Arg Phe Asn Gly Ile Gly Val Thr Gln Asn Val Leu Tyr
        915                 920                 925
Glu Asn Gln Lys Leu Ile Ala Asn Gln Phe Asn Ser Ala Ile Gly Lys
    930                 935                 940
Ile Gln Asp Ser Leu Ser Ser Thr Ala Ser Ala Leu Gly Lys Leu Gln
945                 950                 955                 960
Asp Val Val Asn Gln Asn Ala Gln Ala Leu Asn Thr Leu Val Lys Gln
                965                 970                 975
Leu Ser Ser Asn Phe Gly Ala Ile Ser Ser Val Leu Asn Asp Ile Leu
            980                 985                 990
Ser Arg Leu Asp Pro Pro Glu Ala Glu Val Gln Ile Asp Arg Leu Ile
        995                 1000                1005
Thr Gly Arg Leu Gln Ser Leu Gln Thr Tyr Val Thr Gln Gln Leu Ile
    1010                1015                1020
Arg Ala Ala Glu Ile Arg Ala Ser Ala Asn Leu Ala Ala Thr Lys Met
1025                1030                1035                1040
Ser Glu Cys Val Leu Gly Gln Ser Lys Arg Val Asp Phe Cys Gly Lys
                1045                1050                1055
Gly Tyr His Leu Met Ser Phe Pro Gln Ser Ala Pro His Gly Val Val
            1060                1065                1070
Phe Leu His Val Thr Tyr Val Pro Ala Gln Glu Lys Asn Phe Thr Thr
        1075                1080                1085
Ala Pro Ala Ile Cys His Asp Gly Lys Ala His Phe Pro Arg Glu Gly
    1090                1095                1100
Val Phe Val Ser Asn Gly Thr His Trp Phe Val Thr Gln Arg Asn Phe
1105                1110                1115                1120
Tyr Glu Pro Gln Ile Ile Thr Thr Asp Asn Thr Phe Val Ser Gly Asn
                1125                1130                1135
Cys Asp Val Val Ile Gly Ile Val Asn Asn Thr Val Tyr Asp Pro Leu
```

```
            1140                1145                1150
Gln Pro Glu Leu Asp Ser Phe Lys Glu Glu Leu Asp Lys Tyr Phe Lys
        1155                1160                1165
Asn His Thr Ser Pro Asp Val Asp Leu Gly Asp Ile Ser Gly Ile Asn
    1170                1175                1180
Ala Ser Val Val Asn Ile Gln Lys Glu Ile Asp Arg Leu Asn Glu Val
1185                1190                1195                1200
Ala Lys Asn Leu Asn Glu Ser Leu Ile Asp Leu Gln Glu Leu Gly Lys
                1205                1210                1215
Tyr Glu Gln


<210> 13
<211> 153
<212> DNA
<213> Artificial Sequence


<220>
<223> Trimerization foldon

<400> 13
agcggcagag agaacctgta cttccaaggc ggaggcggcg gaagcggcta tattcctgaa      60

gctcctagag atggccaggc ctacgtgcgg aaagatggcg aatgggtcct gctgagcacc     120

tttctgggac accaccacca tcaccattag taa                                  153


<210> 14
<211> 49
<212> PRT
<213> Artificial Sequence


<220>
<223> Trimerization foldon encoded by SEQ ID NO: 13

<400> 14
Ser Gly Arg Glu Asn Leu Tyr Phe Gln Gly Gly Gly Gly Gly Ser Gly
1               5                   10                  15
Tyr Ile Pro Glu Ala Pro Arg Asp Gly Gln Ala Tyr Val Arg Lys Asp
            20                  25                  30
Gly Glu Trp Val Leu Leu Ser Thr Phe Leu Gly His His His His His
        35                  40                  45
His


<210> 15
<211> 2418
<212> DNA
<213> Homo sapiens


<220>
<223> hACE2

<300>
<308> NCBI Reference Sequence NM_021804.2
<309> Version 2 of June 11, 2019

<400> 15
atgtcaagct cttcctggct ccttctcagc cttgttgctg taactgctgc tcagtccacc      60
```

```
attgaggaac aggccaagac atttttggac aagtttaacc acgaagccga agacctgttc     120
tatcaaagtt cacttgcttc ttggaattat aacaccaata ttactgaaga gaatgtccaa     180
aacatgaata atgctgggga caaatggtct gcctttttaa aggaacagtc cacacttgcc     240
caaatgtatc cactacaaga aattcagaat ctcacagtca agcttcagct gcaggctctt     300
cagcaaaatg ggtcttcagt gctctcagaa gacaagagca aacggttgaa cacaattcta     360
aatacaatga gcaccatcta cagtactgga aaagtttgta acccagataa tccacaagaa     420
tgcttattac ttgaaccagg tttgaatgaa ataatggcaa acagtttaga ctacaatgag     480
aggctctggg cttgggaaag ctggagatct gaggtcggca agcagctgag gccattatat     540
gaagagtatg tggtcttgaa aaatgagatg gcaagagcaa atcattatga ggactatggg     600
gattattgga gaggagacta tgaagtaaat ggggtagatg gctatgacta cagccgcggc     660
cagttgattg aagatgtgga acataccttt gaagagatta aaccattata tgaacatctt     720
catgcctatg tgagggcaaa gttgatgaat gcctatcctt cctatatcag tccaattgga     780
tgcctccctg ctcatttgct tggtgatatg tggggtagat tttggacaaa tctgtactct     840
ttgacagttc cctttggaca gaaaccaaac atagatgtta ctgatgcaat ggtggaccag     900
gcctgggatg cacagagaat attcaaggag gccgagaagt tctttgtatc tgttggtctt     960
cctaatatga ctcaaggatt ctgggaaaat tccatgctaa cggacccagg aaatgttcag    1020
aaagcagtct gccatcccac agcttgggac ctggggaagg gcgacttcag gatccttatg    1080
tgcacaaagg tgacaatgga cgacttcctg acagctcatc atgagatggg gcatatccag    1140
tatgatatgg catatgctgc acaacctttt ctgctaagaa atggagctaa tgaaggattc    1200
catgaagctg ttggggaaat catgtcactt tctgcagcca cacctaagca tttaaaatcc    1260
attggtcttc tgtcacccga ttttcaagaa gacaatgaaa cagaaataaa cttcctgctc    1320
aaacaagcac tcacgattgt tgggactctg ccatttactt acatgttaga gaagtggagg    1380
tggatggtct ttaaagggga aattcccaaa gaccagtgga tgaaaaagtg gtgggagatg    1440
aagcgagaga tagttggggt ggtggaacct gtgccccatg atgaaacata ctgtgacccc    1500
gcatctctgt tccatgtttc taatgattac tcattcattc gatattacac aaggaccctt    1560
taccaattcc agtttcaaga agcactttgt caagcagcta aacatgaagg ccctctgcac    1620
aaatgtgaca tctcaaactc tacagaagct ggacagaaac tgttcaatat gctgaggctt    1680
ggaaaatcag aaccctggac cctagcattg gaaaatgttg taggagcaaa gaacatgaat    1740
gtaaggccac tgctcaacta ctttgagccc ttatttacct ggctgaaaga ccagaacaag    1800
aattcttttg tgggatggag taccgactgg agtccatatg cagaccaaag catcaaagtg    1860
aggataagcc taaaatcagc tcttggagat aaagcatatg aatggaacga caatgaaatg    1920
```

tacctgttcc gatcatctgt tgcatatgct atgaggcagt actttttaaa agtaaaaaat 1980

cagatgattc tttttgggga ggaggatgtg cgagtggcta atttgaaacc aagaatctcc 2040

tttaatttct ttgtcactgc acctaaaaat gtgtctgata tcattcctag aactgaagtt 2100

gaaaaggcca tcaggatgtc ccggagccgt atcaatgatg ctttccgtct gaatgacaac 2160

agcctagagt ttctggggat acagccaaca cttggacctc ctaaccagcc ccctgtttcc 2220

atatggctga ttgtttttgg agttgtgatg ggagtgatag tggttggcat tgtcatcctg 2280

atcttcactg ggatcagaga tcggaagaag aaaaataaag caagaagtgg agaaaatcct 2340

tatgcctcca tcgatattag caaaggagaa aataatccag gattccaaaa cactgatgat 2400

gttcagacct ccttttag 2418

<210> 16
<211> 1479
<212> DNA
<213> Homo sapiens

<220>
<223> hTMPRSS2

<300>
<308> NCBI Reference Sequence NM_005656.4
<309> Version 4 of September 20, 2020

<400> 16
atggctttga actcagggtc accaccagct attggacctt actatgaaaa ccatggatac 60

caaccggaaa acccctatcc cgcacagccc actgtggtcc ccactgtcta cgaggtgcat 120

ccggctcagt actacccgtc ccccgtgccc cagtacgccc cgagggtcct gacgcaggct 180

tccaaccccg tcgtctgcac gcagcccaaa tccccatccg ggacagtgtg cacctcaaag 240

actaagaaag cactgtgcat caccttgacc ctggggacct tcctcgtggg agctgcgctg 300

gccgctggcc tactctggaa gttcatgggc agcaagtgct ccaactctgg gatagagtgc 360

gactcctcag gtacctgcat caacccctct aactggtgtg atggcgtgtc acactgcccc 420

ggcggggagg acgagaatcg gtgtgttcgc ctctacggac caaacttcat ccttcaggtg 480

tactcatctc agaggaagtc ctggcaccct gtgtgccaag acgactggaa cgagaactac 540

gggcgggcgg cctgcaggga catgggctat aagaataatt tttactctag ccaaggaata 600

gtggatgaca gcggatccac cagctttatg aaactgaaca caagtgccgg caatgtcgat 660

atctataaaa aactgtacca cagtgatgcc tgttcttcaa aagcagtggt ttctttacgc 720

tgtatagcct gcggggtcaa cttgaactca agccgccaga gcaggattgt gggcggcgag 780

agcgcgctcc cgggggcctg gccctggcag gtcagcctgc acgtccagaa cgtccacgtg 840

tgcggaggct ccatcatcac ccccgagtgg atcgtgacag ccgcccactg cgtggaaaaa 900

cctcttaaca atccatggca ttggacggca tttgcgggga ttttgagaca atctttcatg 960

ttctatggag ccggatacca agtagaaaaa gtgatttctc atccaaatta tgactccaag 1020

accaagaaca atgacattgc gctgatgaag ctgcagaagc ctctgacttt caacgaccta 1080

gtgaaaccag tgtgtctgcc caacccaggc atgatgctgc agccagaaca gctctgctgg 1140

atttccgggt gggggccac cgaggagaaa gggaagacct cagaagtgct gaacgctgcc 1200

aaggtgcttc tcattgagac acagagatgc aacagcagat atgtctatga caacctgatc 1260

acaccagcca tgatctgtgc cggcttcctg caggggaacg tcgattcttg ccagggtgac 1320

agtggagggc ctctggtcac ttcgaagaac aatatctggt ggctgatagg ggatacaagc 1380

tggggttctg gctgtgccaa agcttacaga ccaggagtgt acgggaatgt gatggtattc 1440

acggactgga tttatcgaca aatgagggca gacggctaa 1479

<210> 17
<211> 4
<212> PRT
<213> Artificial Sequence

<220>
<223> Pilot peptide, YxxL motif

<220>
<221> SITE
<222> 2
<223> Xaa represents any amino acid residue

<220>
<221> SITE
<222> 3
<223> Xaa represents any amino acid residue

<400> 17
Tyr Xaa Xaa Leu
1

<210> 18
<211> 4
<212> PRT
<213> Artificial Sequence

<220>
<223> Pilot peptide, YxxL motif

<400> 18
Tyr Ile Asn Leu
1

<210> 19
<211> 4
<212> PRT
<213> Artificial Sequence

<220>
<223> Pilot peptide, YxxL motif

<400> 19
Tyr Ser His Leu
1

<210> 20
<211> 5
<212> PRT
<213> Artificial Sequence

<220>
<223> Pilot peptide, DYxxL motif

<220>
<221> SITE
<222> 3
<223> Xaa represents any amino acid residue

<220>
<221> SITE
<222> 4
<223> Xaa represents any amino acid residue

<400> 20
Asp Tyr Xaa Xaa Leu
1 5

<210> 21
<211> 5
<212> PRT
<213> Artificial Sequence

<220>
<223> Pilot peptide, DYxxL motif

<400> 21
Asp Tyr Ile Asn Leu
1 5

<210> 22
<211> 4
<212> PRT
<213> Artificial Sequence

<220>
<223> Pilot peptide, YxxF motif

<220>
<221> SITE
<222> 2
<223> Xaa represents any amino acid residue

<220>
<221> SITE
<222> 3
<223> Xaa represents any amino acid residue

<400> 22

Tyr Xaa Xaa Phe
1

<210> 23
<211> 5
<212> PRT
<213> Artificial Sequence

<220>
<223> Pilot peptide, DYxxF motif

<220>
<221> SITE
<222> 3
<223> Xaa represents any amino acid residue

<220>
<221> SITE
<222> 4
<223> Xaa represents any amino acid residue

<400> 23
Asp Tyr Xaa Xaa Phe
1 5

<210> 24
<211> 4
<212> PRT
<213> Artificial Sequence

<220>
<223> Pilot peptide, PxxP motif

<220>
<221> SITE
<222> 2
<223> Xaa represents any amino acid residue

<220>
<221> SITE
<222> 3
<223> Xaa represents any amino acid residue

<400> 24
Pro Xaa Xaa Pro
1

<210> 25
<211> 4
<212> PRT
<213> Artificial Sequence

<220>
<223> Pilot peptide, PxxP motif

<400> 25
Pro Ser Ala Pro
1

<210> 26

<211> 4
<212> PRT
<213> Artificial Sequence

<220>
<223> Pilot peptide, PxxP motif

<400> 26
Pro Thr Ala Pro
1

<210> 27
<211> 40
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any amino acid residue

<220>
<221> SITE
<222> 6
<223> Xaa represents any amino acid residue

<220>
<221> SITE
<222> 7
<223> Xaa represents any amino acid residue

<220>
<221> SITE
<222> 8
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE

<222> 13
<223> Xaa represents any amino acid residue

<220>
<221> SITE
<222> 15
<223> Xaa represents any amino acid residue

<220>
<221> SITE
<222> 17
<223> Xaa represents any amino acid residue

<220>
<221> SITE
<222> 18
<223> Xaa represents any amino acid residue

<220>
<221> SITE
<222> 20
<223> Xaa represents any amino acid residue

<220>
<221> SITE
<222> 22
<223> Xaa represents any amino acid residue

<220>
<221> SITE
<222> 23
<223> Xaa represents any amino acid residue

<220>
<221> SITE
<222> 26
<223> Xaa represents any amino acid residue

<220>
<221> SITE
<222> 28
<223> Xaa represents any amino acid residue

<220>
<221> SITE
<222> 29
<223> Xaa represents any amino acid residue

<220>
<221> SITE
<222> 31
<223> Xaa represents any amino acid residue

<220>
<221> SITE
<222> 33
<223> Xaa represents any amino acid residue

<220>
<221> SITE
<222> 34
<223> Xaa represents any amino acid residue

```
<220>
<221> SITE
<222> 38
<223> Xaa represents any amino acid residue

<220>
<221> SITE
<222> 39
<223> Xaa represents any amino acid residue

<400> 27
Pro Xaa Xaa Pro Xaa Xaa Xaa Xaa Pro Xaa Xaa Pro Xaa Ser Xaa Tyr
1               5                   10                  15
Xaa Xaa Leu Xaa Pro Xaa Xaa Pro Glu Xaa Tyr Xaa Xaa Leu Xaa Pro
            20                  25                  30
Xaa Xaa Pro Asp Tyr Xaa Xaa Leu
        35                  40

<210> 28
<211> 37
<212> PRT
<213> Artificial Sequence


<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any one or several amino acid residue(s)

<220>
<221> SITE
<222> 7
<223> Xaa represents any amino acid residue

<220>
<221> SITE
<222> 8
<223> Xaa represents any amino acid residue

<220>
<221> SITE
<222> 10
<223> Xaa represents any one or several amino acid residue(s)

<220>
<221> SITE
<222> 12
<223> Xaa represents any amino acid residue

<220>
```

<221> SITE
<222> 14
<223> Xaa represents any amino acid residue

<220>
<221> SITE
<222> 15
<223> Xaa represents any amino acid residue

<220>
<221> SITE
<222> 17
<223> Xaa represents any one or several amino acid residue(s)

<220>
<221> SITE
<222> 19
<223> Xaa represents any amino acid residue

<220>
<221> SITE
<222> 20
<223> Xaa represents any amino acid residue

<220>
<221> SITE
<222> 23
<223> Xaa represents any one or several amino acid residue(s)

<220>
<221> SITE
<222> 25
<223> Xaa represents any amino acid residue

<220>
<221> SITE
<222> 26
<223> Xaa represents any amino acid residue

<220>
<221> SITE
<222> 28
<223> Xaa represents any one or several amino acid residue(s)

<220>
<221> SITE
<222> 30
<223> Xaa represents any amino acid residue

<220>
<221> SITE
<222> 31
<223> Xaa represents any amino acid residue

<220>
<221> SITE
<222> 35
<223> Xaa represents any amino acid residue

<220>
<221> SITE
<222> 36
<223> Xaa represents any amino acid residue

<400> 28
Pro Xaa Xaa Pro Xaa Pro Xaa Xaa Pro Xaa Ser Xaa Tyr Xaa Xaa Leu
1               5                   10                  15
Xaa Pro Xaa Xaa Pro Glu Xaa Tyr Xaa Xaa Leu Xaa Pro Xaa Xaa Pro
            20                  25                  30
Asp Tyr Xaa Xaa Leu
        35

<210> 29
<211> 44
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any amino acid residue

<220>
<221> SITE
<222> 6
<223> Xaa represents any amino acid residue

<220>
<221> SITE
<222> 7
<223> Xaa represents any amino acid residue

<220>
<221> SITE
<222> 8
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE
<222> 13
<223> Xaa represents any amino acid residue

<220>
<221> SITE
<222> 15
<223> Xaa represents any amino acid residue

<220>
<221> SITE
<222> 17
<223> Xaa represents any amino acid residue

<220>
<221> SITE
<222> 18
<223> Xaa represents any amino acid residue

<220>
<221> SITE
<222> 20
<223> Xaa represents any amino acid residue

<220>
<221> SITE
<222> 22
<223> Xaa represents any amino acid residue

<220>
<221> SITE
<222> 23
<223> Xaa represents any amino acid residue

<220>
<221> SITE
<222> 26
<223> Xaa represents any amino acid residue

<220>
<221> SITE
<222> 28
<223> Xaa represents any amino acid residue

<220>
<221> SITE
<222> 29
<223> Xaa represents any amino acid residue

<220>
<221> SITE
<222> 31
<223> Xaa represents any amino acid residue

<220>
<221> SITE
<222> 33
<223> Xaa represents any amino acid residue

<220>
<221> SITE
<222> 34
<223> Xaa represents any amino acid residue

<220>
<221> SITE
<222> 38

<223> Xaa represents any amino acid residue

<220>
<221> SITE
<222> 39
<223> Xaa represents any amino acid residue

<220>
<221> SITE
<222> 41
<223> Xaa represents any amino acid residue

<220>
<221> SITE
<222> 42
<223> Xaa represents any amino acid residue

<220>
<221> SITE
<222> 43
<223> Xaa represents any amino acid residue

<220>
<221> SITE
<222> 44
<223> Xaa represents any amino acid residue

<400> 29
```
Pro Xaa Xaa Pro Xaa Xaa Xaa Xaa Pro Xaa Xaa Pro Xaa Ser Xaa Tyr
1               5                   10                  15
Xaa Xaa Leu Xaa Pro Xaa Xaa Pro Glu Xaa Tyr Xaa Xaa Leu Xaa Pro
            20                  25                  30
Xaa Xaa Pro Asp Tyr Xaa Xaa Leu Xaa Xaa Xaa Xaa
        35                  40
```

<210> 30
<211> 41
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any one or several amino acid residue(s)

<220>
<221> SITE
<222> 7
<223> Xaa represents any amino acid residue

<220>
<221> SITE
<222> 8
<223> Xaa represents any amino acid residue

<220>
<221> SITE
<222> 10
<223> Xaa represents any one or several amino acid residue(s)

<220>
<221> SITE
<222> 12
<223> Xaa represents any amino acid residue

<220>
<221> SITE
<222> 14
<223> Xaa represents any amino acid residue

<220>
<221> SITE
<222> 15
<223> Xaa represents any amino acid residue

<220>
<221> SITE
<222> 17
<223> Xaa represents any one or several amino acid residue(s)

<220>
<221> SITE
<222> 19
<223> Xaa represents any amino acid residue

<220>
<221> SITE
<222> 20
<223> Xaa represents any amino acid residue

<220>
<221> SITE
<222> 23
<223> Xaa represents any one or several amino acid residue(s)

<220>
<221> SITE
<222> 25
<223> Xaa represents any amino acid residue

<220>
<221> SITE
<222> 26
<223> Xaa represents any amino acid residue

<220>
<221> SITE
<222> 28
<223> Xaa represents any one or several amino acid residue(s)

<220>
<221> SITE

<222> 30
<223> Xaa represents any amino acid residue

<220>
<221> SITE
<222> 31
<223> Xaa represents any amino acid residue

<220>
<221> SITE
<222> 35
<223> Xaa represents any amino acid residue

<220>
<221> SITE
<222> 36
<223> Xaa represents any amino acid residue

<220>
<221> SITE
<222> 38
<223> Xaa represents any amino acid residue

<220>
<221> SITE
<222> 39
<223> Xaa represents any amino acid residue

<220>
<221> SITE
<222> 40
<223> Xaa represents any amino acid residue

<220>
<221> SITE
<222> 41
<223> Xaa represents any amino acid residue

<400> 30
```
Pro Xaa Xaa Pro Xaa Pro Xaa Xaa Pro Xaa Ser Xaa Tyr Xaa Xaa Leu
1               5                   10                  15
Xaa Pro Xaa Xaa Pro Glu Xaa Tyr Xaa Xaa Leu Xaa Pro Xaa Xaa Pro
            20                  25                  30
Asp Tyr Xaa Xaa Leu Xaa Xaa Xaa Xaa
        35                  40
```

<210> 31
<211> 20
<212> PRT
<213> Artificial Sequence

<220>
<223> Pilot peptide

<220>
<221> SITE
<222> 2
<223> Xaa represents any amino acid residue

<220>
<221> SITE
<222> 3

<223> Xaa represents any amino acid residue

<220>
<221> SITE
<222> 5
<223> Xaa represents any amino acid residue

<220>
<221> SITE
<222> 6
<223> Xaa represents any amino acid residue

<220>
<221> SITE
<222> 7
<223> Xaa represents any amino acid residue

<220>
<221> SITE
<222> 8
<223> Xaa represents any amino acid residue

<220>
<221> SITE
<222> 9
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE
<222> 12
<223> Xaa represents any amino acid residue

<220>
<221> SITE
<222> 13
<223> Xaa represents any amino acid residue

<220>
<221> SITE
<222> 14
<223> Xaa represents any amino acid residue

<220>
<221> SITE
<222> 15
<223> Xaa represents any amino acid residue

<220>
<221> SITE
<222> 16
<223> Xaa represents any amino acid residue

<220>

<221> SITE
<222> 18
<223> Xaa represents any amino acid residue

<220>
<221> SITE
<222> 19
<223> Xaa represents any amino acid residue

<400> 31
```
Pro Xaa Xaa Pro Xaa Xaa Xaa Xaa Xaa Xaa Xaa Xaa Xaa Xaa Xaa Xaa
1               5                   10                  15
Tyr Xaa Xaa Leu
            20
```

<210> 32
<211> 20
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any amino acid residue

<220>
<221> SITE
<222> 6
<223> Xaa represents any amino acid residue

<220>
<221> SITE
<222> 7
<223> Xaa represents any amino acid residue

<220>
<221> SITE
<222> 8
<223> Xaa represents any amino acid residue

<220>
<221> SITE
<222> 9
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE
<222> 12
<223> Xaa represents any amino acid residue

<220>
<221> SITE
<222> 13
<223> Xaa represents any amino acid residue

<220>
<221> SITE
<222> 14
<223> Xaa represents any amino acid residue

<220>
<221> SITE
<222> 15
<223> Xaa represents any amino acid residue

<220>
<221> SITE
<222> 18
<223> Xaa represents any amino acid residue

<220>
<221> SITE
<222> 19
<223> Xaa represents any amino acid residue

<400> 32
```
Pro Xaa Xaa Pro Xaa Xaa Xaa Xaa Xaa Xaa Xaa Xaa Xaa Xaa Xaa Asp
1               5                   10                  15
Tyr Xaa Xaa Leu
            20
```

<210> 33
<211> 20
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE

<222> 5
<223> Xaa represents any amino acid residue

<220>
<221> SITE
<222> 6
<223> Xaa represents any amino acid residue

<220>
<221> SITE
<222> 8
<223> Xaa represents any amino acid residue

<220>
<221> SITE
<222> 9
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE
<222> 12
<223> Xaa represents any amino acid residue

<220>
<221> SITE
<222> 13
<223> Xaa represents any amino acid residue

<220>
<221> SITE
<222> 14
<223> Xaa represents any amino acid residue

<220>
<221> SITE
<222> 15
<223> Xaa represents any amino acid residue

<220>
<221> SITE
<222> 16
<223> Xaa represents any amino acid residue

<220>
<221> SITE
<222> 18
<223> Xaa represents any amino acid residue

<220>
<221> SITE
<222> 19
<223> Xaa represents any amino acid residue

<400> 33
Pro Xaa Xaa Pro Xaa Xaa Tyr Xaa Xaa Xaa Xaa Xaa Xaa Xaa Xaa Xaa
1 5 10 15
Tyr Xaa Xaa Leu
20

<210> 34
<211> 20
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any amino acid residue

<220>
<221> SITE
<222> 6
<223> Xaa represents any amino acid residue

<220>
<221> SITE
<222> 8
<223> Xaa represents any amino acid residue

<220>
<221> SITE
<222> 9
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE
<222> 12
<223> Xaa represents any amino acid residue

<220>
<221> SITE
<222> 13

<223> Xaa represents any amino acid residue

<220>
<221> SITE
<222> 14
<223> Xaa represents any amino acid residue

<220>
<221> SITE
<222> 15
<223> Xaa represents any amino acid residue

<220>
<221> SITE
<222> 18
<223> Xaa represents any amino acid residue

<220>
<221> SITE
<222> 19
<223> Xaa represents any amino acid residue

<400> 34

```
Pro Xaa Xaa Pro Xaa Xaa Tyr Xaa Xaa Xaa Xaa Xaa Xaa Xaa Xaa Asp
1               5                   10                  15
Tyr Xaa Xaa Leu
            20
```

<210> 35
<211> 20
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 6
<223> Xaa represents any amino acid residue

<220>
<221> SITE
<222> 8
<223> Xaa represents any amino acid residue

<220>
<221> SITE
<222> 9
<223> Xaa represents any amino acid residue

<220>

```
<221> SITE
<222> 11
<223> Xaa represents any amino acid residue

<220>
<221> SITE
<222> 13
<223> Xaa represents any amino acid residue

<220>
<221> SITE
<222> 14
<223> Xaa represents any amino acid residue

<220>
<221> SITE
<222> 18
<223> Xaa represents any amino acid residue

<220>
<221> SITE
<222> 19
<223> Xaa represents any amino acid residue

<400> 35
Pro Xaa Xaa Pro Glu Xaa Tyr Xaa Xaa Leu Xaa Pro Xaa Xaa Pro Asp
1               5                   10                  15
Tyr Xaa Xaa Leu
            20

<210> 36
<211> 9
<212> PRT
<213> Artificial Sequence


<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any one or several amino acid residue(s)

<220>
<221> SITE
<222> 7
<223> Xaa represents any amino acid residue

<220>
<221> SITE
<222> 8
<223> Xaa represents any amino acid residue
```

<400> 36
Pro Xaa Xaa Pro Xaa Tyr Xaa Xaa Leu
1               5

<210> 37
<211> 10
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any one or several amino acid residue(s)

<220>
<221> SITE
<222> 8
<223> Xaa represents any amino acid residue

<220>
<221> SITE
<222> 9
<223> Xaa represents any amino acid residue

<400> 37
Pro Xaa Xaa Pro Xaa Asp Tyr Xaa Xaa Leu
1               5                   10

<210> 38
<211> 11
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>

<221> SITE
<222> 5
<223> Xaa represents any one or several amino acid residue(s)

<220>
<221> SITE
<222> 7
<223> Xaa represents any one or several amino acid residue(s)

<220>
<221> SITE
<222> 9
<223> Xaa represents any amino acid residue

<220>
<221> SITE
<222> 10
<223> Xaa represents any amino acid residue

<400> 38
```
Pro Xaa Xaa Pro Xaa Tyr Xaa Tyr Xaa Xaa Leu
1               5                   10
```

<210> 39
<211> 12
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any one or several amino acid residue(s)

<220>
<221> SITE
<222> 7
<223> Xaa represents any one or several amino acid residue(s)

<220>
<221> SITE
<222> 10
<223> Xaa represents any amino acid residue

<220>
<221> SITE
<222> 11
<223> Xaa represents any amino acid residue

<400> 39

Pro Xaa Xaa Pro Xaa Tyr Xaa Asp Tyr Xaa Xaa Leu
1 5 10

<210> 40
<211> 20
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 6
<223> Xaa represents any one or several amino acid residue(s)

<220>
<221> SITE
<222> 8
<223> Xaa represents any amino acid residue

<220>
<221> SITE
<222> 9
<223> Xaa represents any amino acid residue

<220>
<221> SITE
<222> 11
<223> Xaa represents any one or several amino acid residue(s)

<220>
<221> SITE
<222> 13
<223> Xaa represents any amino acid residue

<220>
<221> SITE
<222> 14
<223> Xaa represents any amino acid residue

<220>
<221> SITE
<222> 18
<223> Xaa represents any amino acid residue

<220>
<221> SITE
<222> 19
<223> Xaa represents any amino acid residue

<400> 40

```
Pro Xaa Xaa Pro Glu Xaa Tyr Xaa Xaa Leu Xaa Pro Xaa Xaa Pro Asp
1               5                   10                  15
Tyr Xaa Xaa Leu
            20
```

<210> 41
<211> 40
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any amino acid residue

<220>
<221> SITE
<222> 6
<223> Xaa represents any amino acid residue

<220>
<221> SITE
<222> 7
<223> Xaa represents any amino acid residue

<220>
<221> SITE
<222> 8
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE
<222> 13
<223> Xaa represents any amino acid residue

<220>
<221> SITE
<222> 14
<223> Xaa represents any amino acid residue

<220>
<221> SITE
<222> 15
<223> Xaa represents any amino acid residue

<220>
<221> SITE
<222> 17
<223> Xaa represents any amino acid residue

<220>
<221> SITE
<222> 18
<223> Xaa represents any amino acid residue

<220>
<221> SITE
<222> 20
<223> Xaa represents any amino acid residue

<220>
<221> SITE
<222> 22
<223> Xaa represents any amino acid residue

<220>
<221> SITE
<222> 23
<223> Xaa represents any amino acid residue

<220>
<221> SITE
<222> 26
<223> Xaa represents any amino acid residue

<220>
<221> SITE
<222> 28
<223> Xaa represents any amino acid residue

<220>
<221> SITE
<222> 29
<223> Xaa represents any amino acid residue

<220>
<221> SITE
<222> 31
<223> Xaa represents any amino acid residue

<220>
<221> SITE
<222> 33
<223> Xaa represents any amino acid residue

<220>
<221> SITE
<222> 34
<223> Xaa represents any amino acid residue

<220>
<221> SITE

```
<222> 38
<223> Xaa represents any amino acid residue

<220>
<221> SITE
<222> 39
<223> Xaa represents any amino acid residue

<400> 41
Pro Xaa Xaa Pro Xaa Xaa Xaa Xaa Pro Xaa Xaa Pro Xaa Xaa Xaa Tyr
1               5                   10                  15
Xaa Xaa Leu Xaa Pro Xaa Xaa Pro Glu Xaa Tyr Xaa Xaa Leu Xaa Pro
            20                  25                  30
Xaa Xaa Pro Asp Tyr Xaa Xaa Leu
        35                  40

<210> 42
<211> 35
<212> PRT
<213> Artificial Sequence


<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any one or several amino acid residue(s)

<220>
<221> SITE
<222> 7
<223> Xaa represents any amino acid residue

<220>
<221> SITE
<222> 8
<223> Xaa represents any amino acid residue

<220>
<221> SITE
<222> 10
<223> Xaa represents any one or several amino acid residue(s)

<220>
<221> SITE
<222> 12
<223> Xaa represents any amino acid residue

<220>
<221> SITE
<222> 13
```

<223> Xaa represents any amino acid residue

<220>
<221> SITE
<222> 15
<223> Xaa represents any one or several amino acid residue(s)

<220>
<221> SITE
<222> 17
<223> Xaa represents any amino acid residue

<220>
<221> SITE
<222> 18
<223> Xaa represents any amino acid residue

<220>
<221> SITE
<222> 21
<223> Xaa represents any one or several amino acid residue(s)

<220>
<221> SITE
<222> 23
<223> Xaa represents any amino acid residue

<220>
<221> SITE
<222> 24
<223> Xaa represents any amino acid residue

<220>
<221> SITE
<222> 26
<223> Xaa represents any one or several amino acid residue(s)

<220>
<221> SITE
<222> 28
<223> Xaa represents any amino acid residue

<220>
<221> SITE
<222> 29
<223> Xaa represents any amino acid residue

<220>
<221> SITE
<222> 33
<223> Xaa represents any amino acid residue

<220>
<221> SITE
<222> 34
<223> Xaa represents any amino acid residue

<400> 42
```
Pro Xaa Xaa Pro Xaa Pro Xaa Xaa Pro Xaa Tyr Xaa Xaa Leu Xaa Pro
1               5                   10                  15
Xaa Xaa Pro Glu Xaa Tyr Xaa Xaa Leu Xaa Pro Xaa Xaa Pro Asp Tyr
            20                  25                  30
Xaa Xaa Leu
```

35

<210> 43
<211> 44
<212> PRT
<213> Artificial Sequence

<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any amino acid residue

<220>
<221> SITE
<222> 6
<223> Xaa represents any amino acid residue

<220>
<221> SITE
<222> 7
<223> Xaa represents any amino acid residue

<220>
<221> SITE
<222> 8
<223> Xaa represents any amino acid residue

<220>
<221> SITE
<222> 10
<223> Xaa represents any amino acid residue

<220>
<221> SITE
<222> 11
<223> Xaa represents any amino acid residue

<220>
<221> SITE
<222> 13
<223> Xaa represents any amino acid residue

<220>
<221> SITE
<222> 14
<223> Xaa represents any amino acid residue

<220>
<221> SITE

<222> 15
<223> Xaa represents any amino acid residue

<220>
<221> SITE
<222> 17
<223> Xaa represents any amino acid residue

<220>
<221> SITE
<222> 18
<223> Xaa represents any amino acid residue

<220>
<221> SITE
<222> 20
<223> Xaa represents any amino acid residue

<220>
<221> SITE
<222> 22
<223> Xaa represents any amino acid residue

<220>
<221> SITE
<222> 23
<223> Xaa represents any amino acid residue

<220>
<221> SITE
<222> 26
<223> Xaa represents any amino acid residue

<220>
<221> SITE
<222> 28
<223> Xaa represents any amino acid residue

<220>
<221> SITE
<222> 29
<223> Xaa represents any amino acid residue

<220>
<221> SITE
<222> 31
<223> Xaa represents any amino acid residue

<220>
<221> SITE
<222> 33
<223> Xaa represents any amino acid residue

<220>
<221> SITE
<222> 34
<223> Xaa represents any amino acid residue

<220>
<221> SITE
<222> 38
<223> Xaa represents any amino acid residue

```
<220>
<221> SITE
<222> 39
<223> Xaa represents any amino acid residue

<220>
<221> SITE
<222> 41
<223> Xaa represents any amino acid residue

<220>
<221> SITE
<222> 42
<223> Xaa represents any amino acid residue

<220>
<221> SITE
<222> 43
<223> Xaa represents any amino acid residue

<220>
<221> SITE
<222> 44
<223> Xaa represents any amino acid residue

<400> 43
Pro Xaa Xaa Pro Xaa Xaa Xaa Xaa Pro Xaa Xaa Pro Xaa Xaa Xaa Tyr
1               5                   10                  15
Xaa Xaa Leu Xaa Pro Xaa Xaa Pro Glu Xaa Tyr Xaa Xaa Leu Xaa Pro
            20                  25                  30
Xaa Xaa Pro Asp Tyr Xaa Xaa Leu Xaa Xaa Xaa Xaa
        35                  40

<210> 44
<211> 39
<212> PRT
<213> Artificial Sequence


<220>
<223> Pilot peptide

<220>
<221> SITE
<222> 2
<223> Xaa represents any amino acid residue

<220>
<221> SITE
<222> 3
<223> Xaa represents any amino acid residue

<220>
<221> SITE
<222> 5
<223> Xaa represents any one or several amino acid residue(s)

<220>
<221> SITE
<222> 7
<223> Xaa represents any amino acid residue

<220>
```

<221> SITE
<222> 8
<223> Xaa represents any amino acid residue

<220>
<221> SITE
<222> 10
<223> Xaa represents any one or several mino acid residue(s)

<220>
<221> SITE
<222> 12
<223> Xaa represents any amino acid residue

<220>
<221> SITE
<222> 13
<223> Xaa represents any amino acid residue

<220>
<221> SITE
<222> 15
<223> Xaa represents any one or several amino acid residue(s)

<220>
<221> SITE
<222> 17
<223> Xaa represents any amino acid residue

<220>
<221> SITE
<222> 18
<223> Xaa represents any amino acid residue

<220>
<221> SITE
<222> 21
<223> Xaa represents any one or several amino acid residue(s)

<220>
<221> SITE
<222> 23
<223> Xaa represents any amino acid residue

<220>
<221> SITE
<222> 24
<223> Xaa represents any amino acid residue

<220>
<221> SITE
<222> 26
<223> Xaa represents any one or several amino acid residue(s)

<220>
<221> SITE
<222> 28
<223> Xaa represents any amino acid residue

<220>
<221> SITE
<222> 29
<223> Xaa represents any amino acid residue

<220>
<221> SITE
<222> 33
<223> Xaa represents any amino acid residue

<220>
<221> SITE
<222> 34
<223> Xaa represents any amino acid residue

<220>
<221> SITE
<222> 36
<223> Xaa represents any amino acid residue

<220>
<221> SITE
<222> 37
<223> Xaa represents any amino acid residue

<220>
<221> SITE
<222> 38
<223> Xaa represents any amino acid residue

<220>
<221> SITE
<222> 39
<223> Xaa represents any amino acid residue

<400> 44
```
Pro Xaa Xaa Pro Xaa Pro Xaa Xaa Pro Xaa Tyr Xaa Xaa Leu Xaa Pro
1               5                   10                  15
Xaa Xaa Pro Glu Xaa Tyr Xaa Xaa Leu Xaa Pro Xaa Xaa Pro Asp Tyr
            20                  25                  30
Xaa Xaa Leu Xaa Xaa Xaa Xaa
        35
```

<210> 45
<211> 27
<212> PRT
<213> Artificial Sequence

<220>
<223> T4-fibritin â\200\234Foldonâ\200\235 trimerization domain

<400> 45
```
Gly Tyr Ile Pro Glu Ala Pro Arg Asp Gly Gln Ala Tyr Val Arg Lys
1               5                   10                  15
Asp Gly Glu Trp Val Leu Leu Ser Thr Phe Leu
            20                  25
```

<210> 46
<211> 5
<212> PRT
<213> Artificial Sequence

<220>
<223> Peptide targeting the MPER subdomain

<400> 46
Pro Trp Tyr Ile Trp
1               5

<210> 47
<211> 6
<212> PRT
<213> Artificial Sequence

<220>
<223> Peptide targeting the MPER subdomain

<400> 47
Trp Pro Trp Tyr Ile Trp
1               5

<210> 48
<211> 7
<212> PRT
<213> Artificial Sequence

<220>
<223> Peptide targeting the MPER subdomain

<400> 48
Lys Trp Pro Trp Tyr Ile Trp
1               5

<210> 49
<211> 8
<212> PRT
<213> Artificial Sequence

<220>
<223> Peptide targeting the MPER subdomain

<400> 49
Ile Lys Trp Pro Trp Tyr Ile Trp
1               5

<210> 50
<211> 9
<212> PRT
<213> Artificial Sequence

<220>
<223> Peptide targeting the MPER subdomain

<400> 50
Tyr Ile Lys Trp Pro Trp Tyr Ile Trp
1               5

<210> 51
<211> 10
<212> PRT
<213> Artificial Sequence

<220>

<223> Peptide targeting the MPER subdomain

<400> 51
Gln Tyr Ile Lys Trp Pro Trp Tyr Ile Trp
1 5 10

<210> 52
<211> 11
<212> PRT
<213> Artificial Sequence

<220>
<223> Peptide targeting the MPER subdomain

<400> 52
Glu Gln Tyr Ile Lys Trp Pro Trp Tyr Ile Trp
1 5 10

<210> 53
<211> 12
<212> PRT
<213> Artificial Sequence

<220>
<223> Peptide targeting the MPER subdomain

<400> 53
Tyr Glu Gln Tyr Ile Lys Trp Pro Trp Tyr Ile Trp
1 5 10

<210> 54
<211> 13
<212> PRT
<213> Artificial Sequence

<220>
<223> Peptide targeting the MPER subdomain

<400> 54
Lys Tyr Glu Gln Tyr Ile Lys Trp Pro Trp Tyr Ile Trp
1 5 10

<210> 55
<211> 14
<212> PRT
<213> Artificial Sequence

<220>
<223> Peptide targeting the MPER subdomain

<400> 55
Gly Lys Tyr Glu Gln Tyr Ile Lys Trp Pro Trp Tyr Ile Trp
1 5 10

<210> 56
<211> 15
<212> PRT
<213> Artificial Sequence

```
<220>
<223> Peptide targeting the MPER subdomain

<400> 56
Leu Gly Lys Tyr Glu Gln Tyr Ile Lys Trp Pro Trp Tyr Ile Trp
1               5                   10                  15
```